# EUROPEAN PATENT APPLICATION

(11) **EP 4 129 282 A1**
(43) Date of publication of application: **08.02.2023**
(21) Application number: 21778854.6
(22) Date of filing: 30.03.2021
(51) Int. Cl.: A61K 31/375, A61K 31/505, C07D 487/04, C12N 15/11, A61K 45/06, A61P 35/00

(54) **PHARMACEUTICAL COMBINATION AND USE THEREOF**

(30) Priority: 31.03.2020 CN 202010249023
(71) Applicant: NATURAL MEDICINE INSTITUTE OF ZHEJIANG YANGSHENGTANG CO., LTD., Xihu District Hangzhou Zhejiang 310024 (CN)
(72) Inventor: XU, Nuo, Hangzhou, Zhejiang 310024 (CN); LIN, Jian, Beijing 100871 (CN)
(74) Representative: Böhm, Brigitte
(86) International application number: PCT/CN2021/084009
(87) International publication number: WO 2021/197334

(57) **Abstract**

A pharmaceutical combination for preventing and/or treating cancer and a kit. The pharmaceutical combination comprises: a) a prophylactically and/or therapeutically effective amount of ascorbic acid or a derivative thereof; and b) a prophylactically and/or therapeutically effective amount of a substance, such as PKF118-130, NCB-0846, PRI-724, siRNA484, siRNA1202, siRNA1387, a compound C-1, and a compound C-2, affecting the formation of a TCF/β-catenin complex.

## Description

### TECHNICAL FIELD

The present application relates to the field of biomedicine, in particular to a pharmaceutical combination and use thereof.

### BACKGROUND OF THE INVENTION

Malignant tumors are diseases that are serious health hazard at present. For example, osteosarcoma as a common malignant bone tumor has an annual incidence of about 3/1,000,000 in China. In general, 20% of patients have developed distant metastasis at the time of diagnosis of osteosarcoma. If the osteosarcoma has developed distant metastasis, the five-year disease-free survival rate of patients is less than 20%. Current drugs for treating osteosarcoma comprise high-dose methotrexate, ifosfamide, doxorubicin, cisplatin, etc. Alternatively, two or more drugs are used in combination for chemotherapy to ensure a sufficient dose intensity. However, these chemotherapy regimens would produce strong side effects that may endanger life and health. Colorectal cancer accounts for 10.2% of the total incidence of cancers and 9.2% of deaths, namely, there are approximately 1,850,000 new cases and 880,000 deaths from colorectal cancer every year. Oxaliplatin is the main clinical treatment for colorectal cancer. However, more than 30% of patients with colon cancer are not sensitive to oxaliplatin. Ovarian cancer accounts for about 3.4% of the malignant tumor incidence, and the clinical chemotherapy regimens comprise platinum-based drugs + cyclophosphamide (PC) and taxol + carboplatin (TP). However, these chemotherapy regimens also have serious side effects, for example, hazards to the digestive system and blood system. These all show that chemotherapeutics exhibit different degrees of toxic and side effects while exhibiting an anti-tumor effect. In addition, some tumor patients are not sensitive to chemotherapeutics. Therefore, it is an urgent problem for the medical community around the world to find more effective anti-tumor drugs and methods.

### SUMMARY OF THE INVENTION

The present application provides a pharmaceutical combination and use or administration method thereof, primarily comprising: using a substance affecting the formation of a TCF/β-catenin complex and ascorbic acid or a derivative thereof as a pharmaceutical combination for treating tumors, or administering a substance affecting the formation of a TCF/β-catenin complex in combination with ascorbic acid or a derivative thereof for treating tumors. The present application has at least one of the following technical effects:
1) the pharmaceutical combination can serve as an alternative to traditional chemotherapeutics to avoid the toxic and side effects caused by the traditional chemotherapy;
2) the pharmaceutical combination is used to treat patients who are not sensitive to traditional chemotherapeutics to achieve the therapeutic effects;
3) the pharmaceutical combination can be used in combination with traditional chemotherapeutic(s) to decrease the dose of chemotherapeutics, thereby reducing the toxic and side effects caused by the traditional chemotherapeutics;
4) the combination of the substance affecting the formation of a TCF/β-catenin complex with ascorbic acid or a derivative thereof produces an unexpected inhibitory effect on tumors, significantly decreases the dose of single-drug administration with equivalent effect, and improves the drug safety;
5) the pharmaceutical combination specifically inhibits the growth of tumors or tumor cells, and would not affect the immune cells or other physiological indications of the subject, indicating the drug safety.

The present application provides a pharmaceutical combination, comprising:
a) a prophylactically and/or therapeutically effective amount of ascorbic acid or a derivative thereof; and
b) a prophylactically and/or therapeutically effective amount of a substance affecting the formation of a TCF/β-catenin complex.

In certain embodiments, the substance affecting the formation of a TCF/β-catenin complex comprises a TCF/LEF inhibitor.

In certain embodiments, the TCF/LEF inhibitor comprises NCB-0846.

In certain embodiments, the substance affecting the formation of a TCF/β-catenin complex comprises a substance affecting the formation of a β-catenin/CBP complex.

In certain embodiments, the substance affecting the formation of a β-catenin/CBP complex comprises PRI-724.

In certain embodiments, the substance affecting the formation of a TCF/β-catenin complex comprises a β-catenin inhibitor.

In certain embodiments, the β-catenin inhibitor comprises a substance down-regulating the expression level and/or activity of the β-catenin protein.

In certain embodiments, the substance down-regulating the expression level and/or activity of the β-catenin protein comprises siRNA and/or shRNA.

In certain embodiments, the substance down-regulating the expression level and/or activity of the β-catenin protein comprises a nucleic acid sequence as shown in any one of SEQ ID NOS: 1-6.

In certain embodiments, the substance affecting the formation of a TCF/β-catenin complex comprises a compound having a structure of Formula I, II or III or a pharmaceutically acceptable salt or hydrate thereof: 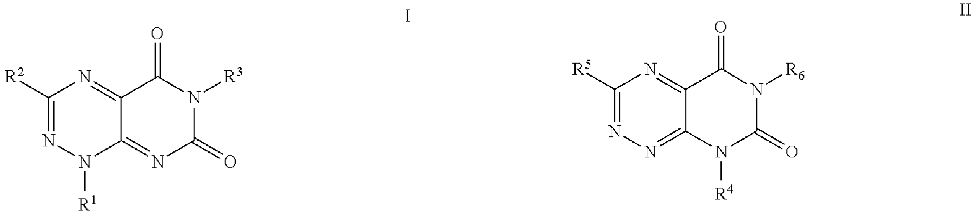 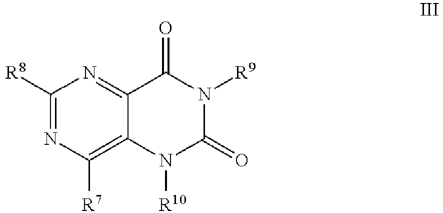
wherein, R¹ and R³ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R² is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl;
R⁴ and R⁶ are each independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R⁵ is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl, optionally substituted heteroaryl, optionally substituted C₁₋₂₀ alkylamino, optionally substituted C₁₋₂₀ alkoxy and optionally substituted C₁₋₂₀ alkylformamido;
R⁷, R⁸, R⁹, and R¹⁰ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl.

In certain embodiments, the R² is C₄₋₆ alkyl.

In certain embodiments, the R² is C₄ alkyl.

In certain embodiments, the R² is isobutyl.

In certain embodiments, the R¹ is selected from the group consisting of hydrogen and C₁₋₆ alkyl.

In certain embodiments, the R¹ is C₃ alkyl.

In certain embodiments, the R¹ is isopropyl.

In certain embodiments, the R³ is selected from the group consisting of hydrogen and C₁₋₆ alkyl.

In certain embodiments, the R³ is methyl.

In certain embodiments, the compound having a structure of Formula I comprises PKF118-310 or a derivative thereof.

In certain embodiments, the derivative of PKF118-310 comprises 3-Methyltoxoflavin and/or Walrycin B.

In certain embodiments, the compound having a structure of Formula II comprises Fervenulin.

In certain embodiments, the substance affecting the formation of a TCF/β-catenin complex comprises BI-D1870 and/or Lumazine.

In certain embodiments, the derivative of ascorbic acid comprises a pharmaceutically acceptable ascorbate.

In certain embodiments, the pharmaceutically acceptable ascorbate comprises sodium ascorbate.

In certain embodiments, the ratio of the effective amount of the substance affecting the formation of a TCF/β-catenin complex to the effective amount of the ascorbic acid or a derivative thereof are about 1% to about 10%.

In certain embodiments, the mass ratio of the substance affecting the formation of a TCF/β-catenin complex to the ascorbic acid or a derivative thereof are about 90% to about 99%.

In certain embodiments, the substance affecting the formation of a TCF/β-catenin complex and the ascorbic acid or a derivative thereof are each present in different containers.

In certain embodiments, the pharmaceutical combination comprises a first preparation and a second preparation, wherein the first preparation comprises the ascorbic acid or a derivative thereof and a pharmaceutically acceptable first carrier, and the second preparation comprises the substance affecting the formation of a TCF/β-catenin complex and a pharmaceutically acceptable second carrier.

In certain embodiments, the pharmaceutical combination comprises a pharmaceutical composition comprising the substance affecting the formation of a TCF/β-catenin complex and the ascorbic acid or a derivative thereof.

In certain embodiments, the substance affecting the formation of a TCF/β-catenin complex has a content of about 5% to 20% (w/w) in the pharmaceutical composition.

In the pharmaceutical combination of the present application, the ascorbic acid or a derivative thereof has a content of about 80% to 95% (w/w) in the pharmaceutical composition.

In another aspect, the present application further provides a kit comprising the pharmaceutical combination as described in the present application.

In another aspect, the present application further provides use of a combination of ascorbic acid or a derivative thereof with a substance affecting the formation of a TCF/β-catenin complex in the manufacture of a drug for preventing and/or treating tumors.

In certain embodiments, the substance affecting the formation of a TCF/β-catenin complex comprises a TCF/LEF inhibitor.

In certain embodiments, the TCF/LEF inhibitor comprises NCB-0846.

In certain embodiments, the substance affecting the formation of a TCF/β-catenin complex comprises a substance affecting the formation of a β-catenin/CBP complex.

In certain embodiments, the substance affecting the formation of a β-catenin/CBP complex comprises PRI-724.

In certain embodiments, the substance affecting the formation of a TCF/β-catenin complex comprises a β-catenin inhibitor.

In certain embodiments, the β-catenin inhibitor comprises a substance down-regulating the expression level and/or activity of the β-catenin protein.

In certain embodiments, the substance down-regulating the expression level and/or activity of the β-catenin protein comprises siRNA and/or shRNA.

In certain embodiments, the substance down-regulating the expression level and/or activity of the β-catenin protein comprises a nucleic acid sequence as shown in any one of SEQ ID NOS: 1-6.

In certain embodiments, the substance affecting the formation of a TCF/β-catenin complex comprises a compound having a structure of Formula I, II or III or a pharmaceu: 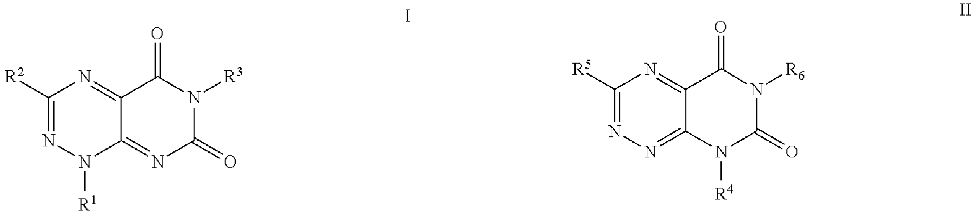 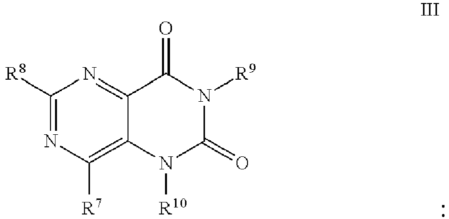
tically acceptable salt or hydrate thereof:wherein, R¹ and R³ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R² is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl;
R⁴ and R⁶ are each independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R⁵ is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl, optionally substituted heteroaryl, optionally substituted C₁₋₂₀ alkylamino, optionally substituted C₁₋₂₀ alkoxy and optionally substituted C₁₋₂₀ alkylformamido;
R⁷, R⁸, R⁹, and R¹⁰ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl.

In certain embodiments, the R² is C₄₋₆ alkyl.

In certain embodiments, the R² is C₄ alkyl.

In certain embodiments, the R² is isobutyl.

In certain embodiments, the R¹ is selected from the group consisting of hydrogen and C₁₋₆ alkyl.

In certain embodiments, the R¹ is C₃ alkyl.

In certain embodiments, the R¹ is isopropyl.

In certain embodiments, the R³ is selected from the group consisting of hydrogen and C₁₋₆ alkyl.

In certain embodiments, the R³ is methyl.

In certain embodiments, the compound having a structure of Formula I comprises PKF118-310 or a derivative thereof.

In certain embodiments, the derivative of PKF118-310 comprises 3-Methyltoxoflavin and/or Walrycin B.

In certain embodiments, the compound having a structure of Formula II comprises Fervenulin.

In certain embodiments, the substance affecting the formation of a TCF/β-catenin complex comprises BI-D1870 and/or Lumazine.

In certain embodiments, the tumors comprise solid tumors or non-solid tumors.

In certain embodiments, the tumors comprise osteosarcoma, colon cancer, ovarian cancer, and lymphoma.

In certain embodiments, the derivative of ascorbic acid comprises a pharmaceutically acceptable ascorbate.

In certain embodiments, the pharmaceutically acceptable ascorbate comprises sodium ascorbate.

In certain embodiments, the substance affecting the formation of a TCF/β-catenin complex and the ascorbic acid or a derivative thereof are formulated to be simultaneously administered to a subject.

In certain embodiments, the substance affecting the formation of a TCF/β-catenin complex and the ascorbic acid or a derivative thereof are formulated to be separated administered to a subject.

In certain embodiments, the drug is formulated so that the substance affecting the formation of a TCF/β-catenin complex is administered at a dose of about 0.05 mg/kg to about 40 mg/kg.

In certain embodiments, the drug is formulated so that the ascorbic acid or a derivative thereof are administered at a dose of about 0.05 g/kg to about 2.5 g/kg.

In certain embodiments, the drug is formulated so that the substance affecting the formation of a TCF/β-catenin complex and the ascorbic acid or a derivative thereof are administered at a mass ratio of about 1:30 to about 1 :5000.

In certain embodiments, the drug is formulated so that the substance affecting the formation of a TCF/β-catenin complex and the ascorbic acid or a derivative thereof are administered at an effective amount ratio of about 1:30 to about 1:5000.

In another aspect, the present application further provides a method for preventing and/or treating tumors comprising administering to a subject in need thereof:
a) ascorbic acid or a derivative thereof; and
b) a substance affecting the formation of a TCF/β-catenin complex.

In another aspect, the present application further provides a method for monitoring a response to a drug in a subject comprising:
a) administering to the subject ascorbic acid or a derivative thereof and a substance affecting the formation of a TCF/β-catenin complex; and
b) detecting a change in one or more of the level and/or activity of blood alkaline phosphatase, the level and/or activity of lactic dehydrogenase, the expression level of CADM1 gene, the expression level of CD44 gene, the expression level of livin gene, the expression level of HIF-1α gene, the expression level of ET-1 gene, the expression level of IGF-1R gene, the expression level of STAT3 gene, the expression level of CEA gene, the expression level of CA199 gene, the expression level of CA125 gene, the expression level of AFP gene, the expression level of CA724 gene, and the expression level of β-HCG gene in the subject after the administration of a).

Persons skilled in the art can readily recognize other aspects and advantages of the present application form the detailed description below. The following detailed description only shows and describes exemplary embodiments of the present application. As persons skilled in the art will appreciate, the present application enables persons skilled in the art to make modifications to the disclosed embodiments without departing from the spirit and scope of the invention involved in the present application. Correspondingly, the accompany drawings and description in the specification of the present application are only illustrative, rather than restrictive.

### BRIEF DESCRIPTION OF THE DRAWING

The specific features of the invention involved in the present application are shown in the appended claims. By referring to the exemplary embodiments as described in detail below and the accompanying drawings, the features and advantages of the invention involved in the present application can be better understood. The accompany drawings are briefly described as follows:
**FIG. 1** shows an effect of PKF118-310 with different concentrations on 143B cells and HOS cells as described in the present application.
**FIG. 2** shows an inhibitory effect of PKF 118-310 in combination with sodium ascorbate on 143B cells and HOS cells as described in the present application.
**FIG. 3** shows an inhibitory effect of PKF118-310 in combination with sodium ascorbate on the migration of 143B cells and HOS cells as described in the present application.
**FIG. 4** shows a result of Luciferin fluorescence imaging of osteosarcoma in mice treated with PKF 118-310 in combination with sodium ascorbate as described in the present application.
**FIG. 5** shows a quantitative statistical result of Luciferin fluorescence imaging of osteosarcoma in mice treated with PKF 118-310 in combination with sodium ascorbate as described in the present application.
**FIG. 6** shows a statistical result of change in the osteosarcoma volume in mice treated with PKF118-310 in combination with sodium ascorbate as described in the present application.
**FIG. 7** shows an inhibitory effect on the osteosarcoma volume in mice treated with PKF118-310 in combination with sodium ascorbate as described in the present application.
**FIG. 8** shows an effect of PKF 118-310 and sodium ascorbate on the weight of mice as described in the present application.
**FIG. 9** shows an effect of PKF118-310 and sodium ascorbate on various organs in mice as described in the present application.
**FIG. 10** shows an inhibitory effect of PRI-724 in combination with sodium ascorbate on HOS cells as described in the present application.
**FIG. 11** shows an inhibitory effect of NCB-0846 in combination with sodium ascorbate on 143B cells and HOS cells as described in the present application.
**FIG. 12** shows an inhibitory effect of siRNA for β-catenin in combination with sodium ascorbate on 143B cells as described in the present application.
**FIG. 13** shows an inhibitory effect of PKF118-310 in combination with sodium ascorbate on SKOV3 cells as described in the present application.
**FIG. 14** shows an inhibitory effect of PKF 118-310 in combination with sodium ascorbate on 3AO cells as described in the present application.
**FIG. 15** shows an effect of oxaliplatin on MC38 cells and HCT116 cells as described in the present application.
**FIG. 16** shows an effect of PKF118-310 with different concentrations on MC38 cells and HCT116 cells as described in the present application.
**FIG. 17** shows an effect of sodium ascorbate with different concentrations on MC38 cells and HCT116 cells as described in the present application.
**FIG. 18** shows an inhibitory effect of PKF 118-310 in combination with sodium ascorbate on MC38 cells and HCT116 cells as described in the present application.
**FIG. 19** shows an effect of PKF 118-310 in combination with sodium ascorbate on T cells as described in the present application.
**FIG. 20** shows an effect of PKF118-310 and sodium ascorbate on macrophages as described the present application.
**FIG. 21** shows an inhibitory effect of the PKF118-310 derivative (Walrycin B) in combination with sodium ascorbate on HOS cells as described in the present application.
**FIG. 22** shows an inhibitory effect of the PKF118-310 derivative (3-Methyltoxoflavin) in combination with sodium ascorbate on HOS cells as described in the present application.
**FIG. 23** shows an inhibitory effect of the PKF118-310 derivative (the compound C-1 of the present application) in combination with sodium ascorbate on 143B cells as described in the present application.
**FIG. 24** shows an inhibitory effect of the PKF118-310 derivative (the compound C-2 of the present application) in combination with sodium ascorbate on 143B cells as described in the present application.
**FIG. 25** shows an inhibitory effect of the PKF118-310 derivative (the compound C-1 of the present application) in combination with sodium ascorbate on MC38 cells as described in the present application.
**FIG. 26** shows an inhibitory effect of the PKF118-310 derivative (Compound C-2 as described in the present application) in combination with sodium ascorbate as described in the present application on MC38 cells.
**FIG. 27** shows an inhibitory effect of PKF118-310 with different concentrations in combination with sodium ascorbate described in the present application on DB cells.

### DETAILED DESCRIPTION OF THE INVENTION

Hereinafter the embodiments of the invention of the application are described by specific examples. Those skilled in the art can easily understand other advantages and effects of the invention as described in the present application from the disclosure in the description.

### DEFINITIONS OF TERMS

In the present application, the term "alkyl" generally refers to a linear or branched saturated hydrocarbon group. The non-limiting examples thereof can comprise methyl, ethyl, and linear and branched propyl and butyl. For example, the alkyl can have, *e.g.,* 1-20 carbon atoms, 1-10 carbon atoms, or 1-6 carbon atoms. For example, the term "alkyl" can further comprise "bridged alkyl", i.e., bicyclic or polycyclic hydrocarbon groups, e.g., norbornyl, adamantyl, bicyclo[2.2.2]octyl, bicyclo[2.2.1]heptyl, bicyclo[3.2.1]octyl or decalinyl. For example, the alkyl groups can be optionally substituted, e.g., with hydroxyl (-OH), oxo (=O), halogen, aryl, heteroaryl, cycloalkyl, heterocycloalkyl, amino, nitro (NOz), or thio.

In the present application, the term "alkynyl" generally refers to a linear or branched hydrocarbon group containing at least one carbon-carbon triple bond. The non-limiting examples thereof can comprise ethynyl, as well as linear or branched propynyl or butynyl. For example, the alkynyl can have, e.g., 2-20 carbon atoms, 2-10 carbon atoms, and/or 2-6 carbon atoms. For example, the alkynyl group can be optionally substituted, e.g., with hydroxyl (-OH), oxo (=O), halogen, aryl, heteroaryl, cycloalkyl, heterocycloalkyl, amino, nitro (NOz), or thio.

In the present application, the term "cycloalkyl" generally refers to a cyclic C₃₋₂₀ hydrocarbon group. For example, cyclopropyl, cyclobutyl, cyclohexyl, cyclopentyl, etc. The term "heterocycloalkyl" can be defined similarly cycloalkyl, except that the ring contains one or more heteroatoms, e.g., 1-3 heteroatoms, which can be independently selected from the group consisting of oxygen, nitrogen, and sulfur. The non-limiting examples of heterocycloalkyl can comprise piperidyl, tetrahydrofuryl, tetrahydropyranyl, dihydrofuryl, etc. The cycloalkyl group and the heterocycloalkyl group can be optionally substituted, e.g., with alkyl, alkylene-OH, -C(O)NH₂, - NH₂, -NOz, oxo (=O), aryl, haloalkyl, halogen, -OH, -SH, etc. The heterocycloalkyl can further be optionally substituted with alkyl, hydroalkyl, alkylene aryl, or alkylene heteroaryl.

In the present application, the term "aryl" generally refers to a monocyclic or polycyclic aromatic group. Examples can comprise a monocyclic or bicyclic aromatic group, e.g., phenyl or naphthyl. Unless otherwise indicated, the aryl can be unsubstituted or substituted with one or more *(e.g.,* 1-4) substituents independently selected from the group consisting of halogen, alkyl, alkenyl, -OCF₃, -NOz, -CN, -NC, -OH, alkoxy, amino, aryl and heteroaryl, for example. Exemplary aryls comprise, but are not limited to, phenyl, naphthyl, tetrahydronaphthyl, chlorophenyl, methylphenyl, methoxyphenyl, trifluoromethylphenyl, nitro phenyl, 2,4-methoxychlorophenyl, etc.

In the present application, the term "alkylformamido" generally refers to a -C(=O)NRR' group, wherein R can be hydrogen, optionally substituted alkyl, optionally substituted alkenyl, optionally substituted alkynyl, optionally substituted cycloalkyl, optionally substituted aryl or optionally substituted heteroaryl, and R' is optionally substituted alkyl, optionally substituted alkenyl, optionally substituted alkynyl or optionally substituted cycloalkyl; or R and R' together with the nitrogen atom to which they are bound form an optionally substituted heterocyclyl.

In the present application, the term "alkoxy" generally refers to a -OR group, wherein R can be optionally substituted alkyl, optionally substituted alkenyl, optionally substituted alkynyl, optionally substituted cycloalkyl, optionally substituted aryl, or optionally substituted heteroaryl.

In the present application, the term "alkenyl" generally refers to a linear or branched hydrocarbon group containing at least one carbon-carbon double bond. The non-limiting examples thereof can comprise ethenyl, as well as linear or branched propenyl or butenyl. The alkenyl can have, e.g., 2-20 carbon atoms, 2-10 carbon atoms, and/or 2-6 carbon atoms. For example, the alkenyl group can be optionally substituted, e.g., with hydroxyl (-OH), oxo (=O), halogen, aryl, heteroaryl, cycloalkyl, heterocycloalkyl, amino, nitro (NOz), or thio.

In the present application, the term "heteroaryl" generally refers to an aromatic heterocyclyl. Examples can comprise an aromatic ring containing at least one heteroatom selected from the group consisting of O, N and S. The heteroaryl can comprise 5-12 ring atoms. The heteroaryl can be a 5-6-membered monocyclic heteroaryl or an 8-12-membered bicyclic heteroaryl. The 5-membered monocyclic heteroaryl ring can comprise two double bonds and 1, 2, 3, or 4 heteroatoms as ring atoms, e.g., furyl, imidazolyl, isoxazolyl, isothiazolyl, oxadiazolyl, oxazolyl, pyrazolyl, pyrrolyl, tetrazolyl, thiadiazolyl, thiazolyl, thienyl, and triazolyl. The 6-membered heteroaryl ring can comprise three double bonds and 1, 2, 3, or 4 heteroatoms as ring atoms, e.g., pyridyl, pyridazinyl, pyrimidinyl, pyrazinyl, and triazinyl. Bicyclic heteroaryl is an 8-12-membered ring system having a monocyclic heteroaryl fused with an aromatic, saturated, or partially saturated carbocycle, or fused with a second monocyclic heteroaryl ring. For example, benzofuryl, benzoxadiazolyl, 1, 3-benzothiazolyl, benzimidazolyl, benzothienyl, indolyl, indazolyl, isoquinolinyl, naphthyridyl, oxazolopyridyl, and quinolyl. The heteroaryl group can be attached to a parent molecule via any substitutable carbon atom or any substitutable nitrogen atom contained in the group.

In the present application, the term "alkylene" generally refers to an alkyl having a substituent. For example, the term "alkylene heterocycloalkyl" generally refers to an alkyl substituted with heterocycloalkyl. The alkylene can have, e.g., 1-20 carbon atoms, 1-10 carbon atoms, and/or 1-6 carbon atoms. The alkylene group can be optionally substituted, e.g., with hydroxyl (-OH), oxo (=O), halogen, aryl, heteroaryl, cycloalkyl, heterocycloalkyl, amino, nitro (NOz), or thio

In the present application, the term "alkenylene" generally refers to an alkenyl having a substituent. For example, the term "alkenylene heterocycloalkyl" generally refers an alkenyl substituted with heterocycloalkyl. The alkenylene can have, e.g., 2-20 carbon atoms, 2-10 carbon atoms, and/or 2-6 carbon atoms. The alkenylene can be optionally substituted, e.g., with hydroxyl (-OH), oxo (=O), halogen, aryl, heteroaryl, cycloalkyl, heterocycloalkyl, amino, nitro (NOz), or thio.

In the present application, the term "alkynylene" is defined similarly to "alkylene", except that the group comprises at least one carbon-carbon triple bond, which generally refers to an alkynyl having a substituent. For example, the term "alkynylene heterocycloalkyl" generally refers to an alkynyl substituted with heterocycloalkyl. The alkynylene can have, e.g., 2-20 carbon atoms, 2-10 carbon atoms, and/or 2-6 carbon atoms. The alkynylene can be optionally substituted, e.g., with hydroxyl (-OH), oxo (=O), halogen, aryl, heteroaryl, cycloalkyl, heterocycloalkyl, amino, nitro (NOz), or thio.

In the present application, the term "halogen" refers to a halogen of Group VIIA in the periodic table, e.g., F, Cl, Br and I.

In the present application, the term "amino" generally refers to a -NH group. In the group, one or more hydrogen atoms can be optionally replaced, e.g., with alkyl, substituted alkyl, cycloalkyl, aryl, or heteroaryl.

In the present application, the term "thio" generally refers to a -SH group. In the group, the hydrogen atom can be optionally replaced, e.g., with alkyl, substituted alkyl, cycloalkyl, aryl, or heteroaryl.

In the present application, the term "acylamino" generally refers to a -NHC(=O)R group, wherein the hydrogen atom can be optionally substituted, e.g., with alkyl, substituted alkyl, cycloalkyl aryl or heteroaryl, and R can be optionally substituted alkyl, optionally substituted alkenyl, optionally substituted alkynyl, optionally substituted cycloalkyl, optionally substituted aryl or optionally substituted heteroaryl.

In the present application, the term "pharmaceutical combination" generally refers to a combination comprising at least two active ingredients/therapeutic agents. In some embodiments, various active ingredients/therapeutic agents can each be formulated into separated preparations (solid, liquid, gel, or the like); in some embodiments, various active ingredients/therapeutic agents can each be present in different containers, and can be simultaneously or separately formulated with a suitable carrier to a desired preparation as required; in some embodiments, various active ingredients/therapeutic agents can be derived from different sources (e.g., prepared, produced, or sold by different manufacturers); and in some embodiments, various active ingredients/therapeutic agents can be mixed to form a pharmaceutical composition.

In the present application, the term "pharmaceutical composition" generally refers to a mixture comprising at least two active ingredients that are administered to a subject to treat a particular disease or disorder affecting the subject. It allows the active ingredients to be in an effective form and does not comprise additional ingredients which are unacceptably toxic to the subject to which the composition is to be administered. The composition can be sterile, and can comprise a pharmaceutically acceptable carrier.

In the present application, the term "inhibitor" generally refers to a compound/substance or a composition capable of completely or partially preventing or reducing the physiological functions of one or more particular biomolecules (e.g., proteins, polypeptides, lipopolysaccharides, glycoproteins, ribonucleoprotein complexes, or the like). The reduction of the physiological functions of one or more particular proteins can comprise the reduction of the activity of the protein itself (e.g., the ability of bonding to other molecules, or the like) or the reduction of the amount of the protein itself. For example, the inhibitor can be present as different crystals, amorphous substance, pharmaceutically acceptable salts, hydrates, and solvates. For example, the inhibitor can block the activation of cell signaling pathways.

In the present application, the term "functional variant" generally means that the functional variant can comprise molecules that are subject to amino acid modification (e.g., group substitution or the like) in the original protein, or insertion, substitution, and/or deletion of one or more amino acids in the original protein sequence while reserving the functional of the original sequence. For example, the functional variant can have better biological activity (function) than that of the original sequence. For example, the reservation need not be a full reservation. For example, the functional variant can substantially reserve the function of the original sequence, e.g., reserving at least 50%, 60%, 65%, 70%, 75%, 80%, 85%, 90%, 95%, 96%, 97%, 98%, or 99% of the function of the original sequence.

In the present application, the term "TCF/LEF" generally refers to a set of transcription factors capable of binding to DNA via the HMG region, or a functional variant thereof. TCF/LEF can participate in the Wnt signaling pathway, recruit β-catenin to enhancer elements of its target gene, and can also recruit co-repressor Groucho members to hinder the target gene expression. A wide range of LEF/TCF transcription factors from a number of species, including, e.g., human TCF-1 (Accession No. P36402), TCF-3, mouse TCF1 (Q00417), avian TCF1, mouse TCF3 (CAA11070), Xenopus TCF3, human TCF-4 (CAA72166), mouse TCF4 (CAA11071), human LEF-1 (A39625), mouse LEF1 (P27782) are known.

In the present application, the term "β-catenin", also known as beta-catenin, generally refers to a dual-functional protein capable of participating in the regulation of intercellular adhesion and gene transcription, or functional variants thereof. The β-catenin can act as a subunit of cadherin complex, and can also play a role in intercellular signal transduction in the Wnt signaling pathway. A wide range of β-catenin from a number of species, including, e.g., human Plagloglobin (Accession No. NP_002221) mouse β-catenin (S35091), African Xenopus β-catenin (AAA49670), etc. are known.

In the present application, the term "TCF/β-catenin complex" generally refers to a transcriptionally active complex formed by an interaction between the LEF/TCF and the β-catenin. It can regulate the transcription of the target gene. Examples comprise a complex formed by a β-catenin originated from various species with the LEF/TCF, e.g., a complex formed between the human β-catenin and the human TCF-1 (Accession No. P36402), human TCF-4 (CAA72166), human LEF-1 (A39625), or human TCF-3.

In the present application, the term "β-catenin/CBP complex" generally refers to a molecular complex formed by an interaction between a CBP and the β-catenin. CBP, also known as CREB binding protein (CREBBP), generally refers to a protein comprising a nuclear receptor interaction domain (RID), KIX domain (an interaction domain of CREB and MYB), cysteine/histidine region (TAZ1/CH1 and TAZ2/CH3) and interferon response binding domain (IBiD) or a functional variant. It can function by the activation of transcription factors. In human, the CBP is encoded by the CREBBP gene (NCBI Gene ID: 12914).

In the present application, the term "effective amount" or "effective dose" generally refers to an amount sufficient to achieve or at least partially achieve the desired effect. "A therapeutically effective amount" or "a therapeutically effective dose" of a drug or therapeutic agent generally refers to any amount of drug facilitating disease regression (which is commonly demonstrated by the reduction in the severity of disease symptoms, the increase of frequency and duration in the asymptomatic period of disease, or the prevention of impairment or disability caused by the disease) when the drug or therapeutic agent is used alone or in combination with another therapeutic agent. "A prophylactically effective amount" or "a prophylactically effective dose" of drug generally refers to an amount of drug inhibiting the development or recurrence of disease when the drug is administered alone or in combination with another therapeutic agent to a subject at a risk of disease development or recurrence. Many methods that are known to those skilled in the art, such as, in a human subject during clinical trials or an animal model system, or by measuring the activity of agent in an *in vitro* assay, can be used to estimate the capacity of a therapeutic or prophylactic agent to facilitate the disease regression or inhibit the disease development or recurrence.

In the present application, the term "ascorbic acid derivative" generally refers to a compound that releases ascorbic acid (vitamin C) *in vivo* or *in vitro* and solvates, hydrates, and salts thereof. This term further comprises ascorbic acid analogs wherein one or more hydroxyl groups in the ascorbic acid are replaced with another moiety, and wherein the ascorbic acid analog substantially reverses the stable activity of ascorbic acid *in vitro* or *in vivo.*

In the present application, the term "container" generally refers to any vessel or device adapted to hold a drug. Examples can comprise drug boxes, drug bottles, drug bags, blisters, tubes, syringes, etc.

In the present application, the term "administer/administration" generally refers to introducing the pharmaceutical combination via any introduction or delivery route into the body of the subject. Any method for contacting cells, organs, or tissues with the pharmaceutical combination that is known to persons skilled in the art can be used. It comprises, but is not limited to, intra-arterial, intranasal, intraperitoneal, intravenous, intramuscular, subcutaneous, transdermal or oral administration. Daily dose can be divided into one, two, or more doses in a suitable form to be administered at one, two, or more time points during a certain period of time.

In the present application, the term "subject" generally refers to a human or a non-human animal, including, but not limited to, cat, dog, horse, pig, cow, sheep, rabbit, mouse, rat, or monkey, etc.

In the present application, the term "carrier" generally refers to any other substance in addition to the active ingredients. Examples can comprise a pharmaceutically acceptable substance, composition, or vehicle involving carrying or transferring a chemical agent. Examples can comprise buffers, surfactants, stabilizers, preservatives, adsorption-promoting agents for enhancing the bioavailability, liquid or solid filling agents, diluents, excipients, solvents, coating materials, and/or other traditional co-solvents or dispersants, etc. Various carriers must be "acceptable" in a sense of compatibility with other ingredients of the preparation, and would not cause any damage to the patient.

In the present application, the term "preparation", also known as pharmaceutical preparation, generally refers to a drug that is prepared in accordance with certain requirement of dosage form and can be provided to a subject for use to meet the requirements of treatment or prevention. For example, the preparation can comprise active ingredients and carriers.

In the present application, the term "treat/treatment" generally refers to prophylactically administering a combination to a healthy subject to prevent the occurrence of a disease or disorder. It can further comprise prophylactically administering a combination to a patient with a pre-allergic disease to be treated. "Prevention" does not require 100% elimination of the likelihood of a disease or condition, in other words, the "prevention" generally refers to a reduction in the likelihood or degree of occurrence of a disease or condition in the presence of the administered combination.

In the present application, the term "treat/treating" generally refers to a clinical intervention for altering a natural process of the treated individual or cells during the clinical pathological process. It can comprise improving disease status, eliminating lesions, or improving prognosis.

In the present application, the term "tumor" generally refers to a neoplasm or solid lesion formed by abnormal cell growth. In the present application, the tumors can be solid tumors or non-solid tumors. For example, a tangible mass that can be detected by clinical examination, such as, X-ray, CT scan, B-ultrasound, or palpation can be called a solid tumor, and a tumor that cannot be seen or detected by X-ray, CT scan, B-ultrasound or palpation can be called a non-solid tumor, such as, leukemia.

In the present application, the term "comprise/comprising" or "include/including" generally refers to including explicitly specified features, but not excluding other elements.

In the present application, the term "about" generally refers to a variation within 0.5%-10% of the specified value, e.g., a variation within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of the specified value. In the present application, as those skilled in the art understand, the terms such as "alkyl", "alkenyl", "cycloalkyl" or the like can be preceded with a label for indicating the number of atoms in the group under certain conditions, e.g., C₁₋₂₀ alkyl, C₃₋₂₀ cycloalkyl, C₂₋₂₀ heterocycloalkyl, etc., wherein the number in the subscript following "C" represents the number of carbon atoms present in the group. For example, C₃ alkyl refers to an alkyl having 3 carbon atoms (e.g., n-propyl, isopropyl); and in C₁₋₂₀, the members of the group can have any number of carbon atoms falling within a range of 1-4.

### DETAILED DESCRIPTION

In an aspect, the present application provides a pharmaceutical combination comprising:
a) a prophylactically and/or therapeutically effective amount of ascorbic acid or a derivative thereof; and
b) a prophylactically and/or therapeutically effective amount of a substance affecting the formation of a TCF/β-catenin complex.

In another aspect, the present application further provides use of a combination of ascorbic acid or a derivative thereof with a substance affecting the formation of a TCF/β-catenin complex in the manufacture of a drug for preventing and/or treating tumors.

In another aspect, the present application further provides a method for preventing and/or treating tumors comprising administering to a subject in need thereof:
a) ascorbic acid or a derivative thereof; and
b) a substance affecting the formation of a TCF/β-catenin complex.

In another aspect, the present application further provides a method for monitoring a response to a drug in a subject comprising:
a) administering to the subject ascorbic acid or a derivative thereof and a substance affecting the formation of a TCF/β-catenin complex; and
b) detecting a change in one or more of the level and/or activity of blood alkaline phosphatase, the level and/or activity of lactic dehydrogenase, the expression level of CADM1 gene, the expression level of CD44 gene, the expression level of livin gene, the expression level of HIF-1α gene, the expression level of ET-1 gene, the expression level of IGF-1R gene, the expression level of STAT3 gene, the expression level of CEA gene, the expression level of CA199 gene, the expression level of CA125 gene, the expression level of AFP gene, the expression level of CA724 gene, and the expression level of β-HCG gene in the subject after the administration of a).

In another aspect, the present application further provides a kit comprising the pharmaceutical combination as described in the present application.

### Pharmaceutical Combination

The pharmaceutical combination as described in the present application comprises: a) a prophylactically and/or therapeutically effective amount of ascorbic acid or a derivative thereof as described in the present application; and b) a prophylactically and/or therapeutically effective amount of a substance affecting the formation of a TCF/β-catenin complex as described in the present application.

For example, the substance affecting the formation of a TCF/β-catenin complex can comprise a substance capable of interacting with a member involved in the formation of a TCF/β-catenin complex and/or disrupting the interaction between members to hinder the formation of a TCF/β-catenin complex. For example, the members can comprise TCF, β-catenin, and CBP. For example, the TCF can comprise TCF-1, TCF-3, TCF-4, and LEF-1. For example, the TCF/β-catenin complex can comprise TCF-1/β-catenin complex, TCF-3/β-catenin complex, TCF-4/β-catenin complex, and LEF-1/β-catenin complex.

For example, the ascorbic acid or a derivative thereof can comprise ascorbic acid, ascorbic acid derivative, or any combination thereof.

For example, the substance affecting the formation of a TCF/β-catenin complex can comprise a TCF/LEF inhibitor, a substance affecting the formation of a β-catenin/CBP complex, a β-catenin inhibitor, or a compound having a structure of Formula I, II, or III or a pharmaceutically acceptable salt or hydrate thereof: 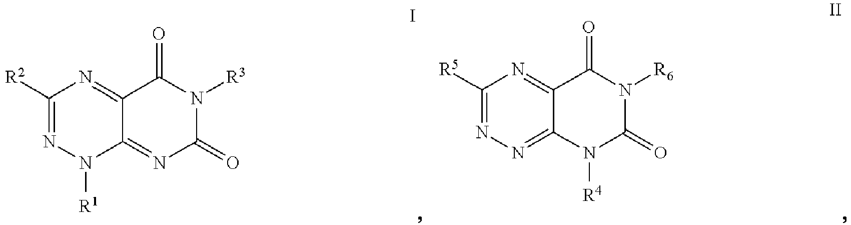 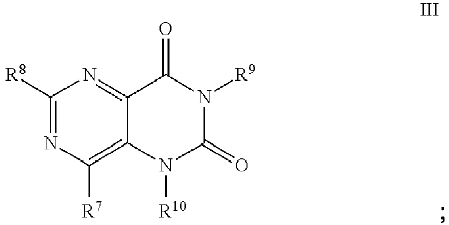 (wherein R¹ and R³ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R² is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl; R⁴ and R⁶ are each independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R⁵ is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl, optionally substituted heteroaryl, optionally substituted C₁₋₂₀ alkylamino, optionally substituted C₁₋₂₀ alkoxy and optionally substituted C₁₋₂₀ alkylformamido; R⁷, R⁸, R⁹, and R¹⁰ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl).

For example, the R² can be C₁₋₆ alkyl, *e.g.,* C₁₋₄ alkyl.

For example, the R² can be C₄₋₆ alkyl.

For example, the R² can be C₄ alkyl.

For example, the R² can be isobutyl.

For example, the R¹ can be selected from the group consisting of hydrogen and C₁₋₆ alkyl.

For example, the R¹ can be C₃ alkyl.

For example, the R¹ can be isopropyl.

For example, the R³ can be selected from the group consisting of hydrogen and C₁₋₆ alkyl.

For example, the R³ can be methyl.

For example, the substance affecting the formation of a TCF/β-catenin complex can comprise a TCF/LEF inhibitor.

For example, the substance affecting the formation of a TCF/β-catenin complex can comprise a substance affecting the formation of a β-catenin/CBP complex.

For example, the substance affecting the formation of a TCF/β-catenin complex can comprise a β-catenin inhibitor.

For example, the substance affecting the formation of a TCF/β-catenin complex can comprise a compound having a structure of Formula I, II, or III below, or a pharmaceutically acceptable salt or hydrate thereof: 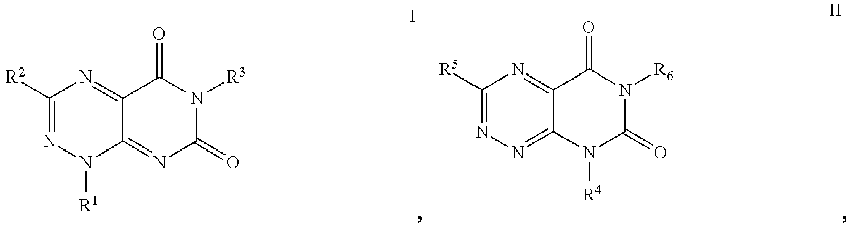 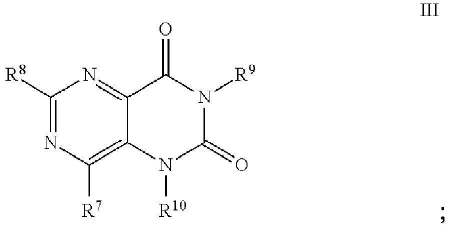
wherein, R¹ and R³ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R² is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl;
R⁴ and R⁶ are each independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R⁵ is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl, optionally substituted heteroaryl, optionally substituted C₁₋₂₀ alkylamino, optionally substituted C₁₋₂₀ alkoxy and optionally substituted C₁₋₂₀ alkylformamido;
R⁷, R⁸, R⁹, and R¹⁰ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl.

For example, the R² can be C₁₋₆ alkyl, *e.g.,* C₁₋₄ alkyl.

For example, the R² can be C₄₋₆ alkyl.

For example, the R² can be C₄ alkyl.

For example, the R² can be isobutyl.

For example, the R¹ can be selected from the group consisting of hydrogen and C₁₋₆ alkyl.

For example, the R¹ can be C₃ alkyl.

For example, the R¹ can be isopropyl.

For example, the R³ can be selected from the group consisting of hydrogen and C₁₋₆ alkyl.

For example, the R³ can be methyl.

For example, the ratio of the effective amount of the substance affecting the formation of a TCF/β-catenin complex and to the effective amount of the ascorbic acid or a derivative thereof is about 0.1% to about 50%.

The ratio of the effective amount of the substance affecting the formation of a TCF/β-catenin complex to the effective amount of the ascorbic acid or a derivative thereof is about 0.1% to about 5%, about 0.1% to about 10%, about 0.1% to about 15%, about 0.1% to about 30%, about 0.1% to about 40%, about 10% to about 50%, about 20% to about 50%, about 0.5% to about 50%, about 0.5% to about 25%, about 1% to about 50%, about 5% to about 50%, or about 1% to about 40%.

For example, the ratio of the effective amount of the substance affecting the formation of a TCF/β-catenin complex to the effective amount of the ascorbic acid or a derivative thereof is about 1% to about 10%.

For example, the ratio of effective amount can comprise a molar ratio of effective amount and/or a mass ratio of effective amount.

For example, the mass ratio of the substance affecting the formation of a TCF/β-catenin complex to the ascorbic acid or a derivative thereof is about 1% to about 200%.

For example, the mass ratio of the substance affecting the formation of a TCF/β-catenin complex to the ascorbic acid or a derivative thereof is about 10% to about 100%, about 1% to about 10%, about 10% to about 200%, about 100% to about 200%, about 50% to about 200%, about 10% to about 150%, about 100% to about 150%, about 10% to about 100%, about 30% to about 100%, about 40% to about 160%, about 10% to about 80%, about 50% to about 100%, about 30% to about 90%, about 10% to about 80%, about 10% to about 90%, about 80% to about 100%, about 70% to about 110%, about 80% to about 130%, about 60% to about 120%, about 50% to about 90%, about 5% to about 25%.

The mass ratio of the substance affecting the formation of a TCF/β-catenin complex to the ascorbic acid or a derivative thereof is about 90% to about 99%.

For example, the substance affecting the formation of a TCF/β-catenin complex and the ascorbic acid or a derivative thereof can each be derived from different sources, e.g., prepared, produced, or sold by different manufacturers. For example, the prepared, produced, or sold ascorbic acid or a derivative thereof is not required to be single-component or pure, as long as it contains a compound capable of releasing ascorbic acid (vitamin C) *in vivo* or *in vitro,* or solvates, hydrates, salts thereof, and/or it contains an ascorbic acid analog (e.g., an analog wherein one or more hydroxyl groups in ascorbic acid are replaced with another moiety, and wherein the ascorbic acid analog substantially reserves the stable activity of ascorbic acid *in vivo* or *in vitro),* all of which are encompassed within the scope of the present application. For the same reason, the prepared, produced, or sold substance affecting the formation of a TCF/β-catenin complex are not required to be single-component or pure, as long as it contains a substance capable of releasing one or more of the substances affecting the formation of a TCF/β-catenin complex as described in the present application *in vivo* and *in vitro,* or solvates, hydrates, salts thereof, and/or it contains analog(s) of any one or more of the substance affecting the formation of a TCF/β-catenin complex as described in the present application (e.g., an analog which comprises a group modification or substitution, and substantially reserves the stable activity of any substance affecting the formation of a TCF/β-catenin complex *in vitro* or *in vivo),* all of which are encompassed within the scope of the present application.

For example, the substance affecting the formation of a TCF/β-catenin complex and the ascorbic acid or a derivative thereof are each present in different containers.

For example, the substance affecting the formation of a TCF/β-catenin complex and the ascorbic acid or a derivative thereof can each be present in different containers, and the amount of the substance affecting the formation of a TCF/β-catenin complex and the amount of the ascorbic acid or a derivative thereof can or cannot be set in accordance with the effective amount ratio thereof.

For example, the substance affecting the formation of a TCF/β-catenin complex can each be present in 1 or more containers, e.g., 2 or more, 3 or more, 4 or more, 5 or more, 6 or more, 7 or more, 8 or more, 9 or more, 10 or more, 11 or more, or 12 or more.

For example, the ascorbic acid or a derivative thereof can each be present in 1 or more containers, e.g., 2 or more, 3 or more, 4 or more, 5 or more, 6 or more, 7 or more, 8 or more, 9 or more, 10 or more, 11 or more, 12 or more.

For example, the containers can comprise drug chests, drug boxes, drug bottles, drug bags, blisters, tubes, syringes, or the like; for example, the drug bottle can comprise sealed glass ampules, tubes, vials, flasks, bottles, or the like; for example, the containers can be made from glass, organic polymers like polycarbonates, polystyrenes, etc., or ceramics, metals, composite films, cellophanes, packing materials partially lined with aluminum foil, alloy foil, or the like, as well as any other suitable material commonly used for retaining an agent.

For example, the substance affecting the formation of a TCF/β-catenin complex and the ascorbic acid or a derivative thereof can each be present in different containers, and can be simultaneously or separately formulated with a suitable carrier into preparation as required.

For example, the substance affecting the formation of a TCF/β-catenin complex and the ascorbic acid or a derivative thereof is mixed and formulated into a suitable preparation.

For example, the substance affecting the formation of a TCF/β-catenin complex and the ascorbic acid or a derivative thereof are each formulated into different suitable preparations.

For example, the substance affecting the formation of a TCF/β-catenin complex and the ascorbic acid or a derivative thereof can each be present in a preparation form in different containers.

For example, the pharmaceutical combination comprises a first preparation and a second preparation, wherein the first preparation comprises the ascorbic acid or a derivative thereof and a pharmaceutically acceptable first carrier, and the second preparation comprises the substance affecting the formation of a TCF/β-catenin complex and a pharmaceutically acceptable second carrier.

For example, the first preparation and the second preparation can be in the same dosage form or different dosage forms.

For example, both the first preparation and the second preparation can be any one of pills, powders, tablets, granules, gels, hard capsules, soft capsules, syrups, mixtures, lotions, injections, aerosols, ointments, films, suppositories, drop pills, or the like, or any combination thereof. For example, both the first preparation and the second preparation are tablets. For example, both the first preparation and the second preparation are injections. For example, the first preparation is a tablet, and the second preparation is an injection. For example, the first preparation is an injection, and the second preparation is a tablet.

For example, the first preparation and the second preparation can each be present in preparation forms suitable for the same administration mode. For example, both the first preparation and the second preparation can be tablets, granules, hard capsules, soft capsules, or syrups suitable for oral administration. For example, both the first preparation and the second preparation can be injections for injection administration.

For example, the first preparation and the second preparation can each be present in preparation forms suitable for different administration modes. For example, the first preparation can be a tablet, granule, hard capsule, soft capsule, or syrup suitable for oral administration, and the second preparation can be an injection suitable for injection administration. For example, the first preparation can be an injection suitable for injection administration. And the second preparation can be a tablet, a granule, a hard capsule, a soft capsule, or a syrup for oral administration.

For example, the pharmaceutical combination comprises a pharmaceutical composition, and the pharmaceutical composition comprises a substance affecting the formation of a TCF/β-catenin complex and ascorbic acid or a derivative thereof.

For example, the substance affecting the formation of a TCF/β-catenin complex has a content of about 1% to 50% (w/w) in the pharmaceutical composition, e.g., about 5% (w/w) to about 50% (w/w), about 10% (w/w) to about 40% (w/w), about 20% (w/w) to about 50% (w/w), about 10% (w/w) to about 30% (w/w), about 20% (w/w) to about 40% (w/w), about 1% (w/w) to about 5% (w/w). About 30% (w/w) to about 50% (w/w), about 5% to about 20% (w/w), about 20% (w/w) to about 30% (w/w), or about 5% to 20% (w/w).

For example, the substance affecting the formation of a TCF/β-catenin complex in the pharmaceutical composition has a content of about 5% to about 20% (w/w).

For example, the ascorbic acid or a derivative thereof has a content of about 30% to 95% (w/w) in the pharmaceutical composition.

For example, the ascorbic acid or a derivative thereof has a content of about 30% (w/w) to about 60% (w/w), about 50% (w/w) to about 60% (w/w), about 50% (w/w) to about 70% (w/w), about 60% (w/w) to about 80% (w/w), about 50% (w/w) to about 90% (w/w), about 60% (w/w) to about 90% (w/w), about 55% (w/w) to about 75% (w/w). About 70% (w/w) to about 90% (w/w), about 80 to about 95% (w/w).

For example, the ascorbic acid or a derivative thereof has a content of about 80% to 95% (w/w) in the pharmaceutical composition.

### Use and Method

The present application further provides use of a combination of ascorbic acid or a derivative thereof with a substance affecting the formation of a TCF/β-catenin complex in the manufacture of a drug.

The present application further provides a method for preventing and/or treating tumors comprising administering to a subject in need thereof:
a) ascorbic acid or a derivative thereof; and
b) a substance affecting the formation of a TCF/β-catenin complex.

The present application further provides a method for monitoring a response to a drug in a subject comprising:
a) administering to the subject ascorbic acid or a derivative thereof and a substance affecting the formation of a TCF/β-catenin complex; and
b) detecting a change in one or more of the level and/or activity of blood alkaline phosphatase, the level and/or activity of lactic dehydrogenase, the expression level of CADM1 gene, the expression level of CD44 gene, the expression level of livin gene, the expression level of HIF-1α gene, the expression level of ET-1 gene, the expression level of IGF-1R gene, the expression level of STAT3 gene, the expression level of CEA gene, the expression level of CA199 gene, the expression level of CA125 gene, the expression level of AFP gene, the expression level of CA724 gene, and the expression level of β-HCG gene in the subject after the administration of a).

For example, the expression level of gene can comprise the modification and/or mutation of genomic DNA, the expression level of mRNA, and the expression level of protein.

For example, the detection can be selected from the group consisting of PCR, real-time quantitative PCR, digital PCR, liquid chip, solid chip, hybridization *in situ,* normal sequencing and high-throughput sequencing for detecting the modification and/or mutation of genomic DNA, and the expression level of mRNA.

For example, the detection can be selected from the group consisting of liquid chip, solid chip, ELISA, radio-immunity, chemiluminescence immune assay, mass spectrum, HPLC, Western blotting and sequencing for detecting the expression level of protein.

For example, the substance affecting the formation of a TCF/β-catenin complex can comprise a substance capable of interacting with a member involved in the formation of a TCF/β-catenin complex and/or disrupting the interaction between members to hinder the formation of a TCF/β-catenin complex. For example, the members can comprise TCF, β-catenin, and CBP. For example, the TCF can comprise TCF-1, TCF-3, TCF-4, and LEF-1. For example, the TCF/β-catenin complex can comprise TCF-1/β-catenin complex, TCF-3/β-catenin complex, TCF-4/β-catenin complex, and LEF-1/β-catenin complex.

For example, the ascorbic acid or a derivative thereof can comprise ascorbic acid, a derivative of ascorbic acid, or any combination thereof.

For example, the substance affecting the formation of a TCF/β-catenin complex can comprise a TCF/LEF inhibitor, a substance affecting the formation of a β-catenin/CBP complex, a β-catenin inhibitor, or a compound having a structure of Formula I, II, or III or a pharmaceutically acceptable salt or hydrate thereof: 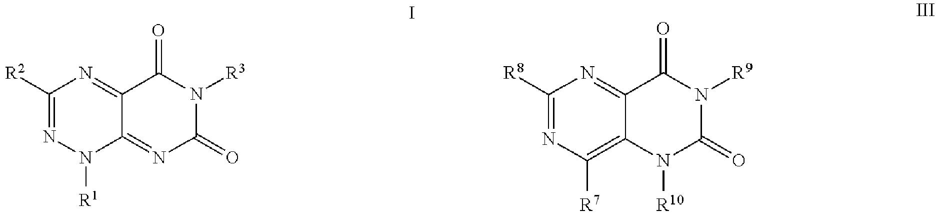 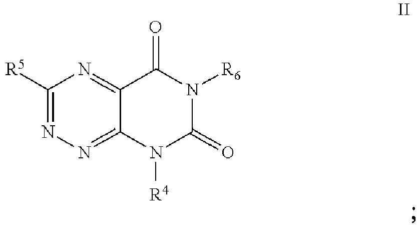 (wherein R¹ and R³ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R² is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl; R⁴ and R⁶ are each independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R⁵ is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀cycloalkyl, optionally substituted C₂₋₂₀ heterocycloalkyl, optionally substituted aryl, optionally substituted heteroaryl, optionally substituted C₁₋₂₀ alkylamino, optionally substituted C₁₋₂₀ alkoxy and optionally substituted C₁₋₂₀ alkylformamido; R⁷, R⁸, R⁹, and R¹⁰ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl).

For example, the R² can be C₁₋₆ alkyl, *e.g.,* C₁₋₄ alkyl.

For example, the R² can be C₄₋₆ alkyl.

For example, the R² can be C₄ alkyl.

For example, the R² can be isobutyl.

For example, the R¹ can be selected from the group consisting of hydrogen and C₁₋₆ alkyl.

For example, the R¹ can be C₃ alkyl.

For example, the R¹ can be isopropyl.

For example, the R³ can be selected from the group consisting of hydrogen and C₁₋₆ alkyl.

For example, the R³ can be methyl.

For example, the tumors comprise solid tumors or non-solid tumors.

For example, the solid tumors comprise osteosarcoma and/or colon cancer.

For example, the non-solid tumors comprise lymphoma.

For example, the substance affecting the formation of a TCF/β-catenin complex and the ascorbic acid or a derivative thereof are formulated to be simultaneously administered to a subject.

For example, the simultaneous administration to the subject can comprise administering the substance affecting the formation of a TCF/β-catenin complex and the ascorbic acid or a derivative thereof to the subject at a time interval of no more than 1 hr, *e.g.,* at a time interval of 60 min, 55 min, 50 min, 45 min, 40 min, 35 min, 30 min, 25 min, 20 min, 15 min, 10 min, 8 min, 5 min, 3 min, 2 min, or 1 min; or they can be mixed together for administration.

For example, the substance affecting the formation of a TCF/β-catenin complex and the ascorbic acid or a derivative thereof are formulated to be separately administered.

For example, the separate administration to the subject can comprise administering the substance affecting the formation of a TCF/β-catenin complex and the ascorbic acid or a derivative thereof to the subject at a time interval of more than 1 hour, e.g., a time interval of 1.5 hr, 2 hr, 2.5 hr, 3 hr, 3.5 hr, 4 hr, 4.5 hr, 5 hr, 5.5 hr, 6 hr, 6.5 hr, 7 hr, 7.5 hr, 8 hr, 9 hr, 10 hr, 11 hr, 12 hr, 15 hr, 18 hr, 21 hr, 24 hr, 2 days, 3 days, 4 days, 5 days, 6 days, 7 days, or longer.

For example, the administration can comprise any one of oral administration, intravenous administration, intramuscular administration, *in situ* administration at tumor site, inhalation, rectal administration, transdermal administration, or subcutaneous depot administration, or any combination thereof.

For example, the separate administration to the subject can comprise alternately administering the substance affecting the formation of a TCF/β-catenin complex and the ascorbic acid or a derivative thereof, e.g., administering the substance affecting the formation of a TCF/β-catenin complex, and then administering the ascorbic acid or a derivative thereof; or administering the ascorbic acid or a derivative thereof, and then administering the substance affecting the formation of a TCF/β-catenin complex.

For example, the administration of the substance affecting the formation of a TCF/β-catenin complex can comprise one administration.

For example, the administration of the substance affecting the formation of a TCF/β-catenin complex can comprise 2 or more continuous administrations, e.g., 3 or more, 4 or more, 5 or more, 6 or more, 7 or more, 8 or more, 9 or more, 10 or more.

For example, the administration of the ascorbic acid or a derivative thereof can comprise one administration.

For example, the administration of the ascorbic acid or a derivative thereof can comprise 2 or more continuous administrations, e.g., 3 or more, 4 or more, 5 or more, 6 or more, 7 or more, 8 or more, 9 or more, 10 or more.

For example, the drug is formulated so that the substance affecting the formation of a TCF/β-catenin complex is administered at a dose of about 0.05 mg/kg to about 40 mg/kg.

For example, the substance affecting the formation of a TCF/β-catenin complex is administered at a dose of about 1 mg/kg to about 30 mg/kg, about 1 mg/kg to about 20 mg/kg, about 1 mg/kg to about 40 mg/kg, about 5 mg/kg to about 20 mg/kg, about 10 mg/kg to about 40 mg/kg, about 5 mg/kg to about 30 mg/kg, about 0.1 mg/kg to about 30 mg/kg, about 0.5 mg/kg to about 20 mg/kg, about 0.5 mg/kg to about 30 mg/kg, about 10 mg/kg to about 30 mg/kg, or about 20 mg/kg to about 40 mg/kg.

For example, the administration subject is a mouse. For example, the drug is formulated so that the substance affecting the formation of a TCF/β-catenin complex is administrated at a dose of about 0.5 mg/kg to about 40 mg/kg.

For example, the administration subject is human. For example, the drug is formulated so that the substance affecting the formation of a TCF/β-catenin complex is administered at a dose of about 0.05 mg/kg to about 4 mg/kg.

For example, the drug is formulated so that the ascorbic acid or a derivative thereof are administered at a dose of about 0.05 g/kg to about 2.5 g/kg.

For example, the ascorbic acid or a derivative thereof are administered at a dose of about 0.05 g/kg to about 0.1 g/kg, about 0.1 g/kg to about 0.2 g/kg, about 0.15 g/kg to about 0.25 g/kg, about 0.05 g/kg to about 0.15 g/kg, about 0.05 g/kg to about 2.5 g/kg, about 0.25 g/kg to about 1 g/kg, about 0.35 g/kg to about 0.5 g/kg, about 0.5 g/kg to about 1 g/kg, about 0.5 g/kg to about 2.5 g/kg, about 0.5 g/kg to about 2 g/kg, about 1 g/kg to about 2 g/kg, about 1 g/kg to about 2.5 g/kg, about 1.5 g/kg to about 2.5 g/kg, about 0.1 g/kg to about 2.5 g/kg, about 0.5 g/kg to about 2 g/kg, or about 0.05 g/kg to about 0.2 g/kg.

For example, the administration subject is a mouse. For example, the drug is formulated so that the ascorbic acid or a derivative thereof are administered at a dose of about 0.5 g/kg to about 2.5 g/kg.

For example, the administration subject is human. For example, the drug is formulated so that the ascorbic acid or a derivative thereof are administered at a dose of about 0.05 g/kg to about 0.25 g/kg.

For example, the drug is formulated so that the substance affecting the formation of a TCF/β-catenin complex and the ascorbic acid or a derivative thereof are administered at a mass ratio of about 1:5 to about 1 :5000.

For example, the drug is formulated so that the substance affecting the formation of a TCF/β-catenin complex and the ascorbic acid or a derivative thereof are administered at a mass ratio of about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1000, about 1:10 to about 1:1500, about 1:10 to about 1:2000, about 1:10 to about 1:3000, about 1:10 to about 1:4000, about 1:10 to about 1:5000, about 1:5 to about 1:100, about 1:5 to about 1:150, about 1:5 to about 1:200, about 1:25 to about 1:100, about 1:25 to about 1:150, about 1:25 to about 1:200, about 1:25 to about 1:1000, about 1:25 to about 1:5000, 1:30 to about 1:5000, about 1:50 to about 1:100, about 1:50 to about 1:150, about 1:50 to about 1:200, about 1:50 to about 1:300, about 1:50 to about 1:500, about 1:50 to about 1:1000, about 1:50 to about 1:2000, about 1:50 to about 1:4000 or about 1:50 to about 1:5000, or about 1:4500 to about 1:5000.

For example, the drug is formulated so that the substance affecting the formation of a TCF/β-catenin complex and the ascorbic acid or a derivative thereof are administered at a mass ratio of about 1:30 to about 1:5000.

For example, the drug is formulated so that the substance affecting the formation of a TCF/β-catenin complex and the ascorbic acid or a derivative thereof are administered at a mass ratio of about 1:300 to about 1:5000, about 1:300 to about 1:3000, about 1:500 to about 1:5000, about 1:200 to about 1:2000, about 1:300 to about 1:1000, about 1:1000 to about 1:2000, about 1:1000 to about 1:3000, about 1:100 to about 1:1000, about 1:100 to about 1:500, about 1:100 to about 1:2000, or about 1:200 to about 1:3000.

For example, the drug is formulated so that the substance affecting the formation of a TCF/β-catenin complex and the ascorbic acid or a derivative thereof are administered at an effective amount ratio of about 1:5 to about 1:5000.

For example, the drug is formulated so that the substance affecting the formation of a TCF/β-catenin complex and the ascorbic acid or a derivative thereof are administered at an effective amount ratio of about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1000, about 1:10 to about 1:1500, about 1:10 to about 1:2000, about 1:10 to about 1:3000, about 1:10 to about 1:4000, about 1:10 to about 1:5000, about 1:5 to about 1:100, about 1:5 to about 1:150, about 1:5 to about 1:200, about 1:25 to about 1:100, about 1:25 to about 1:150, about 1:25 to about 1:200, about 1:25 to about 1:1000, about 1:25 to about 1:5000, 1:30 to about 1:5000, about 1:50 to about 1:100, about 1:50 to about 1:150, about 1:50 to about 1:200, about 1:50 to about 1:300, about 1:50 to about 1:500, about 1:50 to about 1:1000, about 1:50 to about 1:2000, about 1:50 to about 1:4000 or about 1:50 to about 1:5000, or about 1:4500 to about 1:5000.

For example, the drug is formulated so that the substance affecting the formation of a TCF/β-catenin complex and the ascorbic acid or a derivative thereof are administered at an effective amount ratio of 1:30 to about 1:5000.

For example, the drug is formulated so that the substance affecting the formation of a TCF/β-catenin complex and the ascorbic acid or a derivative thereof are administered at an effective amount ratio of about 1:300 to about 1:5000, about 1:300 to about 1:3000, about 1:500 to about 1:5000, about 1:200 to about 1:2000, about 1:300 to about 1:1000, about 1:1000 to about 1:2000, about 1:1000 to about 1:3000, about 1:100 to about 1:1000, about 1:100 to about 1:500, about 1:100 to about 1:2000, or about 1:200 to about 1:3000.

### Substance Affecting the Formation of a TCF/β-Catenin Complex

In the present application, the substance affecting the formation of a TCF/β-catenin complex can comprise a substance capable of interacting with a member involved in the formation of a TCF/β-catenin complex and/or disrupting the interaction between members to hinder the formation of a TCF/β-catenin complex.

For example, the substance affecting the formation of a TCF/β-catenin complex can comprise a TCF/LEF inhibitor.

For example, the TCF/LEF inhibitor comprises NCB-0846.

For example, the substance affecting the formation of a TCF/β-catenin complex can comprise the substance affecting the formation of a β-catenin/CBP complex.

For example, the substance affecting the formation of a β-catenin/CBP complex comprises PRI-724.

For example, the substance affecting the formation of a TCF/β-catenin complex can comprise a β-catenin inhibitor.

For example, the substance affecting the formation of a TCF/β-catenin complex can comprise BI-D1870 and/or Lumazine.

For example, the substance affecting the formation of a TCF/β-catenin complex comprises a compound having a structure of Formula I, II, or III below, or a pharmaceutically acceptable salt or hydrate thereof: 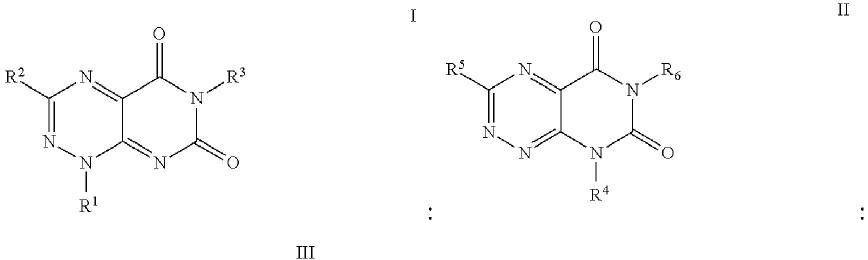 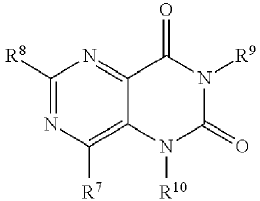
wherein R¹ and R³ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl, and
R² is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl, and when R¹ and R³ are both methyl, R² is not selected from hydrogen;
R⁴ and R⁶ are each independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl, and
R⁵ is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl, optionally substituted heteroaryl, optionally substituted C₁₋₂₀ alkylamino, optionally substituted C₁₋₂₀ alkoxy, and optionally substituted C₁₋₂₀ alkylformamido;
R⁷, R⁸, R⁹, and R¹⁰ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl.

For example, the R² can be C₁₋₆ alkyl, *e.g.,* C₁₋₄ alkyl.

For example, the R² can be C₄₋₆ alkyl.

For example, the R² can be C₄ alkyl.

For example, the R² can be isobutyl.

For example, the R¹ can be selected from the group consisting of hydrogen and C₁₋₆ alkyl.

For example, the R¹ can be C₃ alkyl.

For example, the R¹ can be isopropyl.

For example, the R³ can be selected from the group consisting of hydrogen and C₁₋₆ alkyl.

For example, the R³ can be methyl.

For example, R⁴ can be selected from the group consisting of hydrogen, C₁₋₂₀ alkyl, C₁₋₂₀ alkylene-OH, C₁₋₂₀ alkylene-NR^{a}R^{b}, optionally substituted C₁₋₂₀ alkylene-aryl and optionally substituted C₁₋₂₀ alkylene-heteroaryl, wherein R^{a} and R^{b} are each independently selected from the group consisting of hydrogen, C₁₋₂₀ alkyl, or R^{a} and R^{b} together with the nitrogen atom to which they are bound form an optionally substituted 3-10-membered heterocyclyl.

For example, R⁴ can be selected from the group consisting of methyl, ethyl, propyl, octyl, 2-(N,N-diethylamino)ethyl, phenyl, fluorophenyl, cyclopentyl, cyclopropyl, optionally substituted C₁₋₃ alkylene-aryl, and optionally substituted C₁₋₃ alkylene-heteroaryl.

For example, R⁴ can be selected from the group consisting of benzyl, 2-phenylethyl, 3-phenylpropyl, methylbenzyl, tert-butylbenzyl, fluorobenzyl, difluorobenzyl, dichlorobenzyl, nitro benzyl, trifluoromethylbenzyl and pyridin-3-ylmethyl.

For example, R⁵ can be selected from the group consisting of optionally substituted C₁₋₂₀ alkylene-aryl, R^{d}C(=O)NR^{c}(C₁₋₂₀ alkyl), optionally substituted R^{e}R^{f}N-(C₁₋₂₀ alkylformamido), optionally substituted (C₁₋₂₀ alkylformamido)phenyl, optionally substituted R^{e}R^{f}N-(C₁₋₂₀ alkylamino), optionally substituted R^{e}R^{f}N-(C₁₋₂₀ alkoxy), optionally substituted C₁₋₂₀ alkylene-NR^{e}R^{f}, optionally substituted C₁₋₂₀ alkoxyphenyl and optionally substituted C₁₋₂₀ alkylaminophenyl, wherein R^{c} can be selected from the group consisting of hydrogen, C₁₋₂₀ alkyl, R^{d} can be selected from the group consisting of hydrogen, C₁₋₂₀ alkyl and optionally substituted C₁₋₂₀ amino alkyl, as well as R^{e} and R^{f} can be independently selected from the group consisting of hydrogen, optionally substituted C₁₋₂₀ alkyl and optionally substituted C₁₋₂₀ amino alkyl, or R^{e} and R^{f} together with the nitrogen atom to which they are bound form an optionally substituted 3-10-membered heterocyclyl.

For example, R⁵ can be selected from the group consisting of optionally substituted C₁₋₃ alkylene-aryl, R^{d}C(=O)NR^{c}(C₁₋₃ alkyl), optionally substituted R^{e}R^{f}N-(C₁₋₃ alkylformamido), optionally substituted (C₁₋₃ alkylformamido)phenyl, optionally substituted R^{e}R^{f}N-(C₁₋₃ alkylamino), optionally substituted R^{e}R^{f}N-(C₁₋₃ alkoxy), optionally substituted C₁₋₃ alkylene-NR^{e}R^{f}, optionally substituted C₁₋₃ alkoxyphenyl and optionally substituted C₁₋₃ alkylaminophenyl, wherein R^{c} can be selected from the group consisting of hydrogen and C₁₋₃ alkyl, R^{d} can be selected from the group consisting of hydrogen, C₁₋₃ alkyl and optionally substituted C₁₋₃ amino alkyl, as well as R^{e} and R^{f} can be independently selected from the group consisting of hydrogen, optionally substituted C₁₋₃ alkyl and optionally substituted C₁₋₃ amino alkyl, or R^{e} and R^{f} together with the nitrogen atom to which they are bound form an optionally substituted 3-6-membered heterocyclyl.

For example, R⁵ can be selected from the group consisting of optionally substituted C₁₋₃ alkylene-aryl-NR^{e}R^{f}, R^{e}R^{f}N-RdC(=O)NR^{c} (C₁₋₃ alkyl), optionally substituted R^{e}R^{f}N-(C₁₋₃ alkylformamido)phenyl, optionally substituted R^{e}R^{f}N-(C₁₋₃ alkoxy)phenyl and optionally substituted R^{e}R^{f}N-(C₁₋₃ alkylamino)phenyl, wherein R^{c} can be selected from the group consisting of hydrogen and C₁₋₃ alkyl, R^{d} can be optionally substituted C₁₋₃ alkyl, as well as R^{e} and R^{f} can be independently selected from the group consisting of hydrogen, optionally substituted C₁₋₃ alkyl and optionally substituted C₁₋₃ amino alkyl, or R^{e} and R^{f} together with the nitrogen atom to which they are bound form an optionally substituted 3-6-membered heterocyclyl.

For example, R⁵ can be selected from the group consisting of pyridyl, phenyl, fluorophenyl,
chlorophenyl,hydroxyphenyl,methoxyphenyl,trifluoromethylphenyl, carboxyphenyl,(2-(4-aminoacetylpiperazin-1-yl)ethoxy)phenyl,(2-(4-(dimethylamino)piperidin-1-yl)ethoxy)phenyl,(2-(3 -aminopyrrolidin-1 -yl)ethoxy)phenyl,(3 -(morpholin-4-yl)propoxy)phenyl,(2-(piperidin-1 - yl)ethoxy)phenyl,(2-(dimethylamino)ethyl)phenyl,(2-(piperidin-1-yl)ethylamino)phenyl,(2-(diethylamino)ethylcarboxamido)phenyl,(2-(4-methylpiperazin-1 -yl)ethoxy)phenyl,(3 -(4-methylpiperazin-1-yl)propyl)phenyl,2-(N,N-diethylamino)ethoxy)phenyl,(2-(morpholin-4-yl)ethoxy)phenyl,benzyl,-CH2N(CH3)C(=O)CH2NH2,-CH2N(CH3)C(=O)CH2CH2NH2,-CH2N(CH3)C(=O)CH2N(CH3)2,-CH2N(CH3)C(=O)CH2CH2N(CH3)2,N-(2-(diethylamino)ethyl)carboxamido,N-(3-(diethylamino)propyl)carboxamido,N-(2-morpholinoethyl)carboxamido,N-(2-(piperazin-1-yl)ethyl)carboxamido,N-(3-(piperazin-1-yl)propyl)carboxamido-(3-morpholinopropyl)carboxamido,IV-(2-(4-methylpiperazin-1-yl)ethyl)carboxamido,IV-(3-(4-methylpiperazin-1-yl)propyl)carboxamido,(2-aminoethyl)carboxamidophenyl,(3-aminopropyl)carboxamidophenyl,(3-(diethylamino)propyl)carboxamidophenyl,(2-(piperazin-1-yl)ethyl)carboxamidophenyl,(3-(piperazin-1-yl)propyl)carboxamidophenyl,(2-(diethylamino)ethyl)carboxamidophenyl,2-(diethylamino)ethylamino,3-(diethylamino)propylamino,2-(dimethylamino)ethylamino,3-(dimethylamino)propylamino,2-aminoethylamino,3-aminopropylamino,2-(methylamino)ethylamino,3-(methylamino)propylamino,2-(2-hydroxylethylamino)ethylamino,3-(2-hydroxylethylamino)propylamino,2-(2-(dimethylamino)ethylamino)ethylamino,3-(2-(dimethylamino)ethylamino)propylamino,2-(2-(diethylamino)ethylamino)ethylamino,3-(2-(diethylamino)ethylamino)propylamino,2-(piperidin- 1 -yl)ethylamino,3 -(piperidin-1 - yl)propylamino, 2-morpholinoethylamino, 3 -morpholinopropylamino, 2-(piperazin-1 - yl)ethylamino,3-(piperazin-1-yl)propylamino,2-(4-methylpiperazin-1-yl)ethylamino,3-(4-methylpiperazin-1-yl)propylamino,2-(diethylamino)ethoxy,3-(diethylamino)propoxy,2-(dimethylamino)ethoxy,3-(dimethylamino)propoxy,2-aminoethoxy,3-aminopropoxy,2-(methylamino)ethoxy,3-(methylamino)propoxy,2-(2-hydroxylethylamino)ethoxy,3-(2-hydroxylethylamino)propoxy,2-(2-(dimethylamino)ethylamino)ethoxy,3-(2-(dimethylamino)ethylamino)propoxy,2-(2-(diethylamino)ethylamino)ethoxy,3-(2-(diethylamino)ethylamino)propoxy,2-(piperidin-1)-yl)ethoxy,3 -(piperidin-1 -yl)propoxy,2-morpholinoethoxy,3-morpholinopropoxy,2-(piperazin-1-yl)ethoxy,3-(piperazin-1-yl)propoxy,2-(4-methylpiperazin-1-yl)ethoxy,3-(4-methylpiperazin-1-yl)propoxy,(2-(diethylamino)ethylamino)methyl,(3-(diethylamino)propylamino)methyl,(2-(dimethylamino)ethylamino)methyl,(3-(dimethylamino)propylamino)methyl,(2-aminoethylamino)methyl,(3-aminopropylamino)methyl,(2-(methylamino)ethylamino)methyl,(3-(methylamino)propylamino)methyl,(2-(2-hydroxylethylamino))ethylamino)methyl,(3-(2-hydroxylethylamino)propylamino)methyl,(2-(piperidin-1-yl)ethylamino)methyl,(3 -(piperidin-1 - yl)propylamino)methyl,(2-morpholinoethylamino)methyl,(3-methylaminopropylamino)methyl,4-(2-aminoethoxy)phenyl,4-(3-aminopropoxy)phenyl,4-(2-(dimethylamino)ethoxy)phenyl,4-(3-(dimethylamino)propoxy)phenyl,4-(2-(diethylamino)ethoxy)phenyl,4-(3-(diethylamino)propoxy)phenyl,4-(2-(2-hydroxylethylamino)ethoxy)phenyl,4-(3-(2-hydroxylethylamino)propoxy)phenyl,4-(2-(2-(2-(dimethylamino)ethylamino)ethoxy)phenyl,4-(3-(2-(dimethylamino)ethylamino)propoxy)phenyl,4-(2-(2-(2-(diethylamino)ethylamino))ethoxy)phenyl,4-(3-(2-(diethylamino)ethylamino)propoxy)phenyl,4-(2-(piperidin-1-yl)ethoxy)phenyl,4-(3-(piperidin-1-yl)propoxy)phenyl,4-(2-morpholinoethoxy)phenyl,4-(3-trifluoropropoxy)phenyl,4-(2-(piperazin-1-yl)ethoxy)phenyl,4-(3-(piperazin-1 -yl)propoxy)phenyl,4-(2-(4-methylpiperazin-1-(yl)ethoxy)phenyl,4-(3 -(4-methylpiperazin-1-yl)propoxy)phenyl,4-(2-(4-(2-(2-aminoacetyl)piperazin-1 -yl)ethoxy)phenyl,4-(3 -(4-(2-aminoacetyl)piperazin-1 -yl)propoxy)phenyl,4-(2-(3-aminopyrrolidin-1 - yl)ethoxy)phenyl,A-(3-(3-aminopyrrolidinon-1-yl)propoxy)phenyl,4-(2-aminoethylamino)phenyl,4-(3-aminopropylamino)phenyl,4-(2-(dimethylamino)ethylamino)phenyl,4-(3-(dimethylamino)propylamino)phenyl,4-(2-(diethylamino)ethylamino)phenyl,4-(3-(diethylamino)propylamino)phenyl,4-(2-(2-hydroxylethylamino)ethylamino)phenyl,4-(3-(2-hydroxylethylamino)propylamino)phenyl,4-(2-(piperidin-1-yl)ethylamino)phenyl,4-(3-(piperidin-1-yl)propylamino)phenyl,4-(2-mercaptoethylamino)phenyl,4-(3 -mercaptopropylamino)phenyl,4-(2-(pyrrolidin-1 - yl)ethylamino)phenyland4-(3 -(pyrrolidin-1 -yl)propylamino)phenyl.

For example, R⁶ can be selected from the group consisting of hydrogen, C₁₋₂₀ alkyl, C₁₋₂₀ alkylene-OH, optionally substituted C₁₋₂₀ alkylene-aryl and optionally substituted C₁₋₂₀ alkylene-heteroaryl.

For example, R⁶ can be selected from the group consisting of methyl, ethyl, 2-(piperidin-1-yl)ethyl and (2-aminoethyl)benzyl.

For example, R⁴ and R⁶ can each be independently selected from the group consisting of methyl, R⁵ is selected from the group consisting of H.

For example, the compound having a structure of Formula II can comprise Fervenulin.

For example, R¹ and R³ can each be independently selected from the group consisting of methyl, R² is selected from the group consisting of hydrogen, methyl and 4-trifluoromethylphenyl.

For example, the compound having a structure of Formula I can comprise PKF118-310 or a derivative thereof.

For example, the derivative of PKF118-310 can further comprise a compound having a structure of Formula II and/or Formula III.

For example, the derivative of PKF118-310 can comprise 3-Methyltoxoflavin and/or Walrycin B.

For example, the derivative of PKF118-310 can comprise Fervenulin.

For example, the derivative of PKF 118-310 can comprise BI-D1870 and/or Lumazine.

For example, the compound having a structure of Formula I, II, or III can comprise those recorded in paragraphs [0051] to [0058] of the patent application (Publication Number: US20110166144A1), which (Publication Number: US20110166144A1) is incorporated herein by reference.

For example, the substance affecting the formation of a TCF/β-catenin complex can comprise the compound having a structure of Formula I or a pharmaceutically acceptable salt or hydrate:

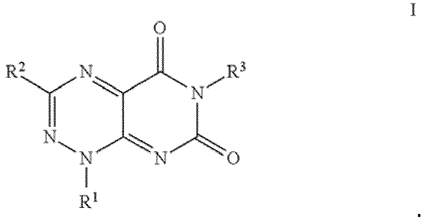

For example, when R² is C₄ alkyl, R¹ and R³ can each be independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl. For example, R¹ and R³ can each be independently selected from the group consisting of H and optionally substituted C₁₋₂₀ alkyl.

For example, the substance affecting the formation of a TCF/β-catenin complex can comprise the compound having a structure of Formula I or a pharmaceutically acceptable salt or hydrate:

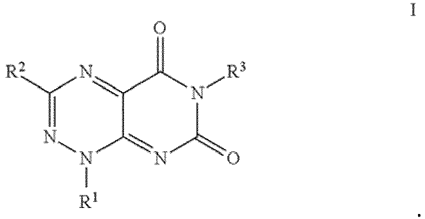

For example, when R² is isobutyl, R¹ and R³ can each be independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl. For example, R¹ and R³ can each be independently selected from the group consisting of H and optionally substituted C₁₋₂₀ alkyl.

For example, the substance affecting the formation of a TCF/β-catenin complex can comprise the compound having a structure of Formula I or a pharmaceutically acceptable salt or hydrate:

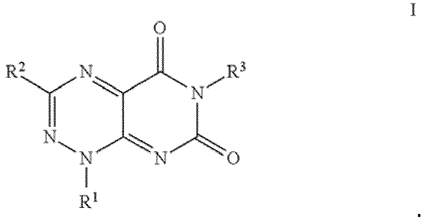

For example, R¹ can be C₃ alkyl; for example, R² can be C₄ alkyl; for example, R³ can be methyl.

For example, the substance affecting the formation of a TCF/β-catenin complex can comprise the compound having a structure of Formula I or a pharmaceutically acceptable salt or hydrate: 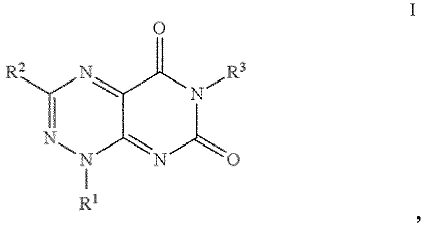 wherein R¹ can be C₃ alkyl, R² can be C₄ alkyl, R³ can be methyl.

For example, the substance affecting the formation of a TCF/β-catenin complex can comprise the compound having a structure of Formula I or a pharmaceutically acceptable salt or hydrate: 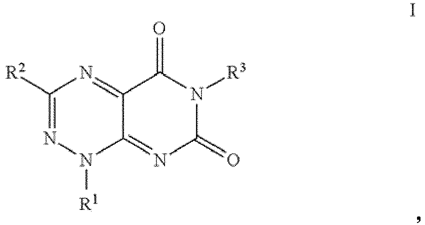 wherein R¹ can be isopropyl, R² can be isobutyl, and R³ can be methyl.

For example, the substance affecting the formation of a TCF/β-catenin complex can comprise:

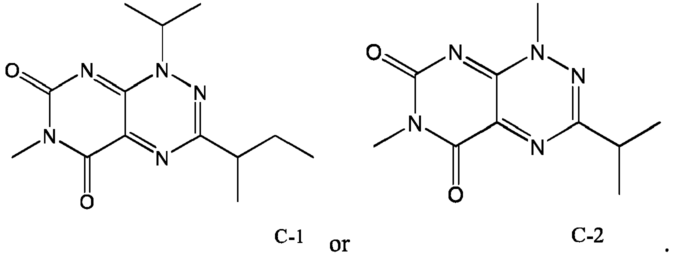

For example, the substance affecting the formation of a TCF/β-catenin complex can comprise

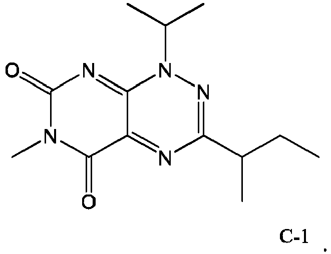

For example, the compound having a structure of Formula I, II, or III can comprise the following compounds:
3-(4-(2-(2-(diethylamino)ethoxy)phenyl)-1,6-dimethylpyrimido[5,4-e][1,2,4]triazin-5,7(1H,6H)-dione;1-benzyl-3-(4-(2-(2-(diethylamino)ethoxy)phenyl)-6-methylpyrimido[5,4-e][1,2,4]triazin-5,7(1H,6H)-dione;1 -(4-fluorobenzyl)-6-methyl-3-(4-(2-(4-methylpiperazin-1 - yl)ethoxy)phenyl)pyrimido[5,4-e][1,2,4]triazin-5,7(1H,6H)-dione;difluorobenzyl)-6-methyl-3-(4-(2-(morpholin-4-yl)ethoxy)phenyl)pyrimido[5,4-e][1,2,4]triazin-5,7(1H,6H)-dione;3-(4-(2-(4-aminoacetylpiperazin-1-yl)ethoxy)phenyl)-6-methyl-1-phenylpyrimidin[5,4-e][1,2,4]triazin-5,7(1H,6H)-dione;3-(4-(2-(4-(dimethylamino)piperidin-1-yl)ethoxy)phenyl)-1-isopropyl-6-methylpyrimidin[5,4-e][1,2,4]triazin-5,7(1H,6H)-dione;3-(4-(2-(3-aminopyrrolidin-1-yl)ethoxy)phenyl)-1-(3-fluorophenyl)-6-methylpyrimidin[5,4-e][1,2,4]triazin-5,7(1H,6H)-dione;1-cyclopentyl-6-methyl-3-(4-(3-(morpholin-4-yl)propoxy)phenyl)pyrimido[5,4-e][1,2,4]triazin-5,7(1H,6H)-dione;1-cyclopropyl-3-(6-(2-(2-(diethylamino)ethoxy)pyridyl-3-yl)-6-methylpyrimidin[5,4-e][1,2,4]triazin-5,7(1H,6H)-dione;1-cyclopentyl-3-(4-(2-(2-piperidin-1-yl)ethoxy)phenyl)pyrimido[5,4-e][1,2,4]triazin-5,7(1H,6H)-dione;3-(4-(2-(dimethylamino)ethyl)phenyl)-6-methyl-1-phenylpyrimido[5,4-e][1,2,4]triazin-5,7(1H,6H)-dione; 1 -(3,4-difluorobenzyl)-6-methyl-3 -(4-(3 -(4-methylpiperazin-1 - yl)propyl)phenyl)pyrimido[5,4-e][1,2,4]triazin-5,7(1H,6H)-dione;
1-isopropyl-6-methyl-3-(4-(2-(piperidin-1-yl)ethylamino)phenyl)pyrimido[5,4-d][1,2,4]triazin-5,7(1H,6H)-dione;1-cyclopropyl-6-(2-(piperidin-1-yl)ethyl)-3-(pyridyl-3-yl)pyrimidin[5,4-e][1,2,4]triazin-5,7(1H,6H)-dione;6-(4-(2-aminoethyl)benzyl)-1-cyclopentyl-3-(4-fluorophenyl)pyrimido[5,4-e][1,2,4]triazin-5,7(1H,6H)-dione;1-cyclopentyl-3-(4-carboxyphenyl)-6-methylpyrimido[5,4-e][1,2,4]triazin-5,7(1H,6H)-dione;
3-(4-(2-(bis(ethylamino)ethyl)carboxamido)phenyl)-1-isopropyl-6-methylpyrimido[5,4-e][1,2,4]triazin-5,7(1H,6H)-dione;3-(4-(2-(diethylamino)ethoxy)phenyl)-8-(3,4-difluorobenzyl)-6-methylpyrimidin[5,4-e][1,2,4]triazin-5,7(6H,8H)-dione;8-benzyl-3-(4-(2-(diethylamino)ethoxy)phenyl)-6-methylpyrimido[5,4-e][1,2,4]triazin-5,7(6H,8H)-dione;8-(4-fluorobenzyl)-6-methyl-3-(4-(2-(4-methylpiperazin-1-yl)ethoxy)phenyl)pyrimido[5,4-e][1,2,4]triazin-5,7(6H,8H)-dione;8-(3,4-difluorobenzyl)-6-methyl-3-(4-(2-(morpholin-4-yl)ethoxy)phenyl)pyrimido[5,4-e][1,2,4]triazin-5,7(6H,8H)-dione;3-(4-(2-(4-aminoacetylpiperazin-1-yl)ethoxy)phenyl)-6-methyl-8-phenylpyrimidin[5,4-e][1,2,4]triazin-5,7(6H,8H)-dione;3-(4-(2-(4-(dimethylamino)piperidin-1-yl)ethoxy)phenyl)-8-isopropyl-6-methylpyrimidin[5,4-e][1,2,4]triazin-5,7(6H,8H)-dione;3-(4-(2-(3-aminopyrrolidin-1-yl)ethoxy)phenyl)-8-(3-fluorophenyl)-6-methylpyrimidin[5,4-e][1,2,4]triazin-5,7(6H,8H)-dione; 8-cyclopentyl-6-methyl-3-(4-(3-(morpholin-4-yl)propoxy)phenyl)pyrimido[5,4-e][1,2,4]triazin-5,7(6H,8H)-dione;8-cyclopropyl-3-(6-(2-(2-(diethylamino)ethoxy)pyridyl-3-yl)-6-methylpyrimidin[5,4-e][1,2,4]triazin-5,7(6H,8H)-dione;8-cyclopentyl-3-(4-(2-(2-piperidin-1-yl)ethoxy)phenyl)pyrimido[5,4-e][1,2,4]triazin-5,7(6H,8H)-dione;3-(4-(2-(dimethylamino)ethyl)phenyl)-6-methyl-8-phenylpyrimido[5,4-e] [1,2,4]triazin-5,7(6H,8H)-dione;8-(3,4-difluorobenzyl)-6-methyl-3-(4-(3-(4-methylpiperazin-1-yl)propyl)phenyl)pyrimido[5,4-e][1,2,4]triazin-5,7(6H,8H)-dione;
8-isopropyl-6-methyl-3-(4-(24-piperidin-1-yl)ethylamino)phenyl)pyrimido[5,4-e][1,2,4]triazin-5,7(6H,8H)-dione;8-cyclopropyl-6-(2-(piperidin-1-yl)ethyl)-3-(pyridyl-3-yl)pyrimidin[5,4-e][1,2,4]triazin-5,7(6H,8H)-dione;6-(4-(2-aminoethyl)benzyl)-8-cyclopentyl-3-(4-fluorophenyl)pyrimido[5,4-e][1,2,4]triazin-5,7(6H,8H)-dione;8-cyclopentyl-3-(4-carboxyphenyl)-6-methylpyrimido[5,4-e][1,2,4]triazin-5,7(6H,8H)-dione;3-(4-(2-(diethylamino)ethylcarboxamido)phenyl)-6,8-dimethylpyrimido[5,4-e][1,2,4]triazin-5,7(6H,8H)-dione;6,8-dimethyl-3-(4-(2-(4-methylpiperazin-1-yl)ethoxy)phenyl)pyrimido[5,4-e][1,2,4]triazin-5,7(6H,8H)-dione;8-methyl-3-(4-(2-(4-(4-methylpiperazin-1-yl)ethoxy)phenyl)pyrimido[5,4-e][1,2,4]triazin-5,7(6H,8H)-dione;3-(4-(2-(diethylamino)ethoxy)phenyl)-6,8-dimethylpyrimido[5,4-e][1,2,4]triazin-5,7(6H,8H)-dione;3-(4-(2-(diethylamino)ethoxy)phenyl)-8-methylpyrimidin[5,4-e][1,2,4]triazin-5,7(6H,8H)-dione;6,8-dimethyl-3-(4-(2-(morpholin-4-yl)ethoxy)phenyl)pyrimido[5,4-e][1,2,4]triazin-5,7(6H,8H)-dione;3-(2-(diethylamino)ethylamino)-6,8-dimethylpyrimido[5,4-e][1,2,4]triazin-5,7(6H,8H)-dione;3-(3-(diethylamino)propylamino)-6,8-dimethylpyrimido[5,4-e][1,2,4]triazin-5,7(6H,8H)-dione;3-(2-(dimethylamino)ethylamino)-6,8-dimethylpyrimido[5,4-e][1,2,4]triazin-5,7(6H,8H)-dione;3-(3-(dimethylamino)propylamino)-6,8-dimethylpyrimido[5,4-e][1,2,4]triazin-5,7(6H,8H)-dione;3-(2-aminoethylamino)-6,8-dimethylpyrimido[5,4-e][1,2,4]triazin-5,7(6H,8H)-dione;3-(3-aminopropylamino)-6,8-dimethylpyrimido[5,4-e][1,2,4]triazin-5,7(6H,8H)-dione;6,8-dimethyl-3-(2-(methylamino)ethylamino)pyrimidin[5,4-e][1,2,4]triazin-5,7(6H,8H)-dione;
6,8-dimethyl-3-(3-(methylamino)propylamino)pyrimidin[5,4-e][1,2,4]triazin-5,7(6H,8H)-dione;3-(2-(2-hydroxylethylamino)ethylamino)-6,8-dimethylpyrimido[5,4-e][1,2,4]triazin-5,7(6H,8H)-dione;3-(3-(2-hydroxylethylamino)propylamino)-6,8-dimethylpyrimido[5,4-e][1,2,4]triazin-5,7(6H,8H)-dione;3-(2-(2-(2-(dimethylamino)ethylamino)ethylamino)-6,8-dimethylpyrimido[5,4-e][1,2,4]triazin-5,7(6H,8H)-dione;34342-(dimethylamino)ethylamino)propylamino)-6,8-dimethylpyrimido[5,4-e][1,2,4]triazin-5,7(6H,8H)-dione;3-(2-(2-(2-(diethylamino)ethylamino)ethylamino)-6,8-dimethylpyrimido[5,4-e][1,2,4]triazin-5,7(6H,8H)-dione;34342-(diethylamino)ethylamino)propylamino)-6,8-dimethylpyrimido[5,4-e][1,2,4]triazin-5,7(6H,8H)-dione;6,8-dimethyl-3-(2-(piperidin-1-yl)ethylamino)pyrimidin[5,4-e][1,2,4]triazin-5,7(6H,8H)-dione;6,8-dimethyl-3-(3-(piperidin-1-yl)propylamino)pyrimidin[5,4-e][1,2,4]triazin-5,7(6H,8H)-dione;6,8-dimethyl-3-(2-morpholinoethylamino)pyrimido[5,4-e][1,2,4]triazin-5,7(6H,8H)-dione;6,8-dimethyl-3-(3-morpholinopropyl)amino)pyrimidin[5,4-e][1,2,4]triazin-5,7(6H,8H)-dione;6,8-dimethyl-3-(2-(piperazin-1-yl)ethylamino)pyrimidin[5,4-e][1,2,4]triazin-5,7(6H,8H)-dione;6,8-dimethyl-3-(3-(piperazin-1-yl)propylamino)pyrimidin[5,4-e][1,2,4]triazin-5,7(6H,8H)-dione;
6,8-dimethyl-3-(2-(4-(methylpiperazin-1-yl)ethylamino)pyrimidin[5,4-e][1,2,4]triazin-5,7(6H,8H)-dione;6,8-dimethyl-3-(3-(4-(methylpiperazin-1-yl)propylamino)pyrimidin[5,4-e][1,2,4]triazin-5,7(6H,8H)-dione;3-(2-(diethylamino)ethoxy)-6,8-dimethylpyrimido[5,4-e][1,2,4]triazin-5,7(6H,8H)-dione;3-(3-(diethylamino)propoxy)-6,8-dimethylpyrimido[5,4-e][1,2,4]triazin-5,7(6H,8H)-dione;3-(2-(dimethylamino)ethoxy)-6,8-dimethylpyrimido[5,4-e][1,2,4]triazin-5,7(6H,8H)-dione;3-(3-(dimethylamino)propoxy)-6,8-dimethylpyrimido[5,4-e][1,2,4]triazin-5,7(6H,8H)-dione;3-(2-aminoethoxy)-6,8-dimethylpyrimido[5,4-e][1,2,4]triazin-5,7(6H,8H)-dione;3-(3-aminopropoxy)-6,8-dimethylpyrimidin[5,4-e][1,2,4]triazin-5,7(6H,8H)-dione;6,8-dimethyl-3-(2-(methylamino)ethoxy)pyrimidin[5,4-e][1,2,4]triazin-5,7(6H,8H)-dione;6,8-dimethyl-3-(3-(methylamino)propoxy)pyrimidin[5,4-e][1,2,4]triazin-5,7(6H,8H)-dione;3-(2-(2-hydroxylethylamino)ethoxy)-6,8-dimethylpyrimido[5,4-e][1,2,4]triazin-5,7(6H,8H)-dione;3-(3-(2-hydroxylethylamino)propoxy)-6,8-dimethylpyrimido[5,4-e][1,2,4]triazin-5,7(6H,8H)-dione;3-(2-(2-(2-(dimethylamino)ethylamino)ethoxy)-6,8-dimethylpyrimido[5,4-e][1,2,4]triazin-5,7(6H,8H)-dione;3-(3-(2-(dimethylamino)ethylamino)propoxy)-6,8-dimethylpyrimido[5,4-e][1,2,4]triazin-5,7(6H,8H)-dione;3-(2-(2-(2-(diethylamino)ethylamino)ethoxy)-6,8-dimethylpyrimido[5,4-e][1,2,4]triazin-5,7(6H,8H)-dione;3-(3-(2-(2-(diethylamino)ethylamino)propoxy)-6,8-dimethylpyrimido[5,4-e][1,2,4]triazin-5,7(6H,8H)-dione;6,8-dimethyl-3-(2-(piperidin-1-yl)ethoxy)pyrimidin[5,4-e][1,2,4]triazin-5,7(6H,8H)-dione;6,8-dimethyl-3-(3-(piperidin-1-yl)propoxy)pyrimidin[5,4-e][1,2,4]triazin-5,7(6H,8H)-dione;
6,8-dimethyl-3-(2-morpholinoethoxy)pyrimidin[5,4-e][1,2,4]triazin-5,7(6H,8H)-dione;6,8-dimethyl-3-(3-morpholinopropoxy)pyrimidin[5,4-e][1,2,4]triazin-5,7(6H,8H)-dione;6,8-dimethyl-3-(2-(piperazin-1-yl)ethoxy)pyrimidin[5,4-e][1,2,4]triazin-5,7(6H,8H)-dione;6, 8-dimethyl-3-(3-(piperazin-1-yl)propoxy)pyrimidin[5,4-d][1,2,4]triazin-5,7(6H,8H)-dione;6,8-dimethyl-3-(2-(4-methylpiperazin-1-yl)ethoxy)pyrimidin[5,4-e][1,2,4]triazin-5,7(6H,8H)-dione;6,8-dimethyl-3-(3-(4-methylpiperazin-1-yl)propoxy)pyrimidin[5,4-e][1,2,4]triazin-5,7(6H,8H)-dione;3-((2-(diethylamino)ethylamino)methyl)-6,8-dimethylpyrimido[5,4-e][1,2,4]triazin-5,7(6H,8H)-dione;3-((3-(diethylamino)propylamino)methyl)-6,8-dimethylpyrimido[5,4-e][1,2,4]triazin-5,7(6H,8H)-dione;3-((2-(dimethylamino)ethylamino)methyl)-6,8-dimethylpyrimido[5,4-e][1,2,4]triazin-5,7(6H,8H)-dione;3-((3-(dimethylamino)propylamino)methyl)-6,8-dimethylpyrimido[5,4-e][1,2,4]triazin-5,7(6H,8H)-dione;3-(2-aminoethylamino)methyl)-6,8-dimethylpyrimido[5,4-e][1,2,4]triazin-5,7(6H,8H)-dione;3-(3-aminopropylamino)methyl)-6,8-dimethylpyrimido[5,4-e][1,2,4]triazin-5,7(6H,8H)-dione;6,8-dimethyl-3-((2-(methylamino)ethylamino)methyl)pyrimidin[5,4-e][1,2,4]triazin-5,7(6H,8H)-dione;6,8-dimethyl-3-(3-(methylamino)propylamino)methyl)pyrimidin[5,4-e][1,2,4]triazin-5,7(6H,8H)-dione;3-((2-(2-hydroxylethylamino)ethylamino)methyl)-6,8-dimethylpyrimido[5,4-e][1,2,4]triazin-5,7(6H,8H)-dione;3-((3-(2-hydroxylethylamino)propylamino)methyl)-6,8-dimethylpyrimido[5,4-e][1,2,4]triazin-5,7(6H,8H)-dione;2-amino-N-((6,8-dimethyl-5,7-dioxolan-5,6,7,8-tetrahydropyrimidin[5,4-e][1,2,4]triazin-3-yl)methyl)-N-methylacetamide;3-amino-N-((6,8-dimethyl-5,7-dioxo-5,6,7,8-tetrahydropyrimidin[5,4-e][1,2,4]triazin-3-yl)methyl)-N-methylpropionamide;N-((6,8-dimethyl-5,7-dioxolan-5,6,7,8-tetrahydropyrimidin[5,4-e][1,2,4]triazin-3-yl)methyl)-2-(dimethylamino)-N-methylacetamide;
N-((6,8-dimethyl-5,7-dioxo-5,6,7,8-tetrahydropyrimidin[5,4-e][1,2,4]triazin-3-yl)methyl)-3-(dimethylamino)-N-methylpropionamide;6,8-dimethyl-3-((2-(piperidin-1-yl)ethylamino)methyl)pyrimidin[5,4-e][1,2,4]triazin-5,7(6H,8H)-dione;6,8-dimethyl-3-(3-(piperidin-1-yl)propylamino)methyl)pyrimidin[5,4-e][1,2,4]triazin-5,7(6H,8H)-dione;6,8-dimethyl-3-(2-morpholinoethylamino)methyl)pyrimidin[5,4-e][1,2,4]triazin-5,7(6H,8H)-dione;6,8-dimethyl-3-(3-morpholinopropylamino)methyl)pyrimidin[5,4-e][1,2,4]triazin-5,7(6H,8H)-dione;N-(2-(diethylamino)ethyl)-6,8-dimethyl-5,7-dioxo-5,6,7,8-tetrahydropyrimidin[5,4-e][1,2,4]triazin-3-carboxamide;N-(3-(diethylamino)propyl)-6,8-dimethyl-5,7-dioxo-5,6,7,8-tetrahydropyrimidin[5,4-e][1,2,4]triazin-3-carboxamide;6,8-dimethyl-N-(2-morpholinoethyl)-5,7-dioxo-5,6,7,8-tetrahydropyrimidin[5,4-e][1,2,4]triazin-3-formamide;6,8-dimethyl-5,7-dioxo-N-(2-(piperazin-1-yl)ethyl)-5,6,7,8-tetrahydropyrimido[5,4-e][1,2,4]triazin-3-carboxamide;6,8-dimethyl-5,7-dioxo-N-(3-(piperazin-1-yl)propyl)-5,6,7,8-tetrahydropyrimido[5,4-e][1,2,4]triazin-3-carboxamide;6,8-dimethyl-N-(3-morpholinopropyl)-5,7-dioxo-5,6,7,8-tetrahydropyrimidin[5,4-e][1,2,4]triazin-3-formamide;6,8-dimethyl-N-(2-(4-methylpiperazin-1-yl)ethyl)-5,7-dioxo-5,6,7,8-tetrahydropyrimido[5,4-e][1,2,4]triazin-3-carboxamide;6,8-dimethyl-N-(3-(4-methylpiperazin-1-yl)propyl)-5,7-dioxo-5,6,7,8-tetrahydropyrimido[5,4-e][1,2,4]triazin-3-carboxamide;8-cyclopentyl-3-(2-(diethylamino)ethylamino)-6-methylpyrimido[5,4-e][1,2,4]triazin-5,7(6H,8H)-dione;8-cyclopentyl-3-(3-(diethylamino)propylamino)-6-methylpyrimido[5,4-e][1,2,4]triazin-5,7(6H,8H)-dione;8-cyclopentyl-3-(2-(dimethylamino)ethylamino)-6-methylpyrimido[5,4-e][1,2,4]triazin-5,7(6H,8H)-dione;8-cyclopentyl-3-(3-(dimethylamino)propylamino)-6-methylpyrimido[5,4-e][1,2,4]triazin-5,7(6H,8H)-dione;3-(2-aminoethylamino)-8-cyclopentyl-6-methylpyrimido[5,4-e][1,2,4]triazin-5,7(6H,8H)-dione; 3-(3-aminopropylamino)-8-cyclopentyl-6-methylpyrimido[5,4-e][1,2,4]triazin-5,7(6H,8H)-dione; 8-cyclopentyl-6-methyl-3-(2-(methylamino)ethylamino)pyrimidin[5,4-e][1,2,4]triazin-5,7(6H,8H)-dione;8-cyclopentyl-6-methyl-3-(3-(methylamino)propylamino)pyrimidin[5,4-e][1,2,4]triazin-5,7(6H,8H)-dione;8-cyclopentyl-3-(2-(2-hydroxylethylamino)ethylamino)-6-methylpyrimido[5,4-e][1,2,4]triazin-5,7(6H,8H)-dione;8-cyclopentyl-3-(3-(2-(hydroxylethylamino)propylamino)-6-methylpyrimido[5,4-e][1,2,4]triazin-5,7(6H,8H)-dione;8-cyclopentyl-3-(2-(2-(2-(dimethylamino)ethylamino)ethylamino)-6-methylpyrimidin[5,4-e][1,2,4]triazin-5,7(6H,8H)-dione;8-cyclopentyl-3-(3-(2-(2-(dimethylamino)ethylamino)propylamino)-6-methylpyrimidin[5,4-e][1,2,4]triazin-5,7(6H,8H)-dione;
8-cyclopentyl-3-(2-(2-(2-(diethylamino)ethylamino)ethylamino)-6-methylpyrimidin[5,4-e][1,2,4]triazin-5,7(6H,8H)-dione;8-cyclopentyl-3-(3-(2-(2-(diethylamino)ethylamino)propylamino)-6-methylpyrimidin[5,4-e][1,2,4]triazin-5,7(6H,8H)-dione;8-cyclopentyl-6-methyl-3-(2-(piperidin-1-yl)ethylamino)pyrimidin[5,4-e][1,2,4]triazin-5,7(6H, 8H)-dione; 8-cyclopentyl-6-methyl-3-(3-(piperidin-1-yl)propylamino)pyrimidin[5,4-e][1,2,4]triazin-5,7(6H,8H)-dione;8-cyclopentyl-6-methyl-3-(2-morpholinoethylamino)pyrimidin[5,4-e][1,2,4]triazin-5,7(6H,8H)-dione;8-cyclopentyl-6-methyl-3-(3-morpholinopropylamino)pyrimido[5,4-e][1,2,4]triazin-5,7(6H,8H)-dione;8-cyclopentyl-6-methyl-3-(2-(piperazin-1-yl)ethylamino)pyrimidin[5,4-e][1,2,4]triazin-5,7(6H,8H)-dione;8-cyclopentyl-6-methyl-3-(3-(piperazin-1-yl)propylamino)pyrimidin[5,4-e][1,2,4]triazin-5,7(6H, 8H)-dione; 8-cyclopentyl-6-methyl-3-(2-(4-methylpiperazin-1-yl)ethylamino)pyrimidin[5,4-e][1,2,4]triazin-5,7(6H,8H)-dione;8-cyclopentyl-6-methyl-3-(3-(4-methylpiperazin-1-yl)propylamino)pyrimidin[5,4-e][1,2,4]triazin-5,7(6H,8H)-dione;8-cyclopentyl-3-(2-(diethylamino)ethoxy)-6-methylpyrimido[5,4-e][1,2,4]triazin-5,7(6H,8H)-dione;8-cyclopentyl-3-(3-(diethylamino)propoxy)-6-methylpyrimido[5,4-e][1,2,4]triazin-5,7(6H,8H)-dione;8-cyclopentyl-3-(2-(dimethylamino)ethoxy)-6-methylpyrimidin[5,4-e][1,2,4]triazin-5,7(6H,8H)-dione;8-cyclopentyl-3-(3-(dimethylamino)propoxy)-6-methylpyrimido[5,4-e][1,2,4]triazin-5,7(6H,8H)-dione;3-(2-aminoethoxy)-8-cyclopentyl-6-methylpyrimido[5,4-e][1,2,4]triazin-5,7(6H,8H)-dione;3-(3-aminopropoxy)-8-cyclopentyl-6-methylpyrimido[5,4-e][1,2,4]triazin-5,7(6H,8H)-dione;8-cyclopentyl-6-methyl-3-(2-(methylamino)ethoxy)pyrimidin[5,4-e][1,2,4]triazin-5,7(6H,8H)-dione;8-cyclopentyl-6-methyl-3-(3-(methylamino)propoxy)pyrimidin[5,4-e][1,2,4]triazin-5,7(6H,8H)-dione;8-cyclopentyl-3-(2-(2-hydroxylethylamino)ethoxy)-6-methylpyrimido[5,4-e][1,2,4]triazin-5,7(6H,8H)-dione;8-cyclopentyl-3-(3-(2-hydroxylethylamino)propoxy)-6-methylpyrimido[5,4-e][1,2,4]triazin-5,7(6H,8H)-dione;8-cyclopentyl-3-(2-(2-(2-(dimethylamino)ethylamino)ethoxy)-6-methylpyrimidin[5,4-e][1,2,4]triazin-5,7(6H,8H)-dione;8-cyclopentyl-3-(3-(2-(2-(dimethylamino)ethylamino)propoxy)-6-methylpyrimidin[5,4-e][1,2,4]triazin-5,7(6H,8H)-dione;8-cyclopentyl-3-(2-(2-(2-(diethylamino)ethylamino)ethoxy)-6-methylpyrimidin[5,4-e][1,2,4]triazin-5,7(6H,8H)-dione;8-cyclopentyl-3-(3-(2-(2-(diethylamino)ethylamino)propoxy)-6-methylpyrimidin[5,4-e][1,2,4]triazin-5,7(6H,8H)-dione;8-cyclopentyl-6-methyl-3-(2-(piperidin-1-yl)ethoxy)pyrimidin[5,4-e][1,2,4]triazin-5,7(6H,8H)-dione; 8-cyclopentyl-6-methyl-3-(3-(piperidin-1-yl)propoxy)pyrimidin[5,4-e][1,2,4]triazin-5,7(6H,8H)-dione; 8-cyclopentyl-6-methyl-3-(2-morpholinoethoxy)pyrimidin[5,4-e][1,2,4]triazin-5,7(6H,8H)-dione; 8-cyclopentyl-6-methyl-3-(3-morpholinopropoxy)pyrimidin[5,4-e][1,2,4]triazin-5,7(6H,8H)-dione; 8-cyclopentyl-6-methyl-3-(2-(piperazin-1-yl)ethoxy)pyrimidin[5,4-e][1,2,4]triazin-5,7(6H,8H)-dione;8-cyclopentyl-6-methyl-3-(3-(piperazin-1-yl)propoxy)pyrimidin[5,4-e][1,2,4]triazin-5,7(6H, 8H)-dione; 8-cyclopentyl-6-methyl-3-(2-(4-methylpiperazin-1-yl)ethoxy)pyrimidin[5,4-e][1,2,4]triazin-5,7(6H,8H)-dione;8-cyclopentyl-6-methyl-3-(3-(4-methylpiperazin-1-yl)propoxy)pyrimidin[5,4-e][1,2,4]triazin-5,7(6H,8H)-dione;8-cyclopentyl-3-((2-(diethylamino)ethylamino)methyl)-6-methylpyrimido[5,4-e][1,2,4]triazin-5,7(6H,8H)-dione;8-cyclopentyl-3-((3-(diethylamino)propylamino)methyl)-6-methylpyrimidin[5,4-e][1,2,4]triazin-5,7(6H,8H)-dione;8-cyclopentyl-3-((2-(dimethylamino)ethylamino)methyl)-6-methylpyrimidin[5,4-e][1,2,4]triazin-5,7(6H,8H)-dione;8-cyclopentyl-3-((3-(dimethylamino)propylamino)methyl)-6-methylpyrimido[5,4-e][1,2,4]triazin-5,7(6H,8H)-dione;3-(2-aminoethylamino)methyl)-8-cyclopentyl-6-methylpyrimido[5,4-e][1,2,4]triazin-5,7(6H,8H)-dione;3-(3-aminopropylamino)methyl)-8-cyclopentyl-6-methylpyrimido[5,4-e][1,2,4]triazin-5,7(6H,8H)-dione;8-cyclopentyl-6-methyl-3-(2-(methylamino)ethylamino)methyl)pyrimidin[5,4-e][1,2,4]triazin-5,7(6H,8H)-dione;8-cyclopentyl-6-methyl-3-(3-(methylamino)propylamino)methyl)pyrimidin[5,4-e][1,2,4]triazin-5,7(6H,8H)-dione;8-cyclopentyl-3-((2-(2-hydroxylethylamino)ethylamino)methyl)-6-methylpyrimidin[5,4-e][1,2,4]triazin-5,7(6H,8H)-dione;8-cyclopentyl-3-(3-(2-hydroxylethylamino)propylamino)methyl)-6-methylpyrimidin[5,4-e][1,2,4]triazin-5,7(6H,8H)-dione;2-amino-N-((8-cyclopentyl-6-methyl-5,7-dioxo-5,6,7,8-tetrahydropyrimidin[5,4-e][1,2,4]triazin-3-yl)methyl)-N-methylacetamide;3-amino-N-((8-cyclopentyl-6-methyl-5,7-dioxo-5,6,7,8-tetrahydropyrimidin[5,4-e][1,2,4]triazin-3-yl)methyl)-N-methylpropionamide;N-(8-cyclopentyl-6-methyl-5,7-dioxo-5,6,7,8-tetrahydropyrimidin[5,4-e][1,2,4]yl)methyl)-2-(dimethylamino)-N-methylacetamide;N-(8-cyclopentyl-6-methyl-5,7-dioxo-5,6,7,8-tetrahydropyrimidin[5,4-e][1,2,4]triazin-3-yl)methyl)-3-(dimethylamino)-N-methylpropionamide; 8-cyclopentyl-6-methyl-3-(2-(piperidin-1-yl)ethylamino)methyl)pyrimidin[5,4-e][1,2,4]triazin-5,7(6H,8H)-dione;8-cyclopentyl-6-methyl-3-(3-(piperidin-1-yl)propylamino)methyl)pyrimidin[5,4-e][1,2,4]triazin-5,7(6H,8H)-dione;8-cyclopentyl-6-methyl-3-(2-morpholinoethylamino)methyl)pyrimidin[5,4-e][1,2,4]triazin-5,7(6H,8H)-dione;8-cyclopentyl-6-methyl-3-(3-morpholinopropylamino)methyl)pyrimidin[5,4-e][1,2,4]triazin-5,7(6H,8H)-dione;
8-cyclopentyl-N-(2-(diethylamino)ethyl)-6-methyl-5,7-dioxo-5,6,7,8-tetrahydropyrimido[5,4-e][1,2,4]triazin-3-carboxamide;8-cyclopentyl-N-(3-(diethylamino)propyl)-6-methyl-5,7-dioxo-5,6,7,8-tetrahydropyrimidin[5,4-e][1,2,4]triazin-3-carboxamide;8-cyclopentyl-6-methyl-N-(2-morpholinoethyl)-5,7-dioxo-5,6,7,8-tetrahydropyrimido[5,4-e][1,2,4]triazin-3-carboxamide;8-cyclopentyl-6-methyl-N-(3-morpholinopropyl)-5,7-dioxo-5,6,7,8-tetrahydropyrimidin[5,4-d][1,2,4]triazin-3-carboxamide;8-cyclopentyl-6-methyl-5,7-dioxo-N-(2-(piperazin-1-yl)ethyl)-5,6,7,8-tetrahydropyrimido[5,4-e][1,2,4]triazin-3-carboxamide;8-cyclopentyl-6-methyl-5,7-dioxo-(3-(piperazin-1-yl)propyl)-5,6,7,8-tetrahydropyrimido[5,4-e][1,2,4]triazin-3-carboxamide;8-cyclopentyl-6-methyl-N-(2-(4-methylpiperazin-1-yl)ethyl)-5,7-dioxo-5,6,7,8-tetrahydropyrimido[5,4-e][1,2,4]triazin-3-carboxamide;8-cyclopentyl-6-methyl-N-(3-(4-methylpiperazin-1-yl)propyl)-5,7-dioxo-5,6,7,8-tetrahydropyrimido[5,4-e][1,2,4]triazin-3-carboxamide;
3-(2-(diethylamino)ethylamino)-8-(4-fluorophenyl)-6-methylpyrimido[5,4-e][1,2,4]triazin-5,7(6H,8H)-dione;3-(3-(diethylamino)propylamino)-8-(4-fluorophenyl)-6-methylpyrimido[5,4-e][1,2,4]triazin-5,7(6H,8H)-dione;3-(2-(dimethylamino)ethylamino)-8-(4-fluorophenyl)-6-methylpyrimido[5,4-e][1,2,4]triazin-5,7(6H,8H)-dione;3-(3-(dimethylamino)propylamino)-8-(4-fluorophenyl)-6-methylpyrimido[5,4-e][1,2,4]triazin-5,7(6H,8H)-dione;3-(2-aminoethylamino)-8-(4-fluorophenyl)-6-methylpyrimido[5,4-e][1,2,4]triazin-5,7(6H,8H)-dione;3-(3-aminopropylamino)-8-(4-fluorophenyl)-6-methylpyrimido[5,4-e][1,2,4]triazin-5,7(6H,8H)-dione;8-(4-fluorophenyl)-6-methyl-3-(2-(methylamino)ethylamino)pyrimidin[5,4-e][1,2,4]triazin-5,7(6H,8H)-dione;8-(4-fluorophenyl)-6-methyl-3-(3-(methylamino)propylamino)pyrimidin[5,4-e][1,2,4]triazin-5,7(6H,8H)-dione;8-(4-fluorophenyl)-3(2-(2-hydroxylethylamino)ethylamino)-6-methylpyrimidin[5,4-e][1,2,4]triazin-5,7(6H,8H)-dione;8-(4-fluorophenyl)-3-(3-(2-hydroxylethylamino)propylamino)-6-methylpyrimidin[5,4-e][1,2,4]triazin-5,7(6H,8H)-dione;3-(2-(2-(2-(dimethylamino)ethylamino)ethylamino)-8-(4-fluorophenyl)-6-methylpyrimidin[5,4-e][1,2,4]triazin-5,7(6H,8H)-dione;3-(3-(2-(dimethylamino)ethylamino)propylamino)-8-(4-fluorophenyl)-6-methyl)pyrimidin[5,4-e][1,2,4]triazin-5,7(6H,8H)-dione;3-(2-(2-(2-(diethylamino)ethylamino)ethylamino)-8-(4-fluorophenyl)-6-methylpyrimidin[5,4-e][1,2,4]triazin-5,7(6H,8H)-dione; 3-(3-(2-(diethylamino)ethylamino)propylamino)-8-(4-fluorophenyl)-6-methylpyrimidin[5,4-e][1,2,4]triazin-5,7(6H,8H)-dione;8-(4-fluorophenyl)-6-methyl-3-(2-(piperidin-1-yl)ethylamino)pyrimidin[5,4-e][1,2,4]triazin-5,7(6H,8H)-dione;8-(4-fluorophenyl)-6-methyl-3-(3-(piperidin-1-yl)propylamino)pyrimidin[5,4-e][1,2,4]triazin-5,7(6H,8H)-dione;8-(4-fluorophenyl)-6-methyl-3-(2-morpholinoethylamino)pyrimido[5,4-e][1,2,4]triazin-5,7(6H,8H)-dione;8-(4-fluorophenyl)-6-methyl-3-(3-morpholinopropylamino)pyrimido[5,4-e][1,2,4]triazin-5,7(6H,8H)-dione;8-(4-fluorophenyl)-6-methyl-3-(2-(piperazin-1-yl)ethylamino)pyrimidin[5,4-e][1,2,4]triazin-5,7(6H,8H)-dione;8-(4-fluorophenyl)-6-methyl-3-(3-(piperazin-1-yl)propylamino)pyrimidin[5,4-e][1,2,4]triazin-5,7(6H,8H)-dione;
8-(4-fluorophenyl)-6-methyl-3-(2-(4-methylpiperazin-1-yl)ethylamino)pyrimidin[5,4-e][1,2,4]triazin-5,7(6H,8H)-dione;8-(4-fluorophenyl)-6-methyl-3-(3-(4-methylpiperazin-1-yl)propylamino)pyrimidin[5,4-e][1,2,4]triazin-5,7(6H,8H)-dione;3-(2-(diethylamino)ethoxy)-8-(4-fluorophenyl)-6-methylpyrimido[5,4-e][1,2,4]triazin-5,7(6H,8H)-dione;3-(3-(diethylamino)propoxy)-8-(4-fluorophenyl)-6-methylpyrimido[5,4-e][1,2,4]triazin-5,7(6H,8H)-dione;3-(2-(dimethylamino)ethoxy)-8-(4-fluorophenyl)-6-methylpyrimido[5,4-e][1,2,4]triazin-5,7(6H,8H)-dione;3-(3-(dimethylamino)propoxy)-8-(4-fluorophenyl)-6-methylpyrimido[5,4-e][1,2,4]triazin-5,7(6H,8H)-dione;3-(2-aminoethoxy)-8-(4-fluorophenyl)-6-methylpyrimidin[5,4-e][1,2,4]triazin-5,7(6H,8H)-one; 3-(3-aminopropoxy)-8-(4-fluorophenyl)-6-methylpyrimido[5,4-e][1,2,4]triazin-5,7(6H,8H)-dione;8-(4-fluorophenyl)-6-methyl-3-(2-(methylamino)ethoxy)pyrimidin[5,4-e][1,2,4]triazin-5,7(6H,8H)-dione;8-(4-fluorophenyl)-6-methyl-3-(3-(methylamino)propoxy)pyrimidin[5,4-d][1,2,4]triazin-5,7(6H,8H)-dione;8-(4-fluorophenyl)-3-(2-(2-hydroxylethylamino)ethoxy)-6-methylpyrimidin[5,4-e][1,2,4]triazin-5,7(6H,8H)-dione;8-(4-fluorophenyl)-3-(3-(2-hydroxylethylamino)propoxy)-6-methylpyrimidin[5,4-e][1,2,4]triazin-5,7(6H,8H)-dione;3-(2-(2-(2-(dimethylamino)ethylamino)ethoxy)-8-(4-fluorophenyl)-6-methylpyrimidin[5,4-e][1,2,4]triazin-5,7(6H,8H)-dione;3-(3-(2-(dimethylamino)ethylamino)propoxy)-8-(4-fluorophenyl)-6-methylpyrimidin[5,4-e][1,2,4]triazin-5,7(6H,8H)-dione;3-(2-(2-(2-(diethylamino)ethylamino)ethoxy)-8-(4-fluorophenyl)-6-methylpyrimidin[5,4-e][1,2,4]triazin-5,7(6H,8H)-dione;3-(3-(2-(diethylamino)ethylamino)propoxy)-8-(4-fluorophenyl)-6-methylpyrimidin[5,4-e][1,2,4]triazin-5,7(6H,8H)-dione;8-(4-fluorophenyl)-6-methyl-3-(2-(piperidin-1-yl)ethoxy)pyrimidin[5,4-e][1,2,4]triazin-5,7(6H,8H)-dione;8-(4-fluorophenyl)-6-methyl-3-(3-(piperidin-1-yl)propoxy)pyrimidin[5,4-e][1,2,4]triazin-5,7(6H,8H)-dione;8-(4-fluorophenyl)-6-methyl-3-(2-morpholinoethoxy)pyrimidin[5,4-e][1,2,4]triazin-5,7(6H,8H)-dione;8-(4-fluorophenyl)-6-methyl-3-(3-morpholinopropoxy)pyrimidin[5,4-e][1,2,4]triazin-5,7(6H,8H)-dione;8-(4-fluorophenyl)-6-methyl-3-(2-(piperazin-1-yl)ethoxy)pyrimidin[5,4-e][1,2,4]triazin-5,7(6H,8H)-dione;8-(4-fluorophenyl)-6-methyl-3-(3-(piperazin-1-yl)propoxy)pyrimidin[5,4-e][1,2,4]triazin-5,7(6H,8H)-dione;8-(4-fluorophenyl)-6-methyl-3-(2-(4-methylpiperazin-1-yl)ethoxy)pyrimidin[5,4-e][1,2,4]triazin-5,7(6H,8H)-dione; 8-(4-fluorophenyl)-6-methyl-3-(3-(4-methylpiperazin-1-yl)propoxy)pyrimidin[5,4-e][1,2,4]triazin-5,7(6H,8H)-dione;3-((2-(diethylamino)ethylamino)methyl)-8-(4-fluorophenyl)-6-methylpyrimidin[5,4-e][1,2,4]triazin-5,7(6H,8H)-dione;3-(3-(diethylamino)propylamino)methyl)-8-(4-fluorophenyl)-6-methylpyrimidin[5,4-e][1,2,4]triazin-5,7(6H,8H)-dione;3-(2-(dimethylamino)ethylamino)methyl)-8-(4-fluorophenyl)-6-methylpyrimidin[5,4-e][1,2,4]triazin-5,7(6H,8H)-dione;3-(3-(dimethylamino)propylamino)methyl)-8-(4-fluorophenyl)-6-methylpyrimido[5,4-e][1,2,4]triazin-5,7(6H,8H)-dione;3-(2-aminoethylamino)methyl)-8-(4-fluorophenyl)-6-methylpyrimido[5,4-e][1,2,4]triazin-5,7(6H,8H)-dione;3-(3-aminopropylamino)methyl)-8-(4-fluorophenyl)-6-methylpyrimido[5,4-e][1,2,4]triazin-5,7(6H,8H)-dione;8-(4-fluorophenyl)-6-methyl-3-(2-(methylamino)ethylamino)methyl)pyrimidin[5,4-e][1,2,4]triazin-5,7(6H,8H)-dione;8-(4-fluorophenyl)-6-methyl-3-(3-(methylamino)propylamino)methyl)pyrimidin[5,4-e][1,2,4]triazin-5,7(6H,8H)-dione;8-(4-fluorophenyl)-3-(2-(2-hydroxylethylamino)ethylamino)methyl)-6-methylpyrimidin[5,4-e][1,2,4]triazin-5,7(6H,8H)-dione;
8-(4-fluorophenyl)-3-(3-(2-hydroxylethylamino)propylamino)methyl)-6-methylpyrimidin[5,4-e][1,2,4]triazin-5,7(6H,8H)-dione;2-amino-N-((8-(4-fluorophenyl)-6-methyl-5,7-dioxo-5,6,7,8-tetrahydropyrimidin[5,4-e][1,2,4]triazin-3-yl)methyl)-N-methylacetamide;3-amino-N-((8-(4-fluorophenyl)-6-methyl-5,7-dioxo-5,6,7,8-tetrahydropyrimidin[5,4-e][1,2,4]triazin-3-yl)methyl)-N-methylpropionamide;2-(dimethylamino)-N-((8-(4-fluorophenyl)-6-methyl-5,7-dioxo-5,6,7,8-tetrahydropyrimidin[5,4-e][1,2,4[triazin-3-yl)methyl)-N-methylacetamide;3-(dimethylamino)-N-((8-(4-fluorophenyl)-6-methyl-5,7-dioxo-5,6,7,8-tetrahydropyrimidin[5,4-e][1,2,4[triazin-3-yl)methyl)-N-methylpropionamide;8-(4-fluorophenyl)-6-methyl-3-(2-(piperidin-1-yl)ethylamino)methyl)pyrimidin[5,4-e][1,2,4]triazin-5,7(6H,8H)-dione;8-(4-fluorophenyl)-6-methyl-3-(3-(piperidin-1-yl)propylamino)methyl)pyrimidin[5,4-e][1,2,4]triazin-5,7(6H,8H)-dione;8-(4-fluorophenyl)-6-methyl-3-(2-morpholinoethylamino)methyl)pyrimidin[5,4-e][1,2,4]triazin-5,7(6H,8H)-dione;8-(4-fluorophenyl)-6-methyl-3-(3-morpholinopropylamino)methyl)pyrimidin[5,4-e][1,2,4]triazin-5,7(6H,8H)-dione;N-(2-(diethylamino)ethyl)-8-(4-fluorophenyl)-6-methyl-5,7-dioxo-5,6,7,8-tetrahydropyrimido[5,4-e][1,2,4]triazin-3-carboxamide;
N-(3-(diethylamino)propyl)-8-(4-fluorophenyl)-6-methyl-5,7-dioxo-5,6,7,8-tetrahydropyrimido[5,4-e][1,2,4]triazin-3-carboxamide;8-(4-fluorophenyl)-6-methyl-N-(2-morpholinoethyl)-5,7-dioxo-5,6,7,8-tetrahydropyrimido[5,4-e][1,2,4]triazin-3-carboxamide;8-(4-fluorophenyl)-6-methyl-N-(3-morpholinopropyl)-5,7-dioxo-5,6,7,8-tetrahydropyrimido[5,4-e][1,2,4]triazin-3-carboxamide;8-(4-fluorophenyl)-6-methyl-5,7-dioxo-N-(2-(piperazin-1-yl)ethyl)-5,6,7,8-tetrahydropyrimido[5,4-e][1,2,4]triazin-3-carboxamide;8-(4-fluorophenyl)-6-methyl-5,7-dioxo-N-(3-(piperazin-1-yl)propyl)-5,6,7,8-tetrahydropyrimido[5,4-e][1,2,4]triazin-3-carboxamide;8-(4-fluorophenyl)-6-methyl-N-(2-(4-methylpiperazin-1-yl)ethyl)-5,7-dioxo-5,6,7,8-tetrahydropyrimidin[5,4-e ][1,2,4]triazin-3-carboxamide; 8-(4-fluorophenyl)-6-methyl-N-(3-(4-methylpiperazin-1-yl)propyl)-5,7-dioxo-5,6,7,8-tetrahydropyrimidin[5,4-e][1,2,4]triazin-3-carboxamide;3-(2-(diethylamino)ethylamino)-8-(3,4-difluorobenzyl)-6-methylpyrimidin[5,4-e][1,2,4]triazin-5,7(6H,8H)-dione;3-(3-(diethylamino)propylamino)-8-(3,4-difluorobenzyl)-6-methylpyrimidin[5,4-e][1,2,4]triazin-5,7(6H,8H)-dione;difluorobenzyl)-3-(2-(dimethylamino)ethylamino)-6-methylpyrimidin[5,4-e][1,2,4]triazin-5,7(6H,8H)-dione;8-(3,4-difluorobenzyl)-3-(3-(dimethylamino)propylamino)-6-methylpyrimidin[5,4-e][1,2,4]triazin-5,7(6H,8H)-dione;3-(2-aminoethylamino)-8-(3,4-difluorobenzyl)-6-methylpyrimido[5,4-e][1,2,4]triazin-5,7(6H,8H)-dione;3-(3-aminopropylamino)-8-(3,4-difluorobenzyl)-6-methylpyrimido[5,4-e][1,2,4]triazin-5,7(6H,8H)-dione;8-(3,4-difluorobenzyl)-6-methyl-3-(2-(methylamino)ethylamino)pyrimidin[5,4-e][1,2,4]triazin-5,7(6H,8H)-dione;8-(3,4-difluorobenzyl)-6-methyl-3-(3-(methylamino)propylamino)pyrimidin[5,4-e][1,2,4]triazin-5,7(6H,8H)-dione;8-(3,4-difluorobenzyl)-3-(2-(2-hydroxylethylamino)ethylamino)-6-methylpyrimidin[5,4-e][1,2,4]triazin-5,7(6H,8H)-dione;8-(3,4-difluorobenzyl)-3-(3-(2-hydroxylethylamino)propylamino)-6-methylpyrimidin[5,4-e][1,2,4]triazin-5,7(6H,8H)-dione;8-(3,4-difluorobenzyl)-3-(2-(2-(2-(dimethylamino)ethylamino)ethylamino)-6-methylpyrimidin[5,4-e][1,2,4]triazin-5,7(6H,8H)-dione;8-(3,4-difluorobenzyl)-3-(3-(2-(dimethylamino)ethylamino)propylamino)-6-methylpyrimidin[5,4-e][1,2,4]triazin-5,7(6H,8H)-dione;3-(2-(2-(2-(diethylamino)ethylamino)ethylamino)-8-(3,4-difluorobenzyl)-6-methylpyrimidin[5,4-e][1,2,4]triazin-5,7(6H,8H)-dione;34342-(diethylamino)ethylamino)propylamino)-8-(3,4-difluorobenzyl)-6-methylpyrimidin[5,4-e][1,2,4]triazin-5,7(6H,8H)-dione;8-(3,4-difluorobenzyl)-6-methyl-3-(2-(piperidin-1-yl)ethylamino)pyrimidin[5,4-e][1,2,4]triazin-5,7(6H,8H)-dione;8-(3,4-difluorobenzyl)-6-methyl-3-(3-(piperidin-1-yl)propylamino)pyrimidin[5,4-e][1,2,4]triazin-5,7(6H,8H)-dione;8-(3,4-difluorobenzyl)-6-methyl-3-(2-morpholinoethylamino)pyrimidin[5,4-e][1,2,4]triazin-5,7(6H,8H)-dione;8-(3,4-difluorobenzyl)-6-methyl-3-(3-morpholinopropylamino)pyrimidin[5,4-e][1,2,4]triazin-5,7(6H,8H)-dione;difluorobenzyl)-6-methyl-3-(2-(piperazin-1-yl)ethylamino)pyrimidin[5,4-e][1,2,4]triazin-5,7(6H,8H)-dione;
8-(3,4-difluorobenzyl)-6-methyl-3-(3-(piperazin-1-yl)propylamino)pyrimidin[5,4-e][1,2,4]triazin-5,7(6H,8H)-dione;8-(3,4-difluorobenzyl)-6-methyl-3-(2-(4-methylpiperazin-1-yl)ethylamino)pyrimidin[5,4-e][1,2,4]triazin-5,7(6H,8H)-dione;8-(3,4-difluorobenzyl)-6-methyl-3-(3-(4-methylpiperazin-1-yl)propylamino)pyrimidin[5,4-e][1,2,4]triazin-5,7(6H,8H)-dione;3-(2-(diethylamino)ethoxy)-8-(3,4-difluorobenzyl)-6-methylpyrimidin[5,4-e][1,2,4]triazin-5,7(6H,8H)-dione;3-(3-(diethylamino)propoxy)-8-(3,4-difluorobenzyl)-6-methylpyrimidin[5,4-e][1,2,4]triazin-5,7(6H,8H)-dione;8-(3,4-difluorobenzyl)-3-(2-(dimethylamino)ethoxy)-6-methylpyrimidin[5,4-e][1,2,4]triazin-5,7(6H,8H)-dione;difluorobenzyl)-3-(3-(dimethylamino)propoxy)-6-methylpyrimidin[5,4-e][1,2,4]triazin-5,7(6H,8H)-dione;3-(2-aminoethoxy)-8-(3,4-difluorobenzyl)-6-methylpyrimido[5,4-e][1,2,4]triazin-5,7(6H,8H)-dione;3-(3-aminopropoxy)-8-(3,4-difluorobenzyl)-6-methylpyrimido[5,4-e][1,2,4]triazin-5,7(6H,8H)-dione;8-(3,4-difluorobenzyl)-6-methyl-3-(2-(methylamino)ethoxy)pyrimidin[5,4-e][1,2,4]triazin-5,7(6H,8H)-dione;difluorobenzyl)-6-methyl-3-(3-(methylamino)propoxy)pyrimidin[5,4-e][1,2,4]triazin-5,7(6H,8H)-dione;8-(3,4-difluorobenzyl)-3-(2-(2-hydroxylethylamino)ethoxy)-6-methylpyrimidin[5,4-e][1,2,4]triazin-5,7(6H,8H)-dione;8-(3,4-difluorobenzyl)-3-(3-(2-hydroxylethylamino)propoxy)-6-methylpyrimidin[5,4-e][1,2,4]triazin-5,7(6H,8H)-dione;8-(3,4-difluorobenzyl)-3-(2-(2-(2-(dimethylamino)ethylamino)ethoxy)-6-methylpyrimidin[5,4-e][1,2,4]triazin-5,7(6H,8H)-dione;8-(3,4-difluorobenzyl)-3-(3-(2-(dimethylamino)ethylamino)propoxy)-6-methylpyrimidin[5,4-e][1,2,4]triazin-5,7(6H,8H)-dione;3-(2-(2-(2-(diethylamino)ethylamino)ethoxy)-8-(3,4-difluorobenzyl)-6-methylpyrimidin[5,4-e][1,2,4]triazin-5,7(6H,8H)-dione;3-(3-(2-(diethylamino)ethylamino)propoxy)-8-(3,4-difluorobenzyl)-6-methylpyrimidin[5,4-e][1,2,4]triazin-5,7(6H,8H)-dione;8-(3,4-difluorobenzyl)-6-methyl-3-(2-(piperidin-1-yl)ethoxy)pyrimidin[5,4-e][1,2,4]triazin-5,7(6H,8H)-dione;8-(3,4-difluorobenzyl)-6-methyl-3-(3-(piperidin-1-yl)propoxy)pyrimidin[5,4-e][1,2,4]triazin-5,7(6H,8H)-dione;8-(3,4-difluorobenzyl)-6-methyl-3-(2-morpholinoethoxy)pyrimidin[5,4-e][1,2,4]triazin-5,7(6H,8H)-dione;8-(3,4-difluorobenzyl)-6-methyl-3-(3-morpholinopropoxy)pyrimidin[5,4-e][1,2,4]triazin-5,7(6H,8H)-dione; 8-(3,4-difluorobenzyl)-6-methyl-3-(2-(piperazin-1-yl)ethoxy)pyrimidin[5,4-e][1,2,4]triazin-5,7(6H,8H)-dione;8-(3,4-difluorobenzyl)-6-methyl-3-(3-(piperazin-1-yl)propoxy)pyrimidin[5,4-e][1,2,4]triazin-5,7(6H,8H)-dione;8-(3,4-difluorobenzyl)-6-methyl-3-(2-(4-methylpiperazin-1-yl)ethoxy)pyrimidin[5,4-e][1,2,4]triazin-5,7(6H,8H)-dione;8-(3,4-difluorobenzyl)-6-methyl-3-(3-(4-methylpiperazin-1-yl)propoxy)pyrimidin[5,4-e][1,2,4]triazin-5,7(6H,8H)-one3-((2-(diethylamino)ethylamino)methyl)-8-(3,4-difluorobenzyl)-6-methylpyrimidin[5,4-e][1,2,4]triazin-5,7(6H,8H)-dione;3-((3-(diethylamino)propylamino)methyl)-8-(3,4-difluorobenzyl)-6-methylpyrimidin[5,4-e][1,2,4]triazin-5,7(6H,8H)-dione;8-(3,4-difluorobenzyl)-3-(2-(dimethylamino)ethylamino)methyl)-6-methylpyrimidin[5,4-e][1,2,4]triazin-5,7(6H,8H)-dione;8-(3,4-difluorobenzyl)-3-(3-(dimethylamino)propylamino)methyl)-6-methylpyrimidin[5,4-d][1,2,4]triazin-5,7(6H,8H-dione;3-(2-aminoethylamino)methyl)-8-(3,4-difluorobenzyl)-6-methylpyrimidin[5,4-e][1,2,4]triazin-5,7(6H,8H)-dione;3-(3-aminopropylamino)methyl)-8-(3,4-difluorobenzyl)-6-methylpyrimidin[5,4-e][1,2,4]triazin-5,7(6H,8H)-dione;difluorobenzyl)-6-methyl-3-((2-(methylamino)ethylamino)methyl)pyrimidin[5,4-e][1,2,4]triazin-5,7(6H,8H)-dione;8-(3,4-difluorobenzyl)-6-methyl-3-(3-(methylamino)propylamino)methyl)pyrimidin[5,4-e][1,2,4]triazin-5,7(6H,8H)-dione;difluorobenzyl)-3-((2-(2-hydroxylethylamino)ethylamino)methyl)-6-methylpyrimidin[5,4-e][1,2,4]triazin-5,7(6H,8H)-dione;8-(3,4-difluorobenzyl)-3-(3-(2-hydroxylethylamino)propylamino)methyl)-6-methylpyrimidin[5,4-e][1,2,4]triazin-5,7(6H,8H)-dione;2-amino-N-((8-(3,4-difluorobenzyl)-6-methyl-5,7-dioxo-5,6,7,8-tetrahydropyrimido[5,4-e][1,2,4[triazin-3-yl)methyl)-N-methylacetamide;3-amino-N-((8-(3,4-difluorobenzyl)-6-methyl-5,7-dioxo-5,6,7,8-tetrahydropyrimido[5,4-e][1,2,4[triazin-3-yl)methyl)-N-methylpropionamide;N-(8-(3,4-difluorobenzyl)-6-methyl-5,7-dioxo-5,6,7,8-tetrahydropyrimidin[5,4-e][1,2,4]triazin-3-yl)methyl)-2-(dimethylamino)-N-methylacetamide;N-(8-(3,4-difluorobenzyl)-6-methyl-5,7-dioxo-5,6,7,8-tetrahydropyrimidin[5,4-e][1,2,4]triazin-3-yl)methyl)-3-(dimethylamino)-N-methylpropionamide;
difluorobenzyl-6-methyl-3-((2-(piperidin-1-yl)ethylamino)methyl)pyrimidin[5,4-e][1,2,4]triazin-5,7(6H,8H)-dione;8-(3,4-difluorobenzyl)-6-methyl-3-(3-(piperidin-1-yl)propylamino)methyl)pyrimidinyl4-(6,8-dimethyl-5,7-dioxo-5,6,7,8-tetrahydropyrimidin[5,4-e][1,2,4]triazin-3-yl)-N-(3-(piperazin-1-yl)propyl)benzamide;3-(4-(2-aminoethoxy)phenyl)-8-cyclopentyl-6-methylpyrimido[5,4-e][1,2,4]triazin-5,7(6H,8H)-dione;3-(4-(3-aminopropoxy)phenyl)-8-cyclopentyl-6-methylpyrimido[5,4-e][1,2,4]triazin-5,7(6H,8H)-dione;8-cyclopentyl-3-(4-(2-(dimethylamino)ethoxy)phenyl)-6-methylpyrimido[5,4-e][1,2,4]triazin-5,7(6H,8H)-dione;8-cyclopentyl-3-(4-(3-(dimethylamino)propoxy)phenyl)-6-methylpyrimidin[5,4-e][1,2,4]triazin-5,7(6H,8H)-dione;8-cyclopentyl-3-(4-(2-(2-(diethylamino)ethoxy)phenyl)-6-methylpyrimidin[5,4-e][1,2,4]triazin-5,7(6H,8H)-dione;8-cyclopentyl-3-(4-(3-(diethylamino)propoxy)phenyl)-6-methylpyrimidin[5,4-e][1,2,4]triazin-5,7(6H,8H)-dione;8-cyclopentyl-3-(4-(2-(2-(2-hydroxylethylamino)ethoxy)phenyl)-6-methylpyrimidin[5,4-e][1,2,4]triazin-5,7(6H,8H)-dione;8-cyclopentyl-3-(4-(3-(2-(hydroxylethylamino)propoxy)phenyl)-6-methylpyrimidin[5,4-e][1,2,4]triazin-5,7(6H,8H)-dione;8-cyclopentyl-3-(4-(2-(2-(2-(dimethylamino)ethylamino)ethoxy)phenyl)-6-methylpyrimidin[5,4-e][1,2,4]triazin-5,7(6H,8H)-dione;8-cyclopentyl-3-(4-(3-(2-(dimethylamino)ethylamino)propoxy)phenyl)-6-methylpyrimidin[5,4-e][1,2,4]triazin-5,7(6H,8H)-dione;8-cyclopentyl-3-(4-(2-(2-(2-(diethylamino)ethylamino)ethoxy)phenyl)-6-methylpyrimidin[5,4-e][1,2,4]triazin-5,7(6H,8H)-dione;8-cyclopentyl-3-(4-(3-(2-(2-(diethylamino)ethylamino)propoxy)phenyl)-6-methylpyrimidin[5,4-e][1,2,4]triazin-5,7(6H,8H)-dione;8-cyclopentyl-6-methyl-3-(4-(2-(piperidin-1-yl)ethoxy)phenyl)pyrimido[5,4-e][1,2,4]triazin-5,7(6H,8H)-dione;8-cyclopentyl-6-methyl-3-(4-(3-(piperidin-1-yl)propoxy)phenyl)pyrimido[5,4-e][1,2,4]triazin-5,7(6H,8H)-dione;8-cyclopentyl-6-methyl-3-(4-(2-morpholinoethoxy)phenyl)pyrimido[5,4-e][1,2,4]triazin-5,7(6H,8H)-dione;8-cyclopentyl-6-methyl-3-(4-(2-(piperazin-1-yl)ethoxy)phenyl)pyrimido[5,4-e][1,2,4]triazin-5,7(6H,8H)-dione;8-cyclopentyl-6-methyl-3-(4-(3-(piperazin-1-yl)propoxy)phenyl)pyrimido[5,4-e][1,2,4]triazin-5,7(6H,8H)-dione;8-cyclopentyl-6-methyl-3-(4-(2-(4-methylpiperazin-1-yl)ethoxy)phenyl)pyrimido[5,4-e][1,2,4]triazin-5,7(6H,8H)-dione;
8-cyclopentyl-6-methyl-3-(4-(3-(4-methylpiperazin-1-yl)propoxy)phenyl)pyrimido[5,4-e][1,2,4]triazin-5,7(6H,8H)-dione;3-(4-(2-(4-(2-aminoacetyl)piperazin-1-yl)ethoxy)phenyl)-8-cyclopentyl-6-methylpyrimidin[5,4-e][1,2,4]triazin-5,7(6H,8H)-dione;3-(4-(3-(4-(2-aminoacetyl)piperazin-1-yl)propoxy)phenyl)-8-cyclopentyl-6-methylpyrimidin[5,4-e][1,2,4]triazin-5,7(6H,8H)-dione;3-(4-(2-(3-aminopyrrolidin-1-yl)ethoxy)phenyl)-8-cyclopentyl-6-methylpyrimidin[5,4-e][1,2,4]triazin-5,7(6H,8H)-dione;3-(4-(3-(3-aminopyrrolidin-1-yl)propoxy)phenyl)-8-cyclopentyl-6-methylpyrimidin[5,4-e][1,2,4]triazin-5,7(6H,8H)-dione;
3-(4-(2-aminoethylamino)phenyl)-8-cyclopentyl-6-methylpyrimido[5,4-e][1,2,4]triazin-5,7(6H,8H)-dione;3-(4-(3-aminopropylamino)phenyl)-8-cyclopentyl-6-methylpyrimido[5,4-e][1,2,4]triazin-5,7(6H,8H)-dione;8-cyclopentyl-3-(4-(2-(dimethylamino)ethylamino)phenyl)-6-methylpyrimidin[5,4-e][1,2,4]triazin-5,7(6H,8H)-dione;8-cyclopentyl-3-(4-(3-(dimethylamino)propylamino)phenyl)-6-methylpyrimidin[5,4-e][1,2,4]triazin-5,7(6H,8H)-dione;8-cyclopentyl-3-(4-(2-(2-(diethylamino)ethylamino)phenyl)-6-methylpyrimido[5,4-e][1,2,4]triazin-5,7(6H,8H)-dione;8-cyclopentyl-3-(4-(3-(diethylamino)propylamino)phenyl)-6-methylpyrimidin[5,4-e][1,2,4]triazin-5,7(6H,8H)-dione;8-cyclopentyl-3-(4-(2-(2-(2-hydroxylethylamino)ethylamino)phenyl)-6-methylpyrimidin[5,4-e][1,2,4]triazin-5,7(6H,8H)-dione;8-cyclopentyl-3-(4-(3-(2-(hydroxylethylamino)propylamino)phenyl)-6-methylpyrimidin[5,4-e][1,2,4]triazin-5,7(6H,8H)-dione;8-cyclopentyl-6-methyl-3-(4-(2-(piperidin-1-yl)ethylamino)phenyl)pyrimido[5,4-e][1,2,4]triazin-5,7(6H,8H)-dione;8-cyclopentyl-6-methyl-3-(4-(3-(piperidin-1-yl)propylamino)phenyl)pyrimido[5,4-e][1,2,4]triazin-5,7(6H,8H)-dione;8-cyclopentyl-6-methyl-3-(4-(2-morpholinoethylamino)phenyl)pyrimido[5,4-e][1,2,4]triazin-5,7(6H,8H)-dione;8-cyclopentyl-6-methyl-3-(4-(3-morpholinopropylamino)phenyl)pyrimido[5,4-e][1,2,4]triazin-5,7(6H,8H)-dione;8-cyclopentyl-6-methyl-3-(4-(2-(pyrrolidin-1-yl)ethylamino)phenyl)pyrimido[5,4-e][1,2,4]triazin-5,7(6H,8H)-dione;8-cyclopentyl-6-methyl-3-(4-(3-(pyrrolidin-1-yl)propylamino)phenyl)pyrimido[5,4-e][1,2,4]triazin-5,7(6H,8H)-dione;N-(2-aminoethyl)-4-(8-cyclopentyl-6-methyl-5,7-dioxo-5,6,7,8-tetrahydropyrimidin[5,4-e][1,2,4]triazin-3-yl)benzamide;N-(3-aminopropyl)-4-(8-cyclopentyl-6-methyl-5,7-dioxo-5,6,7,8-tetrahydropyrimidin[5,4-e][1,2,4]triazin-3-yl)benzamide;4-(8-cyclopentyl-6-methyl-5,7-dioxo5,6,7,8-tetrahydropyrimidin[5,4-e][1,2,4]triazin-3-yl)-N-(2-(diethylamino)ethyl)benzamide;4-(8-cyclopentyl-6-methyl-5,7-dioxo5,6,7,8-tetrahydropyrimidin[5,4-e][1,2,4]triazin-3-yl)-N-(3-(diethylamino)propyl)benzamide;4-(8-cyclopentyl-6-methyl-5,7-dioxo5,6,7,8-tetrahydropyrimidin[5,4-e][1,2,4]triazin-3 -yl)-N-(2-(piperazin-1 -yl)ethyl)benzamide;4-(8-cyclopentyl-6-methyl-5,7-dioxo5,6,7,8-tetrahydropyrimidin[5,4-e][1,2,4]triazin-3-yl)-N-(3-(piperazin-1-yl)propyl)benzamide;3-(4-(2-aminoethoxy)phenyl)-8-(4-fluorophenyl)-6-methylpyrimido[5,4-e][1,2,4]triazin-5,7(6H,8H)-dione;3-(4-(3-aminopropoxy)phenyl)-8-(4-fluorophenyl)-6-methylpyrimido[5,4-e][1,2,4]triazin-5,7(6H,8H)-dione;3-(4-(2-(dimethylamino)ethoxy)phenyl)-8-(4-fluorophenyl)-6-methylpyrimidin[5,4-e][1,2,4]triazin-5,7(6H,8H)-dione;3-(4-(3-(dimethylamino)propoxy)phenyl)-8-(4-fluorophenyl)-6-methylpyrimidin[5,4-e][1,2,4]triazin-5,7(6H,8H)-dione;3-(4-(2-(diethylamino)ethoxy)phenyl)-8-(4-fluorophenyl)-6-methylpyrimidin[5,4-e][1,2,4]triazin-5,7(6H,8H)-dione;3-(4-(3-(diethylamino)propoxy)phenyl)-8-(4-fluorophenyl)-6-methylpyrimidin[5,4-e][1,2,4]triazin-5,7(6H,8H)-dione;8-(4-fluorophenyl)-3-(4-(2-(2-hydroxylethylamino)ethoxy)phenyl)-6-methylpyrimidin[5,4-e][1,2,4]triazin-5,7(6H,8H)-dione;8-(4-fluorophenyl)-3-(4-(3-(2-hydroxylethylamino)propoxy)phenyl)-6-methylpyrimidin[5,4-e][1,2,4]triazin-5,7(6H,8H)-dione;
3-(4-(2-(2-(2-(dimethylamino)ethylamino)ethoxy)phenyl)-8-(4-fluorophenyl)-6-methylpyrimidin[5,4-e][1,2,4]triazin-5,7(6H,8H)-dione;3-(4-(2-(2-(2-(diethylamino)ethylamino)ethoxy)phenyl)-8-(4-fluorophenyl)-6-methylpyrimidin[5,4-e][1,2,4]triazin-5,7(6H,8H)-dione;3-(4-(3-(2-(2-(diethylamino)ethylamino)propoxy)phenyl)-8-(4-fluorophenyl)-6-methylpyrimidin[5,4-e][1,2,4]triazin-5,7(6H,8H)-dione;8-(4-fluorophenyl)-6-methyl-3-(4-(2-(piperidin-1-yl)ethoxy)phenyl)pyrimido[5,4-e][1,2,4]triazin-5,7(6H,8H)-dione;8-(4-fluorophenyl)-6-methyl-3-(4-(3-(piperidin-1-yl)propoxy)phenyl)pyrimido[5,4-e][1,2,4]triazin-5,7(6H,8H)-dione;8-(4-fluorophenyl)-6-methyl-3-(4-(2-morpholinoethoxy)phenyl)pyrimido[5,4-e][1,2,4]triazin-5,7(6H,8H)-dione;8-(4-fluorophenyl)-6-methyl-3-(4-(3-morpholinopropoxy)phenyl)pyrimido[5,4-e][1,2,4]triazin-5,7(6H,8H)-dione;8-(4-fluorophenyl)-6-methyl-3-(4-(2-(piperazin-1-yl)ethoxy)phenyl)pyrimido[5,4-e][1,2,4]triazin-5,7(6H,8H)-dione; 8-(4-fluorophenyl)-6-methyl-3-(4-(3-(piperazin-1-yl)propoxy)phenyl)pyrimido [5,4-e][1,2,4]triazin-5,7(6H,8H)-dione; 8-(4-fluorophenyl)-6-methyl-3-(4-(2-(4-methylpiperazin-1 - yl)ethoxy)phenyl)pyrimido[5,4-e] [1,2,4]triazin-5,7(6H,8H)-dione;8-(4-fluorophenyl)-6-methyl-3-(4-(3-(4-methylpiperazin-1-yl)propoxy)phenyl)pyrimido[5,4-e] [1,2,4]triazin-5,7(6H,8H)-dione;3-(4-(2-(4-(2-aminoacetyl)piperazin-1-yl)ethoxy)phenyl)-8-(4-fluorophenyl)-6-methylpyrimidin[5,4-e][1,2,4]triazin-5,7(6H,8H)-dione;3-(4-(3-(4-(2-aminoacetyl)piperazin-1-yl)propoxy)phenyl)-8-(4-fluorophenyl)-6-methylpyrimidin[5,4-e][1,2,4]triazin-5,7(6H,8H)-dione;3-(4-(2-(3-aminopyrrolidin-1-yl)ethoxy)phenyl)-8-(4-fluorophenyl)-6-methylpyrimidin[5,4-e][1,2,4]triazin-5,7(6H,8H)-dione;3-(4-(3-(3-aminopyrrolidin-1-yl)propoxy)phenyl)-8-(4-fluorophenyl)-6-methylpyrimidin[5,4-e][1,2,4]triazin-5,7(6H,8H)-dione;3-(4-(2-aminoethylamino)phenyl)-8-(4-fluorophenyl)-6-methylpyrimido[5,4-e][1,2,4]triazin-5,7(6H,8H)-one3-(4-(3-aminopropylamino)phenyl)-8-(4-fluorophenyl)-6-methylpyrimido[5,4-e][1,2,4]triazin-5,7(6H,8H)-dione;3-(4-(2-(dimethylamino)ethylamino)phenyl)-8-(4-fluorophenyl)-6-methylpyrimidin[5,4-e][1,2,4]triazin-5,7(6H,8H)-dione;3-(4-(3-(dimethylamino)propylamino)phenyl)-8-(4-fluorophenyl)-6-methylpyrimidin[5,4-e][1,2,4]triazin-5,7(6H,8H)-dione;3-(4-(2-(diethylamino)ethylamino)phenyl)-8-(4-fluorophenyl)-6-methylpyrimidin[5,4-e][1,2,4]triazin-5,7(6H,8H)-dione;3-(4-(3-(diethylamino)propylamino)phenyl)-8-(4-fluorophenyl)-6-methylpyrimidin[5,4-e][1,2,4]triazin-5,7(6H,8H)-dione;8-(4-fluorophenyl)-3-(4-(2-(2-hydroxylethylamino)ethyl(amino)phenyl)-6-methylpyrimidin[5,4-e][1,2,4]triazin-5,7(6H,8H)-dione;8-(4-fluorophenyl)-3-(4-(3-(2-hydroxyethyl(amino)propylamino)phenyl)-6-methylpyrimido[5,4-e][1,2,4]triazin-5,7(6H,8H)-dione;8-(4-fluorophenyl)-6-methyl-3-(4-(2-(2-piperidin-1-yl)ethylamino)phenyl)pyrimido[5,4-d][1,2,4]triazin-5,7(6H,8H)-dione;8-(4-fluorophenyl)-6-methyl-3-(4-(2-morpholinoethylamino)phenyl)pyrimido[5,4-e][1,2,4]triazin-5,7(6H,8H)-dione; 8-(4-fluorophenyl)-6-methyl-3-(4-(3-morpholinopropylamino)phenyl)pyrimido[5,4-e][1,2,4]triazin-5,7(6H,8H)-dione;8-(4-fluorophenyl)-6-methyl-3-(4-(2-(pyrrolidin-1-yl)ethylamino)phenyl)pyrimido[5,4-e][1,2,4]triazin-5,7(6H,8H)-dione;
8-(4-fluorophenyl)-6-methyl-3-(4-(3-(pyrrolidin-1-yl)propylamino)phenyl)pyrimido[5,4-e][1,2,4]triazin-5,7(6H,8H)-dione;N-(2-aminoethyl)-4-(8-(4-fluorophenyl)-6-methyl5,7-dioxo-5,6,7,8-tetrahydropyrimido[5,4-e][1,2,4]triazin-3-yl)benzamide;N-(3-aminopropyl)-4-(8-(4-fluorophenyl))-6-methyl-5,7-dioxo-5,6,7,8-tetrahydropyrimido[5,4-e][1,2,4]triazin-3-yl)benzamide;N-(2-(diethylamino)ethyl)-4-(8-(4-fluorophenyl)-6-methyl-5,7-dioxo-5,6,7,8-tetrahydropyrimido[5,4-e][1,2,4]triazin-3-yl)benzamide;N-(3-(diethylamino)propyl)-4-(Q-(4-fluorophenyl)-6-methyl-5,7-dioxo-5,6,7,8-tetrahydropyrimido[5,4-e][1,2,4]triazin-3-yl)benzamide;4-(8-(4-fluorophenyl)-6-methyl-5,7-dioxo-5,6,7,8-tetrahydropyrimidin[5,4-e][1,2,4]triazin-3-yl)-N-(2-(piperazin-1-yl)ethyl)benzamide; 4-(8-(4-fluorophenyl)-6-methyl-5,7-dioxo-5,6,7,8-tetrahydropyrimidin[5,4-e][1,2,4]triazin-3-yl)-N-(3-(piperazin-1-yl)propyl)benzamide;3-(4-(2-aminoethoxy)phenyl)-8-(3,4-difluorobenzyl)-6-methylpyrimidin[5,4-e][1,2,4]triazin-5,7(6H,8H)-dione;3-(4-(3-aminopropoxy)phenyl)-8-(3,4-difluorobenzyl)-6-methylpyrimidin[5,4-e][1,2,4]triazin-5,7(6H,8H)-dione;8-(3,4-difluorobenzyl)-3-(4-(2-(dimethylamino)ethoxy)phenyl)-6-methylpyrimidin[5,4-e][1,2,4]triazin-5,7(6H,8H)-dione;8-(3,4-difluorobenzyl)-3-(4-(3-(dimethylamino)propoxy)phenyl)-6-methylpyrimidin[5,4-e][1,2,4]triazin-5,7(6H,8H)-dione;3-(4-(2-(diethylamino)ethoxy)phenyl)-8-(3,4-difluorobenzyl)-6-methylpyrimidin[5,4-e][1,2,4]triazin-5,7(6H,8H)-dione;3-(4-(3-(diethylamino)propoxy)phenyl)-8-(3,4-difluorobenzyl)-6-methylpyrimidin[5,4-e][1,2,4]triazin-5,7(6H,8H)-dione;8-(3,4-difluorobenzyl)-3-(4-(2-(2-hydroxylethylamino)ethoxy)phenyl)-6-methylpyrimidin[5,4-e][1,2,4]triazin-5,7(6H,8H)-dione;8-(3,4-difluorobenzyl)-3-(4-(3-(2-hydroxylethylamino)propoxy)phenyl)-6-methylpyrimidin[5,4-e][1,2,4]triazin-5,7(6H,8H)-dione;8-(3,4-difluorobenzyl)-3-(4-(2-(2-(2-(dimethylamino)ethylamino)ethoxy)phenyl)-6-methylpyrimidin[5,4-e][1,2,4]triazin-5,7(6H,8H)-dione;8-(3,4-difluorobenzyl)-3-(4-(3-(2-(dimethylamino)ethylamino)propoxy)phenyl)-6-methylpyrimidin[5,4-e][1,2,4]triazin-5,7(6H,8H)-dione;3-(4-(2-(2-(2-(diethylamino)ethylamino)ethoxy)phenyl)-8-(3,4-difluorobenzyl)-6-methylpyrimidin[5,4-e][1,2,4]triazin-5,7(6H,8H)-dione;3444342-(diethylamino)ethylamino)propoxy)phenyl)-8-(3,4-difluorobenzyl)-6-methylpyrimidin[5,4-e][1,2,4]triazin-5,7(6H,8H)-dione;8-(3,4-difluorobenzyl)-6-methyl-3-(4-(2-(piperidin-1 -yl)ethoxy)phenyl)pyrimido[5,4-e][1,2,4]triazin-5,7(6H,8H)-dione;8-(3,4-difluorobenzyl)-6-methyl-3-(4-(3-(piperidin-1-yl)propoxy)phenyl)pyrimido[5,4-e][1,2,4]triazin-5-dione8-(3,4-difluorobenzyl)-6-methyl-3 -(4-(2-(piperazin-1 - yl)ethoxy)phenyl)pyrimido[5,4-e][1,2,4]triazin-5,7(6H,8H)-dione;8-(3,4-difluorobenzyl)-6-methyl-3-(4-(3-morpholinopropoxy)phenyl))pyrimido[5,4-e][1,2,4]triazin-5,7(6H,8H)-dione;difluorobenzyl)-6-methyl-3-(4-(2-(4-methylpiperazin-1-yl)ethoxy)phenyl)pyrimido[5,4-e][1,2,4]triazin-5,7(6H,8H)-dione;8-(3,4-difluorobenzyl)-6-methyl-3-(4-(3-(piperazin-1-yl)propoxy)phenyl)pyrimido[5,4-e][1,2,4]triazin-5,7(6H,8H)-dione;3-(4-(2-(4-(2-aminoacetyl)piperazin-1-yl)ethoxy)phenyl)-8-(3,4-difluorobenzyl)-6-methylpyrimido [5,4-e][1,2,4]triazin-5,7(6H,8H)-dione;3-(4-(3-(4-(2-aminoacetyl)piperazin-1-yl)propoxy)phenyl)-8-(3,4-difluorobenzyl)-6-methylpyrimidin[5,4-e][1,2,4]triazin-5,7(6H,8H)-dione;3-(4-(2-(3-aminopyrrolidin-1-yl)ethoxy)phenyl)-8-(3,4-difluorobenzyl)-6-methylpyrimidin[5,4-e][1,2,4]triazin-5,7(6H,8H)-dione;3-(4-(3-(3-aminopyrrolidin-1-yl)propoxy)phenyl)-8-(3,4-difluorobenzyl)-6-methylpyrimidin[5,4-e][1,2,4]triazin-5,7(6H,8H)-dione;3-(4-(2-aminoethylamino)phenyl)-8-(3,4-difluorobenzyl)-6-methylpyrimidin[5,4-e] [1,2,4]triazin-5,7(6H,8H)-dione;3-(4-(3-aminopropylamino)phenyl)-8-(3,4-difluorobenzyl)-6-methylpyrimidin[5,4-e][1,2,4]triazin-5,7(6H,8H)-dione;8-(3,4-difluorobenzyl)-3-(4-(2-(dimethylamino)ethylamino)phenyl)-6-methylpyrimidin[5,4-e][1,2,4]triazin-5,7(6H,8H)-dione;8-(3,4-difluorobenzyl)-3-(4-(3-(dimethylamino)propylamino)phenyl)-6-methylpyrimidin[5,4-e][1,2,4]triazin-5,7(6H,8H)-dione;3-(4-(2-(diethylamino)ethylamino)phenyl)-8-(3,4-difluorobenzyl)-6-methylpyrimidin[5,4-e][1,2,4]triazin-5,7(6H,8H)-dione;3-(4-(3-(diethylamino)propylamino)phenyl)-8-(3,4-difluorobenzyl)-6-methylpyrimidin[5,4-e][1,2,4]triazin-5,7(6H,8H)-dione;8-(3,4-difluorobenzyl)-3-(4-(2-(2-hydroxylethylamino)ethylamino)phenyl)-6-methylpyrimidin[5,4-e][1,2,4]triazin-5,7(6H,8H)-dione;8-(3,4-difluorobenzyl)-3-(4-(3-(2-hydroxylethylamino)propylamino)phenyl)-6-methylpyrimidin[5,4-e][1,2,4]triazin-5,7(6H,8H)-dione;8-(3,4-difluorobenzyl)-6-methyl-3-(4-(2-(piperidin-1-yl)ethylamino)phenyl)pyrimido[5,4-e][1,2,4]triazin-5,7(6H,8H)-dione;8-(3,4-difluorobenzyl)-6-methyl-3-(4-(3-(piperidin-1-yl)propylamino)phenyl)pyrimido[5,4-e][1,2,4]triazin-5,7(6H,8H)-dione;8-(3,4-difluorobenzyl)-6-methyl-3-(4(2-morpholinoethylamino)phenyl)pyrimido[5,4-e][1,2,4]triazin-5,7(6H,8H)-dione;difluorobenzyl)-6-methyl-3-(4-(3-morpholinopropylamino)phenyl)pyrimido[5,4-e][1,2,4]triazin-5,7(6H,8H)-dione;8-(3,4-difluorobenzyl)-6-methyl-3-(4-(2-(pyrrolidin-1-yl)ethylamino)phenyl)pyrimido[5,4-e][1,2,4]triazin-5,7(6H,8H)-dione;difluorobenzyl)-6-methyl-3-(4-(3-(pyrrolidin-1-yl)propylamino)phenyl)pyrimido[5,4-e][1,2,4]triazin-5,7(6H,8H)-dione;N-(2-aminoethyl)-4-(8-(3,4-difluorobenzyl-6-methyl-5,7-dioxo-5,6,7,8-tetrahydropyrimidin[5,4-e][1,2,4]triazin-3-yl)benzamide;N-(3-aminopropyl)-4-(8-(3,4-difluorobenzyl)-6-methyl-5,7-dioxo-5,6,7,8-tetrahydropyrimidin[5,4-e][1,2,4]triazin-3-yl)benzamide;N-(2-(diethylamino)ethyl)-4-(8-(3,4-difluorobenzyl)-6-methyl)-5,7-dioxo-5,6,7,8-tetrahydropyrimido[5,4-d][1,2,4]triazin-3-yl)benzamide;N-(3-(diethylamino)propyl)-4-(8-(3,4-difluorobenzyl)-6-methyl-5,7-dioxo-5,6,7,8-tetrahydropyrimido[5,4-e][1,2,4]triazin-3-yl)benzamide;4-(8-(3,4-difluorobenzyl)-6-methyl-5,7-dioxo-5,6,7,8-tetrahydropyrimidin[5,4-e][1,2,4]triazin-3-yl)-N-(2-(piperazin-1-yl)ethyl)benzamide; 4-(8-(3,4-difluorobenzyl)-6-methyl-5,7-dioxo-5,6,7,8-tetrahydropyrimidin[5,4-e][1,2,4]triazin-3-yl)-N-(3-(piperazin-1-yl)propyl)benzamide;3-(4-(2-aminoethoxy)phenyl)-6-methyl-8-(pyridin-3-ylmethyl)pyrimidin[5,4-e][1,2,4]triazin-5,7(6H,8H)-dione;3-(4-(3-aminopropoxy)phenyl)-6-methyl-8-(pyridin-3-ylmethyl)pyrimidin[5,4-e][1,2,4]triazin-5,7(6H,8H)-dione;
3-(4-(2-(dimethylamino)ethoxy)phenyl)-6-methyl-8-(pyridin-3-ylmethyl)pyrimidin[5,4-e][1,2,4]triazin-5,7(6H,8H)-dione;3-(4-(3-(dimethylamino)propoxy)phenyl)-6-methyl-8-(pyridin-3-ylmethyl)pyrimidin[5,4-e][1,2,4]triazin-5,7(6H,8H)-dione;3-(4-(2-(diethylamino)ethoxy)phenyl)-6-methyl-8-(pyridin-3-ylmethyl)pyrimidin[5,4-e][1,2,4]triazin-5,7(6H,8H)-dione;3-(4-(3-(diethylamino)propoxy)phenyl)-6-methyl-8-(pyridin-3-ylmethyl)pyrimidin[5,4-e][1,2,4]triazin-5,7(6H,8H)-dione;
3-(4-(2-(2-hydroxylethylamino)ethoxy)phenyl)-6-methyl-8-(pyridin-3-ylmethyl)pyrimidin[5,4-e][1,2,4]triazin-5,7(6H,8H)-dione;3-(4-(3-(2-hydroxylethylamino)propoxy)phenyl)-6-methyl-8-(pyridin-3-ylmethyl)pyrimidin[5,4-e][1,2,4]triazin-5,7(6H,8H)-dione;3-(4-(2-(2-(2-(dimethylamino)ethylamino)ethoxy)phenyl)-6-methyl-8-(pyridin-3-ylmethyl)pyrimidin[5,4-e][1,2,4]triazin-5,7(6H,8H)-dione;3444342-(dimethylamino)ethylamino)propoxy)phenyl)-6-methyl-8-(pyridin-3-ylmethyl)pyrimidin[5,4-e][1,2,4]triazin-5,7(6H,8H)-dione;3-(4-(2-(2-(2-(diethylamino)ethylamino)ethoxy)phenyl)-6-methyl-8-(pyridin-3-ylmethyl)pyrimidin[5,4-e][1,2,4]triazin-5,7(6H,8H)-dione;3-(4-(3-(2-(2-(diethylamino)ethylamino)propoxy)phenyl)-6-methyl-8-(pyridin-3-ylmethyl)pyrimidin[5,4-e][1,2,4]triazin-5,7(6H,8H)-dione;6-methyl-3-(4-(2-(piperidin-1-yl)ethoxy)phenyl)-8-(pyridin-3-ylmethyl)pyrimido[5,4-e][1,2,4]triazin-5,7(6H,8H)-dione;6-methyl-3-(4-(3-(piperidin-1-yl)propoxy)phenyl)-8-(pyridin-3-ylmethyl)pyrimido[5,4-e][1,2,4]triazin-5,7(6H,8H)-dione;6-methyl-3-(4-(2-morpholinoethoxy)phenyl)-8-(pyridin-3-ylmethyl)pyrimidin[5,4-e][1,2,4]triazin-5,7(6H,8H)-dione;6-methyl-3-(4-(3-morpholinopropoxy)phenyl)-8-(pyridin-3-ylmethyl)pyrimidin[5,4-e][1,2,4]triazin-5,7(6H,8H)-dione; 6-methyl-3-(4-(2-(piperazin-1-yl)ethoxy)phenyl)-8-(pyridin-3-ylmethyl)pyrimido[5,4-e][1,2,4]triazin-5,7(6H,8H)-dione;6-methyl-3-(4-(3-(piperazin-1-yl)propoxy)phenyl)-8-(pyridin-3-ylmethyl)pyrimidin[5,4-e][1,2,4]triazin-5,7(6H,8H)-dione;6-methyl-3-(4-(2-(4-methylpiperazin-1-yl)ethoxy)phenyl)-8-(pyridin-3-ylmethyl)pyrimido[5,4-e][1,2,4]triazin-5,7(6H,8H)-dione;6-methyl-3-(4-(3-(4-methylpiperazin-1-yl)propoxy)phenyl)-8-(pyridin-3-ylmethyl)pyrimido[5,4-e][1,2,4]triazin-5,7(6H,8H)-dione;3-(4-(2-(4-(2-aminoacetyl)piperazin-1-acyl) ethoxy)phenyl)-6-methyl-8-(pyridin-3-ylmethyl)pyrimidin[5,4-e][1,2,4]triazin-5,7(6H,8H)-dione;3-(4-(3-(4-(2-aminoacetyl)piperazin-1-yl)propoxy)phenyl)-6-methyl-8-(pyridin-3-ylmethyl)pyrimido[5,4-e][1,2,4]triazin-5,7(6H,8H)-dione;3-(4-(2-(3-aminopyrrolidin-1-yl)ethoxy)phenyl)-6-methyl-8-(pyridin-3-ylmethyl)pyrimido[5,4-e][1,2,4]triazin-5,7(6H,8H)-dione;3-(4-(3-(3-aminopyrrolidin-1-yl)propoxy)phenyl)-6-methyl-8-(pyridin-3-ylmethyl)pyrimido[5,4-e][1,2,4]triazin-5,7(6H,8H)-dione;3-(4-(2-aminoethylamino)phenyl)-6-methyl-8-(pyridin-3-ylmethyl)pyrimidin[5,4-e][1,2,4]triazin-5,7(6H,8H)-dione;3-(4-(3-aminopropylamino)phenyl)-6-methyl-8-(pyridin-3-ylmethyl)pyrimidin[5,4-e][1,2,4]triazin-5,7(6H,8H)-dione;3-(4-(2-(dimethylamino)ethylamino)phenyl)-6-methyl-8-(pyridin-3-ylmethyl)pyrimidin[5,4-e][1,2,4]triazin-5,7(6H,8H)-dione;3-(4-(3-(dimethylamino)propylamino)phenyl)-6-methyl-8-(pyridin-3-ylmethyl)pyrimidin[5,4e][1,2,4]triazin-5,7(6H,8H)-dione;3-(4-(2-(diethylamino)ethylamino)phenyl)-6-methyl-8-(pyridin-3-ylmethyl)pyrimidin[5,4-e][1,2,4]triazin-5,7(6H,8H)-dione;3-(4-(3-(diethylamino)propylamino)phenyl)-6-methyl-8-(pyridin-3-ylmethyl)pyrimidin[5,4-e][1,2,4]triazin-5,7(6H,8H)-dione;3-(4-(2-(2-hydroxylethylamino)ethylamino)phenyl)-6-methyl-8-(pyridin-3-ylmethyl)pyrimidin[5,4-e][1,2,4]triazin-5,7(6H,8H)-dione;3-(4-(3-(2-hydroxylethylamino)propylamino)phenyl)-6-methyl-8-(pyridin-3-ylmethyl)pyrimidin[5,4-e][1,2,4]triazin-5,7(6H,8H)-dione; 6-methyl-3-(4-(2-(2-piperidin-1-yl)ethylamino)phenyl)-8-(pyridin-3-ylmethyl)pyrimido[5,4-e][1,2,4]triazin-5,7(6H,8H)-dione;6-methyl-3-(4-(3-(piperidin-1-yl)propylamino)phenyl)-8-(pyridin-3-ylmethyl)pyrimidin[5,4-e][1,2,4]triazin-5,7(6H,8H)-dione;6-methyl-3-(4-(2-morpholinoethylamino)phenyl)-8-(pyridin-3-ylmethyl)pyrimidin[5,4-e][1,2,4]triazin-5,7(6H,8H)-dione;6-methyl-3-(4-(3-morpholinopropylamino)phenyl)-8-(pyridin-3-ylmethyl)pyrimidin[5,4-e][1,2,4]triazin-5,7(6H,8H)-dione;6-methyl-8-(pyridin-3-ylmethyl)-3-(4-(2-(pyrrolidin-1-yl)ethylamino)phenyl)pyrimido[5,4-e][1,2,4]triazin-5,7(6H,8H)-dione;6-methyl-8-(pyridin-3-ylmethyl)-3-(4-(3-(pyrrolidin-1-yl)propylamino)phenyl)pyrimido[5,4-e][1,2,4]triazin-5,7(6H,8H)-dione;
N-(2-aminoethyl)-4-(6-methyl-5,7-dioxa-8-(pyridin-3-ylmethyl)-5,6,7,8-tetrahydropyrimido[5,4-e][1,2,4]triazin-3-yl)benzamide;N-(3-aminopropyl)-4-(6-methyl-5,7-dioxa-8-(pyridin-3-ylmethyl)-5,6,7,0-tetrahydropyrimido[5,4-e][1,2,4]triazin-3-yl)benzamide;N-(2-(diethylamino)ethyl)-4-(6-methyl-5,7-dioxa-8-(pyridin-3-ylmethyl)-5,6,7,8-tetrahydropyrimido[5,4-e][1,2,4]triazin-3-yl)benzamide;N-(3-(diethylamino)propyl)-4-(6-methyl-5,7-dioxo-8-(pyridin-3-ylmethyl)-5,6,7,8-tetrahydropyrimido[5,4-e][1,2,4]triazin-3-yl)benzamide;4-(6-methyl-5,7-dioxa-8-(pyridin-3-ylmethyl)-5,6,7,8-tetrahydropyrimidin[5,4-e][1,2,4]triazin-3-yl)-N-(2-(piperazin-1-yl)ethyl)benzamide;4-(6-methyl-5,7-dioxa-8-(pyridin-3-ylmethyl)-5,6,7,8-tetrahydropyrimidin[5,4-e][1,2,4]triazin-3-yl)-N-(3-(piperazin-1-yl)propyl)benzamide;1-benzyl-6-(4-(2-(diethylamino)ethoxy)phenyl)-3-methylpyrimido[5,4-d]pyrimidin-2,4(1H,3H)-dione;1-(4-fluorobenzyl)-3-methyl-6-(4-(2-(4-methylpiperazin-1-yl)ethoxy)phenyl)pyrimido[5,4-d]pyrimidin-2,4(1H,3H)-dione;1-(3,4-difluorobenzyl)-3-methyl-6-(4-(2-(morpholin-4-yl)ethoxy)phenyl)pyrimido[5,4-d]pyrimidin-2,4(1H,3H)-dione;6-(4-(2-(4-aminoacetylpiperazin-1 -yl)ethoxy)phenyl)-3 -methyl-1 -phenylpyrimidin[5,4-d]pyrimidin-2,4(1H,3H)-dione;6-(4-(2-(4-(4-(dimethylamino)piperidin-1-yl)ethoxy)phenyl)-1-isopropyl-3-methylpyrimidin[5,4-d]pyrimidin-2,4(1H,3H)-dione;6-(4-(2-(3-aminopyrrolidin-1-yl)ethoxy)phenyl)-1-(3-fluorophenyl)-3-methylpyrimidin[5,4-d]pyrimidin-2,4(1H,3H)-dione;
1-cyclopentyl-3-methyl-6-(4-(3-(morpholin-4-yl)propoxy)phenyl)pyrimido[5,4-d]pyrimidin-2,4(1H,3H)-dione;1-cyclopropyl-6-(6-(2-(2-(diethylamino)ethoxy)pyridyl-3-yl)-3-methylpyrimidin[5,4-d]pyrimidin-2,4(1H,3H)-dione;1-cyclopentyl-6-(4-(2-(2-piperidin-1-yl)ethoxy)phenyl)pyrimido[5,4-d]pyrimidin-2,4(1H,3H)-dione;6-(4-(2-(2-(dimethylamino)ethyl)phenyl)-3-methyl-1-phenylpyrimido[5,4-d]pyrimidin-2,4(1H,3H)-dione;1-(3,4-difluorobenzyl)-3-methyl-6-(4-(3-(4-methylpiperazin-1-yl)propyl)phenyl)pyrimido[5,4-d]pyrimidin-2,4(1H,3H)-dione;1-isopropyl-3-methyl-6-(4-(2-(2-piperidin-1-yl)ethylamino)phenyl)pyrimido[5,4-d]pyrimidin-2,4(1H,3H)-dione;1-cyclopropyl-3-(2-(piperidin-1-yl)ethyl)-6-(pyridyl-3-yl)pyrimido[5,4-d]pyrimidin-2,4(1H,3H)-dione;3-(4-(2-aminoethyl)benzyl)-1-cyclopentyl-6-(4-fluorophenyl)pyrimido[5,4-d]pyrimidin-2,4(1H,3H)-dione;1-cyclopentyl-6-(4-carboxyphenyl)-3-methylpyrimido[5,4-d]pyrimidin-2,4(1H,3H)-dione;6-(4-(2-(2-(diethylamino)ethylcarboxamido)phenyl)-1,3-dimethylpyrimido[5,4-d]pyrimidin-2,4(1H,3H)-dione;1,3-dimethyl-6-(4-(2-(4-methylpiperazin-1-yl)ethoxy)phenyl)pyrimido[5,4-d]pyrimidin-2,4(1H,3H)-dione;1-methyl-6-(4-(2-(4-(4-methylpiperazin-1-yl)ethoxy)phenyl)pyrimido[5,4-d]pyrimidin-2,4(1H,3H)-dione;6-(4-(2-(diethylamino)ethoxy)phenyl)-1,3-dimethylpyrimido[5,4-d]pyrimidin-2,4(1H,3H)-dione;6-(4-(2-(diethylamino)ethoxy)phenyl)-1-methylpyrimido[5,4-d]pyrimidin-2,4(1H,3H)-dione;1,3-dimethyl-6-(4-(2-(morpholin-4-yl)ethoxy)phenyl)pyrimido[5,4-d]pyrimidin-2,4(1H,3H)-dione;6-(4-(2-(4-methylpiperazin-1-yl)ethoxy)phenyl)-1,3,8-trimethylpyrimido[5,4-d]pyrimidin-2,4(1H,3H)-dione;1,8-dimethyl-6-(4-(2-(4-methylpiperazin-1-yl)ethoxy)phenyl)pyrimido[5,4-d]pyrimidin-2,4-(1H,3H)-dione;6-(4-(2-(diethylamino)ethoxy)phenyl)-1,3,8-trimethylpyrimido[5,4-d]pyrimidin-2,4 (1H,3H)-dione; 6-(4-(2-(2-(diethylamino)ethoxy)phenyl)-1,8-dimethylpyrimido[5,4-d]pyrimidin-2,4 (1H,3H)-dione; 6-(4-(2-(morpholin-4-yl)ethoxy)phenyl)-1,3,8-trimethylpyrimido[5,4-d]pyrimidin-2,4 (1H,3H)-dione; and mixtures, pharmaceutically acceptable salts or hydrates.

For example, the compound having a structure of Formula I, II, or III can comprise an alkali functional group, and thus can form a pharmaceutically acceptable salt with a pharmaceutically acceptable acid. For example, the pharmaceutically acceptable salt can be relatively nontoxic inorganic or organic acid addition salt of the compound. For example, the salt can be *in situ* prepared in the administration medium or during the preparation of dosage form, or can be prepared by independently reacting the purified compound of the present invention in a free base form with an appropriate organic or inorganic acid, followed by isolating the resultant salt in the subsequent purification. For example, the pharmaceutically acceptable salt can comprise hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, gluconate, lactobionate, lauryl sulfonate, or the like (see, *e.g.,* Berge *et al.,* (1977) "Pharmaceutical Salts", J.Pharm.Sci. 66:1-19).

For example, the pharmaceutically acceptable salt can further comprise a conventional nontoxic salt or quaternary ammonium salt, *e.g*., those derived from a nontoxic organic acid or inorganic acid. For example, the conventional nontoxic salt can comprise those derived from an inorganic acid, such as, hydrochloric acid, hydrobromic acid, sulfuric acid, amino sulfuric acid, phosphoric acid, nitric acid, or the like; an organic acid, such as, acetic acid, propionic acid, succinic acid, glycolic acid, stearic acid, lactic acid, malic acid, tartaric acid, citric acid, ascorbic acid, palmitic acid, maleic acid, hydroxy maleic acid, phenylacetic acid, glutamic acid, benzoic acid, salicylic acid, sulfanilic acid, 2-acetoxy benzoic acid, fumaric acid, toluenesulfonic acid, methanesulfonic acid, ethanedisulfonic acid, oxalic acid, isothionic acid, or the like.

For example, the compound having a structure of Formula I, II, or III can comprise an acidic functional group, and thus can form a pharmaceutically acceptable salt with a pharmaceutically acceptable base. For example, the pharmaceutically acceptable salt can be a relatively nontoxic inorganic or organic base addition salt of the compound. For example, the salt can also be *in situ* prepared in the administration medium or during the preparation of dosage form, or can be prepared by independently reacting the purified compound of the present invention in a free acid form with an appropriate base, such as, a hydroxide, carbonate, or bicarbonate of a pharmaceutically acceptable cation, ammonia, or a pharmaceutically acceptable organic primary, secondary, or tertiary amine. Representative alkali or alkaline earth metal salts comprise lithium, sodium, potassium, calcium, magnesium, and aluminum salts, etc. Representative organic amines for forming a base addition salt comprise ethylamine, diethylamine, triethylamine, ethanolamine, diethanolamine, piperazine, or the like.

For example, the substance capable of interacting with a member involved in the formation of a TCF/β-catenin complex can further comprise a substance down-regulating the expression level and/or activity of the member involved in the formation of a TCF/β-catenin complex. For example, the substance down-regulating the expression level and/or activity of the member involved in the formation of a TCF/β-catenin complex can further comprise siRNA and/or shRNA. For example, the siRNA and the shRNA can comprise the nucleotide sequences of the mRNA(s) of TCF-1, TCF-3, TCF-4, LEF-1, β-catenin, CBP to down-regulate the expression level/activity of the corresponding molecules.

For example, the siRNA can be a type of double-stranded RNA molecules of about 12-35 base pairs in length.

For example, the siRNA can cause the degradation of mRNA by complementary binding with the mRNA.

For example, the siRNA can have a length of 18, 19, 20, 21, 22, 23, 24, 25, 26, 27, 28 bases or base pairs.

For example, the siRNA is artificially prepared.

For example, the siRNA is obtained from intracellular long double-stranded RNA or shRNA via treatment with Dicer enzyme.

In the present application, the shRNA refers to a type of RNAs capable of forming a short hairpin structure.

For example, the shRNA can comprise two short inverted repeats, and a stem loop sequence located between the two short inverted repeats.

For example, the shRNA comprises a sequence of at least 8 consecutive nucleic acids capable of complementary binding to the target mRNA, *e.g*., at least 9, *e.g.,* at least 0, *e.g.,* at least 11, *e.g.,* at least 12, *e.g.,* at least 13, *e.g.,* at least 14, *e.g.,* at least 15, *e.g.,* at least 16.

For example, the β-catenin inhibitor comprises a substance down-regulating the expression level and/or activity of the β-catenin protein.

For example, the substance down-regulating the expression level and/or activity of the β-catenin protein comprises siRNA and/or shRNA.

For example, the substance down-regulating the expression level and/or activity of the β-catenin protein comprises a nucleic acid sequence as shown in any one of SEQ ID NOS: 1-6.

For example, the siRNA comprises a nucleic acid sequence as shown in any one of SEQ ID NOS: 1-6.

For example, the siRNA comprises a nucleic acid sequence as shown in any one of SEQ ID NOS: 1-6, and have 2 or more T bases at 3'-end.

### Ascorbic Acid and A Derivative thereof

In the present application, the ascorbic acid or a derivative thereof comprises a member selected from the group consisting of ascorbic acid (vitamin C), an ascorbic acid derivative, and a combination thereof.

For example, the ascorbic acid derivative can further comprise a dehydroascorbic acid, an ascorbate or a salt of ascorbic acid derivative.

Ascorbic acid is sensitive to the influence of environmental parameters (*e.g.,* light, heat, oxygen, etc.) due to its α-ketolactone structure. It may be unstable in water or other aqueous solution. Ascorbic acid derivatives that have a higher stability than the parent compound are prepared by means of chemically stabilizing the ascorbic acid molecule (*e.g.,* see the U.S. Patents US 5, 137, 723 and US 5, 078, 989), the content of which is incorporated into the present application by reference.

For example, the ascorbic acid derivative can be an ascorbic acid analog. Representative ascorbic acid derivatives comprise those in which at least one hydroxyl group (*e.g.,* 2-OH, 3-OH, 5-OH, 6-OH) in the ascorbic acid molecule is derived with a modification group (*e.g.,* see the U.S. Patent US 5, 078, 989 issued to Ando, et al). For example, one or more of the hydroxyl groups can be substituted with another moiety. For example, the ascorbic acid or a derivative thereof comprises ascorbic acid and at least one ascorbic acid derivative.

For example, the ascorbic acid derivative can comprise free 2-OH and free 3-OH.

For example, the ascorbic acid derivative comprises an ascorbic acid ester derived from at least one of 5-OH and 6-OH.

For example, the ascorbic acid derivative comprise an ester, such as, 6-O-octanoyl-ascorbic acid, 6-O-dodecanoyl-ascorbic acid, 6-O-tetradecanoyl-ascorbic acid, 6-O-octodecanoyl-ascorbic acid, 6-O-dodecanedioyl-ascorbic acid, 6-O-decosanedioyl-ascorbic acid, 6-O-thapsoyl-ascorbic acid, 6-O-decanedioyl-ascorbic acid, or 6-O-hexanedioyl-ascorbic acid.

For example, the ascorbic acid derivative can further comprise an ester in which the lipophilic moiety of the molecule is a mono- or poly-unsaturated fatty acid. For example, the unsaturated fatty acid can comprise a health-associated essential fatty acid, *e.g*., ω-3 (a-linolenic acid), ω-6 or ω-9 fatty acid. For example, it can further comprise an ester containing amino acid residue(s).

For example, the ascorbic acid derivative can further comprise a 2-O-alkyl or 3-O-alkyl derivative of ascorbic acid. The 3-O-alkyl-ascorbic acid is reported by Nihro et al. in Chem. Pharm. Bull.1991, 39: 1731-1735, which is incorporated into the present application by reference.

For example, the ascorbic acid derivative can comprise a glycoside of ascorbic acid; such as, ascorbic acid 1-glycoside, ascorbic acid 2-glycoside, ascorbic acid 3-glycoside, ascorbic acid 5-glycoside, or ascorbic acid 6-glycoside.

For example, the ascorbic acid derivative can comprise 2-O-(α-D-pyranoglucosyl)-ascorbic acid (see, *e.g.,* the U.S. Patent US 5, 137, 723) and 2-O-(β-D-pyranoglucosyl)-ascorbic acid (see, *e.g.,* the U.S. Patent Application No. US 2005/0113312). The above-listed literatures are incorporated into the present application by reference.

For example, the ascorbic acid derivative can comprise a dual-functionalized derivative, such as, *e.g.,* 6-O-acyl-2-O-(α-D-pyranoglucosyl) ascorbic acid (see, *e.g.,* Yamamoto et. al, J.Med.Chem.2002, 45(2): 462-468). This literature is incorporated into the present application by reference.

For example, the ascorbic acid derivative can comprise an ascorbyl phosphate. For example, the ascorbyl phosphate is alkali metal salt, alkali earth metal salt, or transition metal salt. Examples can comprise magnesium ascorbyl phosphate, sodium ascorbyl phosphate (*e.g.,* sodium ascorbyl-2-monophosphate), calcium ascorbyl phosphate, and potassium ascorbyl phosphate, and mixed salts, *e.g*., sodium magnesium ascorbyl phosphate, sodium calcium ascorbyl phosphate, aminopropyl ascorbyl phosphate.

For example, the ascorbyl phosphate can be present as a hydrate, commonly a dihydrate. A representative dihydrate can be purchased from DSM under the product name of STAY-C50, for example.

In the present application, the ascorbic acid derivative comprises a pharmaceutically acceptable ascorbate.

For example, the pharmaceutically acceptable ascorbate comprises an alkali metal salt, such as, sodium salt, potassium salt, etc.; an alkali earth metal salt, such as, calcium salt, magnesium salt, or the like; an alkali amino acid salt, such as, arginine salt or the like; and an organic amine salt, such as, triethanolamine salts, etc.

For example, the ascorbate or the derivative of ascorbic acid can be an edible (*e.g.,* pharmaceutically acceptable) salt, such as, calcium, sodium, magnesium, potassium, or zinc salt, as well as a mixed salt of ascorbic acid or ascorbic acid derivative.

For example, the pharmaceutically acceptable ascorbate comprises sodium ascorbate.

Other well-recognized ascorbic acid derivatives can also be used for the purpose of the present application.

### Carrier and Preparation

In the present application, the dosage forms of the first preparation, the second preparation, and the pharmaceutical composition can comprise tablets, capsules, granules, powders, syrups, suspensions, suppositories, ointments, creams, gels, patches, inhalations, injections, etc. These preparations can be prepared by conventional methods.

For example, in the case of liquid preparations, they can be in a form of solution or suspension in water or other suitable solvents in use.

For example, the tablets and the granules can be coated by well-known methods.

For example, in the case of injections, the active ingredients (including the ascorbic acid or a derivative thereof as described in the present application and the substance affecting the formation of a TCF/β-catenin complex as described in the present application) can be dissolved in water to prepare the preparation, or can be dissolved in normal saline or a dextrose solution as required. For example, a buffer or preservative can also be added.

For example, those can also be provided in any preparation form for oral administration or non-oral administration. For example, those can be prepared as a preparation for oral administration, such as, granule, fine granule, powder, hard capsule, soft capsule, syrup, emulsion, suspension or liquid, or the like.

For example, those can also be prepared as a preparation for non-oral administration, such as, injection for intravenous, intramuscular or subcutaneous administration, infusion, transdermal absorption preparation, transmucosal absorption preparation, nose drop, inhalation preparation, suppositories, etc.

For example, the injection, infusion or the like can be prepared as powdered dosage form such as lyophilized powder, which is used by dissolving in an appropriate aqueous medium (e.g., normal saline or the like) in use.

For example, a sustained-release preparation coated with a polymer or the like can be directly administered into an organ such as the brain.

Persons skilled in the art can properly select the type of additive(s) for preparation (carrier), the ratio of the additive(s) for preparation (carrier) to the active ingredients, or the method for preparing the preparation as used in the manufacture of the pharmaceutical preparation in accordance with the form of the preparation. For example, an inorganic or organic substance, or a solid or liquid substance can be used as the additive(s) for preparation (carrier), *e.g*., in an amount of 1-90% by weight relative to the weight of the active ingredient. For example, the carrier can comprise lactose, dextrose, mannitol, dextrin, cyclodextrin, starch, sucrose, aluminum magnesium metasilicate, synthetic aluminum silicate, sodium carboxymethylcellulose, hydroxypropyl starch, calcium carboxymethylcellulose, ion exchange resin, methylcellulose, gelatin, gum Arabic, hydroxypropyl cellulose, hydroxypropyl methylcellulose, polyvinylpyrrolidone, polyvinyl alcohol, light anhydrous silicic acid, magnesium stearate, talc, tragacanth gum, bentonite, propolis, titania, sorbitan fatty acid ester, sodium lauryl sulfate, glycerin, glyceryl fatty Acid, purified lanolin, glycerogelatin, polysorbate, polyethylene glycol, vegetable oil, wax, liquid paraffin, white petrolatum, fluorocarbon, non-ionic surfactant, propylene glycol, water, or the like.

For example, during the manufacture of solid preparation for oral administration, the active ingredients (including ascorbic acid or a derivative thereof as described in the present application and the substance affecting the formation of a TCF/β-catenin complex as described in the present application) can be mixed with the carrier ingredients (*e.g.,* lactose, starch, crystalline cellulose, calcium lactate, anhydrous silicic acid) to prepare a powder. For example, binders such as sucrose, hydroxylpropylcellulose, polyvinylpyrrolidione or the like and disintegrants such as hydroxylmethylcellulose, calcium hydroxylmethylcellulose or the like can be further added, and the mixture is subject to dry or wet pelleting to prepare granules. For example, in the manufacture of tablets, the powders and/or granules can be subject to direct tableting. For example, lubricants such as magnesium stearate, talc powder, or the like can be further added for tableting. For example, the granules or tablets can also be coated with an enteric base such as hydroxypropyl methylcellulose phthalate, methacrylic acid-methyl methacrylate polymer, or the like to prepare enteric preparations. For example, those can also be coated with ethylcellulose, carnauba wax, hydrogenated oil or the like to prepare a prolonged action preparation. For example, in the manufacture of capsules, powders or granules can be filled in a hard capsule. For example, the active ingredients can be coated with a gelatin film directly or after dissolved in glycerin, polyethylene glycol, sesame oil, olive oil or the like to prepare a soft capsule.

For example, in the manufacture of injections, the active ingredients can be dissolved together with a pH adjuster such as hydrochloric acid, sodium hydroxide, lactose, lactic acid, sodium lactate, disodium hydrogen phosphate or the like and an isotonic agent such as sodium chloride, dextrose, or the like in distilled water for injection. For example, those can be further subject to sterile filtration and filled in an ampule. For example, mannitol, dextrin, cyclodextrin, gelatin or the like can be further added, and the mixture is vacuum lyophilized to prepare a ready-to-use injection. For example, lecithin, polysorbate 80, polyoxyethylene hydrogenated castor oil or the like can be added to the active ingredients, and the mixture can be emulsified in water to prepare an emulsion for injection.

For example, in the manufacture of preparations for rectal administration, the active ingredients can be moistened and dissolved together with a base for suppositories such as cocoa butter, triglyceride fatty acids, diglyceride fatty acids, monoglyceride fatty acid, polyethylene glycol, or the like, and then poured into a mold for cooling. For example, the active ingredients can also be dissolved in polyethylene glycol, soybean oil, or the like, and then coated with a gelatin film.

For example, in the manufacture of external preparations for skin, the active ingredients can be added into white petrolatum, beeswax, liquid paraffin, polyethylene glycol or the like, moistened as required, and kneaded to prepare an ointment. For example, those can also be kneaded with binders such as rosin, alkyl acrylate polymer, or the like, and then spread on a non-woven fabric such as a polyalkyl to prepared a tape-shape preparation.

For example, those can also be prepared as sustained-release preparations such as implantable sheets or a delivery system encapsulated in microcapsules, which can be prepared with a carrier that can prevent immediate removal from the body. For example, biodegradable and biocompatible polymers such as ethylene-vinyl acetate, polyanhydride, polyglycolic acid, collagen, polyorthoester, polylactic acid or the like can be used. Persons skilled in the art can readily prepare those materials. For example, a suspension of liposome can also be used as the pharmaceutically acceptable carrier. The liposome can be prepared as a lipid composition comprising phosphatidylcholine, cholesterol, and PEG-derivatized phosphatidylethanolamine (PEG-PE), which can be prepared to an appropriate size with a filter of appropriate pore size, through appropriate pore size, and purified by reverse phase evaporation.

In the present application, the dose and number of administration can be properly selected in accordance with conditions such as prophylactic and/or therapeutic purpose of disease progress of the subject to be treated, types of diseases, weight and age of patients, or the like. For example, in the case of oral administration, it can be administered once or several times every day, or can be administered every several days. In the case of injections, it can be administered continuously or intermittently.

### Kits

The kit of the present application can comprise the pharmaceutical combination as described in the present application. The pharmaceutical combination can be provided in a form of kit. Various constituents of the pharmaceutical combination can be packaged in different containers, and are mixed before administration or administered separately without mixing.

For example, separate packaging can be for long-term storage without losing the function of the active constituents.

For example, the preparation contained in the kit can each be present in any type of containers in which the ingredients can effectively maintaining the activity for a long term, are not adsorbed by the material of the container, and are not easy to deteriorated. For example, sealed ampules, *e.g*., made from glass, organic polymers such as polycarbonate, polystyrene or the like, ceramic, metal, or any other suitable materials commonly used for holding an agent, etc. Other suitable examples of containers comprise simple bottles made from materials similar to ampules, or packages lined with foils such as aluminum or alloy foils. Other containers comprise tubes, vials, flasks, bottles, syringes, or the like. The container has a sterile access port for bottle with a stopper that can be penetrated with an injection needle.

For example, the kit can further comprise a buffer that is packaged in the presence of a neutral, non-reactive gas, such as nitrogen.

For example, the kit can further comprise auxiliary administration devices, *e.g*., measuring cups, measuring spoons, *e.g*., syringes suitable for injection, infusion tubes, infusion needles, or the like.

For example, the kit can additionally comprise an instruction for use. The instruction for use of the kit constituted of the pharmaceutical composition can be printed on paper or other materials, and/or can be supplied in an electrically or electromagnetically readable manner such as Floppy discs, CD-ROMs, DVD-ROMs, Zip discs, video tapes, audio tapes, or the like. Detailed instruction for use can be physically attached to the kit, or can be posted on a website designated or notified with email by the manufacturer or distributor.

Without being limited by any theory, the following examples are only for illustrating the pharmaceutical combination, application method, and use of the present application or the like, and are not intended to limit the scope of the invention of the application.

### Examples

The drugs used in the examples of the present application are listed in Table 1, all of which are commercially available.

**Table 1: List of Drugs**

| Number | Drug Name | Item Number | Manufacturer |
|---|---|---|---|
| 1 | NCB-0846 | T4011 | Targetmol |
| 2 | PRI-724 | S8262 | Selleck |
| 3 | PKF118-310 | K4394 | SIGMA |
| 4 | sodium ascorbate | S105024 | Aladdin |

Where, the drugs numbered 1-3 are used with DMSO as the solvent, and sodium ascorbate is used with PBS as the solvent.

HEK293 cells, HOS human osteosarcoma cells, 143B human osteosarcoma cells, DB lymphoma cells, SKOV3 cells, 3AO cells, MC38 cells, HCT116 cells, T cells and macrophages in the examples of the present application are all purchased from ATCC.

### Example 1. Inhibitory effect of PKF118-310 in combination with sodium ascorbate on osteosarcoma cells

### 1.1 Cell line and cell culture

### HEK293 cells, HOS human osteosarcoma cells, and 143B human osteosarcoma cells were subcultured in an incubator at 37°C, 5% CO₂ using a RPMI-1640 medium containing 10% fetal bovine serum (FBS).

### 1.2 Detection of cell viability of single-drug administration

(1) Cells were plated in a 10 cm culture plate to grow a density of about 90%. The medium was removed, and the adherent cells were washed once with a PBS buffer. 1 mL of trypsin was added, and immediately discarded after mixing well. The cells were placed in the incubator and digested for about 5 min, and then about 10 ml of fresh medium was added. 10 µL was taken for counting.
(2) After counting, the cells were diluted to 1 × 10⁵ cells/ml. The diluted cells were plated into a 96-well plate with 100 µL per well, i.e., about 10,000 cells per well.
(3) The cells plated in the 96-well plate were cultured for 24 h, and then PKF118-310 with different concentration gradients were added thereto, wherein the gradient was set to five concentration gradients of 0.1 µM, 0.5 µM, 1 µM, 5 µM, 10 µM.
(4) After 24h of dosing, 10 µL of CCK-8 solution was added. The cells were incubated in the incubator for 1-2 hours, and then measured at 490 nm using a microplate reader for the absorbance (OD value) of each well. The results were recorded. The relative cell viability was calculated as follows:

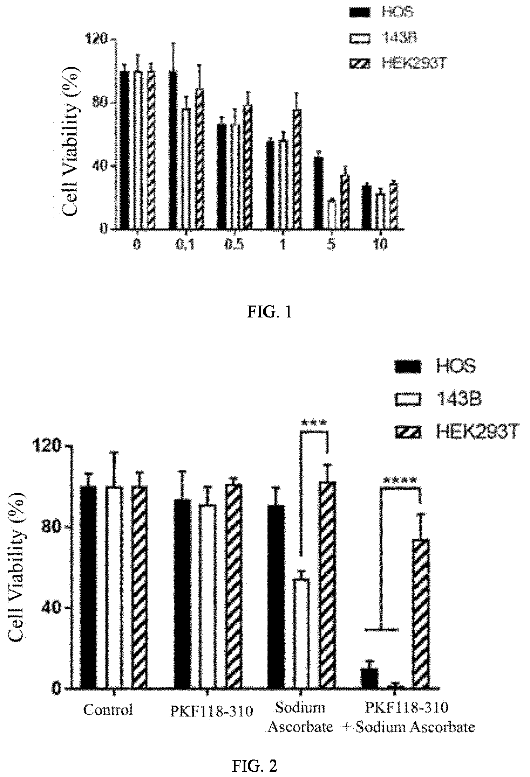

Cell Viability = (Absorbance of Test Group - Absorbance of Blank Group) / (Absorbance of Control Group - Absorbance of Blank Group) × 100%, wherein the test group is a cell group to which different concentrations of drugs are added, the control group is a cell group to which only solvent (DMSO) is added, and the blank group is a group to which no cell is added and only solvent (DMSO) is added. The results of cell viability are obtained by the above equation, and the cell inhibition rate of drugs = 100% - the cell viability. The cell viability of the control group obtained according the equation is 100%. The results of cells treated with PKF 118-310 with the concentration gradient are as shown in Fig. 1.

### 1.3 Cell counts of drug combination administration

(1) Cells were plated in a 10 cm culture plate to grow a density of about 90%. The medium was removed, and the adherent cells were washed once with a PBS buffer. 1 mL of trypsin was added, and immediately discarded after mixing well. The cells were placed in the incubator and digested for about 5 min, and then about 10 ml of fresh medium was added. 10 µL was taken for counting.
(2) 10 µL of the well-mixed cells were taken and mixed into 10 µL of Trypan Blue solution, and counted.
(3) After counting, the cells were diluted to 1 × 10⁵ cells/ml. The diluted cells were mixed well, and plated into a 24-well plate with 500 µL per well, i.e., about 50,000 cells per well.
(4) The cells plated in the 24-well plate were cultured for about 24h, and then drugs were added. Various test groups comprise 5 groups as follows: the single-drug group of PKF 118-310 (with a concentration of 0.1 µM), the group of ascorbic acid (with a concentration of 0.5 mM), the group of PKF118-310 (with a concentration of 0.1 µM) in combination with ascorbic acid (with a concentration of 0.5 mM); the control group that is a cell group to which only solvent DMSO is added; and the blank group that is a group to which no cell is added and only solvent (DMSO) is added.
(5) After 24h of dosing, the medium was discarded. About 150 µL of trypsin was added and then discarded. The cells were digested for 5 min;
(6) After digestion, equivalent amounts of PBS were added into each well and mixed uniformly. Then, 10 µL /well of Trypan Blue solution was added and mixed uniformly. 10 µL of the well-mixed solution was taken and counted. In accordance with "Cell viability = Cell Count of Test Group / Cell Count of Control Group × 100%", the cell viability of various groups was calculated.

### 1.4 Data processing

Data are all expressed as mean ± standard deviation (Mean ± SD), and one-way ANOVA was performed using GraphPad Prism 7.0 software. When P<0.05, the difference was statistically significant. * means P<0.05, ** means P<0.01, *** means P<0.001, **** means P<0.0001.

The coefficient of drug in interaction (CDI) was used to evaluate the nature of the interaction between the two drugs. The test groups were divided into the control group, the group of A drug, the group of B drug and the group of AB in combination, and used for detecting the cell viability in A, B and AB groups, respectively.

CDI = the cell viability in group AB / the cell viability in group A × cell viability in group B. CDI < 1 indicates that the interaction between the two drugs is synergistic; CDI < 0.7 indicates that the interaction between the two drugs is highly synergistic; CDI = 1 indicates the interaction between the two drugs are additive; and CDI > 1 indicates that the interaction between the two drugs is antagonistic.

The results of combination: under the condition of treatment with a single-drug of 0.1 µM of PKF118-310, the HOS cells have cell viability of 94%, the 143B cells have cell viability of 91%; under the condition of treatment with 0.5 mM of sodium ascorbate, the HOS cells have cell viability of 91%, the 143B cells have cell viability of 54%; and under the condition of treatment with 0.1 µM of PKF118-310 in combination with 0.5 mM of sodium ascorbate, the HOS cells have cell viability of 10%, the 143B cells have cell viability of 2%. In accordance with the above-described CDI calculation equation, the CDI value is 0.12 for the HOS cells, and 0.04 for the 143B cells, indicating that they have a synergistic effect therebetween. At the same time, the as compared with the HEK293 cells, the combination of PKF 118-310 with ascorbic acid can specifically inhibit the cell viability of the 143B cells and the HOS cells, and have significantly better inhibitory effects on the 143B cells and the HOS cells than the corresponding single-drug groups. The results are shown in Fig. 2.

### Example 2. Effect of PKF118-310 and sodium ascorbate on migration of osteosarcoma cells

### 2.1 Cell line and cell culture

(1) HOS human osteosarcoma cells and 143B human osteosarcoma cells were subcultured at 37°C, 5% CO₂ in an incubator with a RPMI-1640 medium containing 10% fetal bovine serum.
(2) Cells were plated in a 10 cm culture plate to grow a density of about 90%. The medium was removed, and the adherent cells were washed once with a PBS buffer. 1 mL of trypsin was added, and immediately discarded after mixing well. The cells were placed in the incubator and digested for about 5 min, and then about 10 ml of fresh medium was added. 10 µL was taken for counting.
(3) After counting, the cells were diluted to 1 x 10⁵ cells/ml. The diluted cells were plated into a 12-well plate with 1 mL per well, i.e., about 100,000 cells per well.
(4) The cells plated in the 12-well plate were cultured to a density of more than 80%. Then, a control group (to which DMSO was added), as well as groups to which 0.1 µM of PKF118-310, 0.5 mM Vc, and a combination of 0.1 µM of PKF118-310 with 0.5 mM sodium ascorbate were respectively added were prepared. Parallel lines were drawn with the tip of a 10 µL pipette at the bottom of the plate.
(5) The cells were placed into a 37°C, 5% CO₂ incubator for incubation, and observed and recorded by photographs at 12h and 24h.

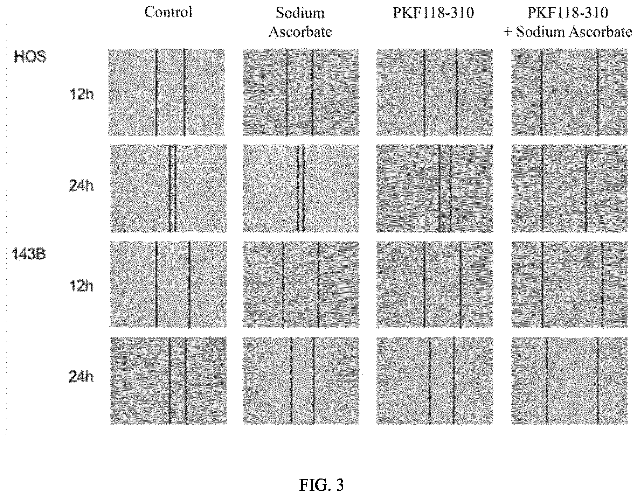

The results are as shown in Fig. 3, and PKF118-310 in combination with Vc can significantly inhibit the migration of HOS human osteosarcoma cells and 143B human osteosarcoma cells.

### Example 3. Inhibitory effect of PKF118-310 and sodium ascorbate on osteosarcoma

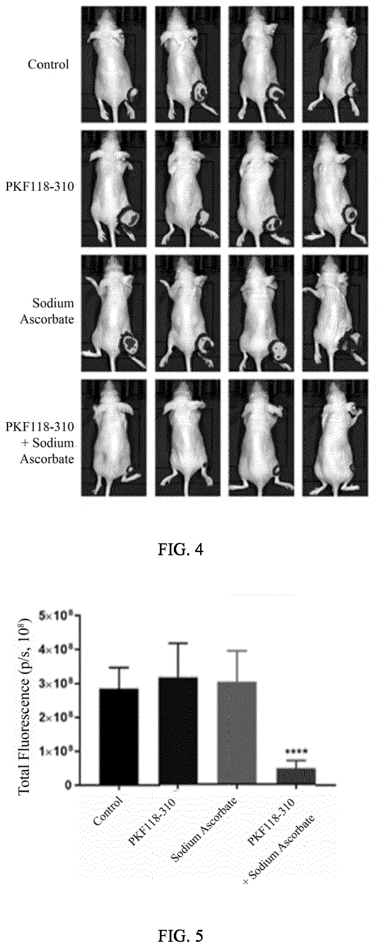

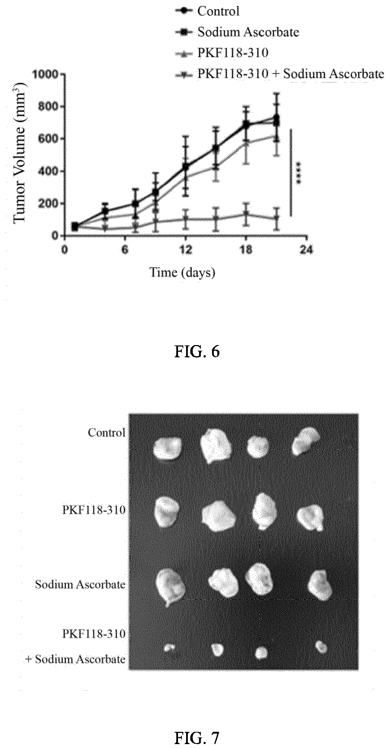

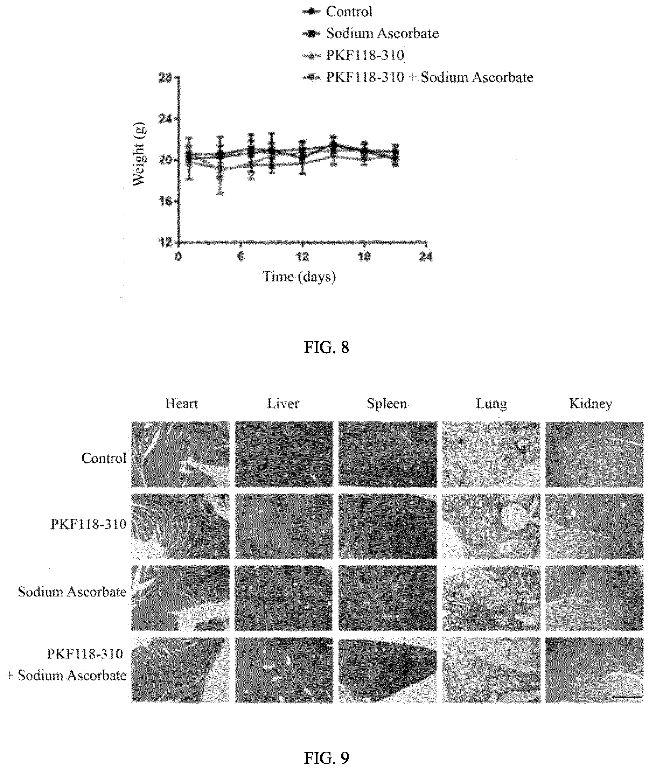

(1) Tumor preparation in nude mice. 6-8-week-old female nude mice (BALB/c-nu) (purchased from Vital River Laboratory Animal Technology Co., Ltd.) were selected. 143B cells were cultured *in vitro* to the log phase. The cells were collected and re-suspended in a PBS buffer solution. The collected cells were subcutaneously injected to the mammary glands of nude mice with 5 x 10⁶ 143B cells/nude mouse to establish a model of tumor *in situ.*
(2) After the tumor formation, the nude mice were divided to four test groups, including the blank control group, the PKF 118-310 group, the sodium ascorbate group, and the PKF 118-310 + sodium ascorbate group. The PKF 118-310 + sodium ascorbate group was subcutaneously injected with 0.7 mg/kg of PKF118-310 compound and intravenously injected with 1 g/kg of ascorbic acid every 3 days for a total of 6 times, and meanwhile the PKF118-310 group and the sodium ascorbate group were injected with equivalent amounts of PKF118-310 alone and ascorbic acid alone, respectively, while the blank control group was injected with an equivalent amount of normal saline. The day of the first injection was taken as Day 1, and then the body weight and the tumor size of the nude mice were examined and recorded: the tumor volume (mm³) = Length (L) x Width (W)² / 2. The tumor was subject to Luciferin fluorescence imaging. That is, a 1 mg/mL of aqueous potassium D-fluorescein solution was prepared, and each mouse was intraperitoneally injected with 100 µL. After 10 min of injection, the mice were subject to Luciferin fluorescence imaging with a small animal live imaging instrument.
   The results are shown in Figs. 4-8. Fig. 4 shows the Luciferin fluorescence imaging results of various groups at Day 21, Fig. 5 is a statistical graph of the Luciferin fluorescence imaging results, and both Fig. 4 and Fig. 5 indicate that the PKF118-310 + sodium ascorbate group significantly inhibits the tumor growth, as compared with the blank control group, the PKF118-310 group, and the sodium ascorbate group. Fig. 6 shows the statistical tumor volume with time, indicating that, as compared with the blank control group, the PKF118-310 group, and the sodium ascorbate group, the PKF 118-310 + sodium ascorbate group significantly inhibits the tumor growth (P<0.001). Fig. 7 is the comparison diagram of the tumor volumes in various groups on Day 21, wherein the tumor volume of the PKF 118-310 + sodium ascorbate group is substantially smaller than those of the blank control group, PKF118-310 group and sodium ascorbate group. Fig. 8 shows that, as compared with the control group, PKF 118-310 and sodium ascorbate, either alone or in combination, have no effect on the body weight of mice, indicating good safety.
(3) On Day 21, the mice were sacrificed. Organs such as heart, liver, spleen, lung, kidney, or the like were taken out, fixed with 4% paraformaldehyde, and subject to H&E staining test (accomplished by Beijing Soonbio Technology Co., Ltd)). The results are as shown in Fig. 9. As compared with the control group, neither the single-drug group nor the combination group causes damage to various organs of the mice, indicating good safety.

### Example 4. Inhibitory effect of PRI-724 and sodium ascorbate on osteosarcoma cells

The inhibitory effect of PRI-724 on the HOS human osteosarcoma cells was detected in accordance with the corresponding method of Example 1.

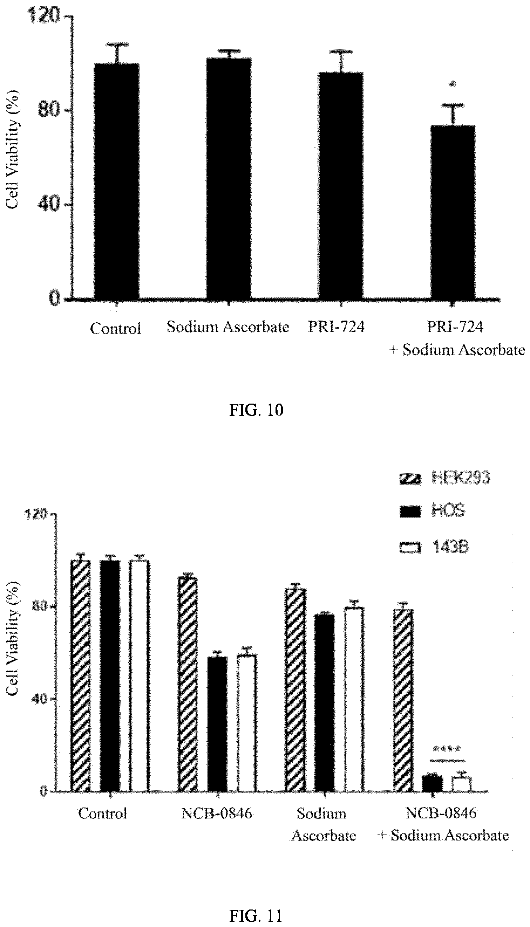

The results of combination: under the condition of treatment with a single-drug of 20 µM of PRI-724, the HOS cells have cell viability of 102%; under the condition of treatment with 0.5 mM of sodium ascorbate, the HOS cells have cell viability of 96%; and under the condition of treatment with 20 µM of PRI-724 in combination with 0.5 mM of sodium ascorbate, the HOS cells have cell viability of 74%. In accordance with the above-described CDI calculation equation, the CDI value is 0.41 for the HOS cells, indicating that they have a synergistic effect therebetween. At the same time, the inhibitory effect of PRI-724 in combination with ascorbic acid on the HOS cells is significantly better than that of the corresponding single-drug groups (P<0.05). The results are shown in Fig. 10.

### Example 5. Inhibitory effect of NCB-0846 and sodium ascorbate on osteosarcoma cells

The inhibitory effect of NCB-0846 on the HOS human osteosarcoma cells and the 143B HOS human osteosarcoma cells was detected in accordance with the corresponding method of Example 1.

The results of combination: under the condition of treatment with a single-drug of 20 µM of NCB-0846, the HOS cells have cell viability of 58%, the 143B cells have cell viability of 59%; under the condition of treatment with 0.5 mM of sodium ascorbate, the HOS cells have cell viability of 76%, the 143B cells have cell viability of 80%; and under the condition of treatment with 20 µM of NCB-0846 in combination with 0.5 mM of sodium ascorbate, the HOS cells have cell viability of 7%, the 143B cells have cell viability of 6%. In accordance with the above-described CDI calculation equation, the CDI value is 0.16 for the HOS cells, and 0.13 for the 143B cells, indicating that they have a synergistic effect therebetween. At the same time, the as compared with the HEK293 cells, the NCB-0846 in combination with ascorbic acid can specifically inhibit the cell viability of the 143B cells and the HOS cells, and have significantly better inhibitory effects on the 143B cells and the HOS cells than the corresponding single-drug groups (P<0.001). The results are shown in Fig. 11.

### Example 6. Inhibitory effect of Administration of siRNA against β -Catenin and sodium ascorbate on osteosarcoma cells

SiRNA is designed against the nucleotide sequence of β-catenin, and the siRNA codes are 484, 1202, and 1387, respectively. The sequences of sense strand and anti-sense strand of 484 are shown in SEQ ID NO. 1-2, respectively, and the sequences of sense strand and anti-sense strand of 1202 are shown in SEQ ID NO. 3-4, respectively, and the sequences of sense strand and anti-sense strand of 1387 are shown in SEQ ID NO. 5-6, respectively, wherein there are 2 T-bases at the 3' end of each strand. 143B human osteosarcoma cells were subcultured at 37°C, 5% CO₂ in an incubator with a RPMI-1640 medium containing 10% fetal bovine serum. The particular steps were as follows:
(1) Cells were plated in a 10 cm culture plate to grow a density of about 90%. The medium was removed, and the adherent cells were washed once with a PBS buffer. 1 mL of trypsin was added, and immediately discarded after mixing well. The cells were placed in the incubator and digested for about 5 min, and then about 10 ml of fresh medium was added. 10 µL was taken for counting.
(2) 10 µL of the well-mixed cells were taken and mixed into 10 µL of Trypan Blue solution, and counted.
(3) After counting, the cells were diluted to 1 x 10⁵ cells/ml. The diluted cells were mixed well, and plated into a 24-well plate with 500 µL per well, i.e., about 50,000 cells per well.
(4) Cells plated in the 24-well plate were cultured for 24 h. Then, the medium was discarded and replaced with 500 µL of serum-free medium.
(5) 20 pmol of siRNA was added into 50 µL of serum-free medium and mixed well.
(6) 1 µL of Lipofectamin 2000 transfection reagent was added into 50 µL of serum-free medium, mixed well, and stood at room temperature for 5 min.
(7) The siRNA diluted in Step (5) and the Lipofectamin 2000 reagent obtained in Step (6) were mixed, shaken well, and stood at room temperature for 20 min.
(8) 100 µL of the mixture obtained in Step (7) was added into the wells of the cell culture plate, cultured at 37°C for 4h, and then the medium was replaced with normal medium. And an ascorbic acid solution with a concentration of 0.5 mM into the ascorbic acid group and the combination group.
(9) After 24h of dosing, the medium was discarded. About 150 µL of trypsin was added and then discarded. The cells were digested for 5 min;
(10) After digestion, equivalent amounts of PBS were added into each well and mixed uniformly. Then, 10 µL /well of Trypan Blue solution was added and mixed uniformly. 10 µL of the well-mixed solution was taken and counted. In accordance with "Cell viability = Cell Count of Test Group / Cell Count of Control Group x 100%", the cell viability of various groups was calculated.

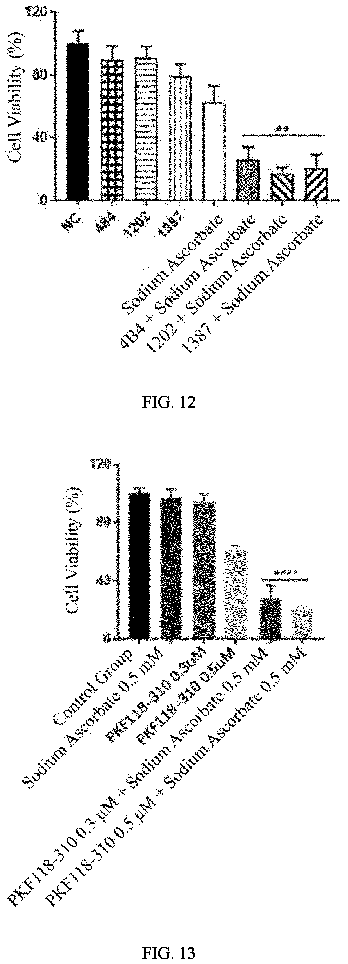

The results of combination: Under the condition of administration of the siRNA alone, the cell viability of the 143B cells is 90%, 91%, 80% in groups 484, 1202, and 1387, respectively; under the condition of treatment with 0.5 mM of sodium ascorbate, the cell viability of the 143B cells is 63%; and under the condition of treatment with the siRNA of groups 484, 1202, 1387 in combination with 0.5 mM of sodium ascorbate, the cell viability of the 143B cells is 26%, 17%, 20%, respectively. In accordance with the above-described CDI calculation equation, the CDI value for 143B cells is 0.46, 0.30, 0.40, respectively, indicating that they have synergistic effect therebetween. At the same time, the inhibitory effects on the 143B cells are all significantly better than those of the corresponding single-drug groups (P<0.01). The results are shown in Fig. 12.

### Example 7. Inhibitory effect of PKF118-310 and sodium ascorbate on ovarian cancer cells

The inhibitory effect of PKF118-310 on the SKOV3 human ovarian cancer cells and the 3AO human ovarian cancer cells was detected in accordance with the corresponding method of Example 1.

The results of combination: under the condition of single-drug treatment with 0.3 µM or 0.5 µM of PKF118-310, the SKOV3 cells have cell viability of 94%, 61%, respectively; under the condition of treatment with 0.5 mM of sodium ascorbate, the SKOV3 cells have cell viability of 97%; under the condition of treatment with 0.3 µM or 0.5 µM of PKF118-310 in combination with 0.5 mM of sodium ascorbate, the SKOV3 cells have cell viability of 28%, 20%, respectively. In accordance with the above-described CDI calculation equation, the CDI values for the SKOV3 cells are 0.31, 0.34, respectively indicating that they have synergistic effect therebetween. At the same time, the 0.3 µM or 0.5 µM of PKF118-310 in combination with ascorbic acid produces significantly better inhibitory effects on the SKOV3 cells than those of the corresponding single-drug groups (P<0.001 at the two different concentrations). The results of the SKOV3 cells are as shown in Fig. 13.

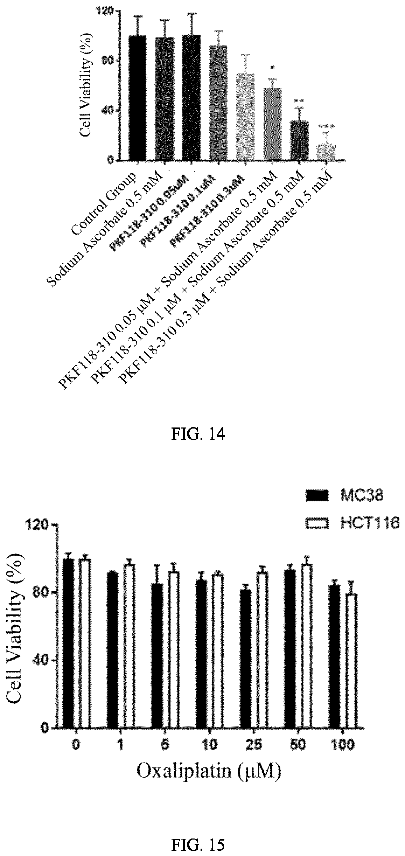

Under the condition of sing-drug treatment with 0.05 µM, 0.1 µM, or 0.3 µM of PKF118-310, the 3AO cells have cell viability of 101%, 92%; 69%, respectively; under the condition of treatment with 0.5 mM of sodium ascorbate, the 3AO cells have cell viability of 99%; and under the condition of treatment with 0.05 µM, 0.1 µM, or 0.3 µM of PKF118-310 in combination with 0.5 mM of sodium ascorbate, the 3AO cells have cell viability of 58%, 31%, 13%, respectively. In accordance with the above-described CDI calculation equation, the CDI value for the 3AO cells is 0.60,0.35,0.19, respectively, indicating that they have synergistic effect therebetween. At the same time, the PKF 118-310 in combination with ascorbic acid produce significantly better inhibitory effect on the cells that the corresponding single-drug groups (P<0.05 in the 0.05 µM combination group, P<0.01 in the 0.1 µM combination group, and P<0.001 in the 0.3 µM combination group). The results of 3AO are as shown in Fig. 14.

### Example 8. Inhibitory effect of PKF118-310 and sodium ascorbate on colon cancer cells

The inhibitory effect of PKF118-310 on the MC38 human colon cancer cells and the HCT116 human ovarian cancer cells was detected in accordance with the corresponding method of Example 1.

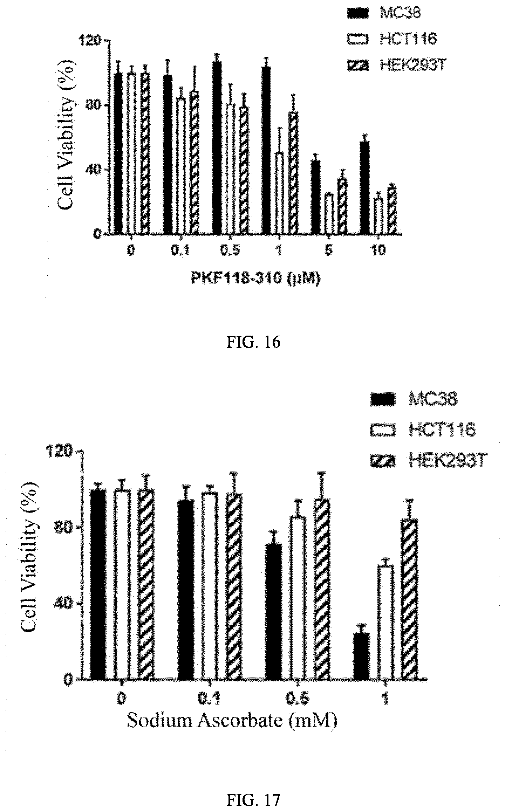

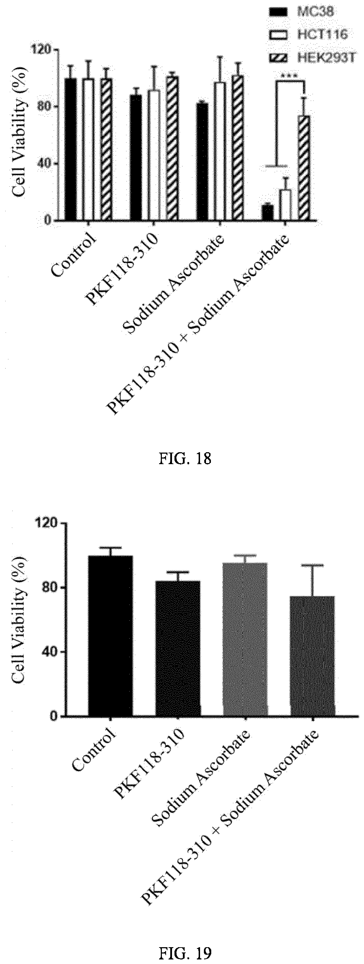

The results are shown in Figs. 15 -18. Fig. 15 shows that oxaliplatin does not have significant effect on MC38 human colon cancer cells and HCT116 human colon cancer cells, and the oxaliplatin with concentrations ranging from 0 to 100 µM does not have significant effect on the cell viability; and Fig. 16 and Fig. 17 shows an effect of PKF 118-310 or ascorbic acid with concentration gradients on the MC38 human colon cancer cells and the HCT116 human colon cancer cells. The results of combination: under the single-drug treatment with 0.3 µM of PKF 118-310, the MC38 cells have cell viability of 88%, and the HCT116 cells have cell viability of 92%; under the condition of treatment with 0.5 mM of sodium ascorbate, the MC38 cells have cell viability of 83%, and the HCT116 cells have cell viability of 98%; and under the condition of treatment with 0.3 µM of PKF118-310 in combination with 0.5 mM of sodium ascorbate, the MC38 cells have cell viability of 11%, and the HCT116 cells have cell viability of 22%, In accordance with the above-described CDI calculation equation, the CDI value is 0.15 for the MC38 cells, and 0.24 for the HCT116 cells, indicating that they have synergistic effect therebetween. At the same time, the as compared with the HEK293 cells, PKF118-310 in combination with ascorbic acid can specifically inhibit the activity of the HCT116 cells and the MC38 cells, and produce significantly better inhibitory effects on the HCT116 cells and the MC38 cells as compared with the corresponding single-drug groups. The results are shown in Fig. 18.

### Example 9. Inhibitory effect of PKF118-310 and sodium ascorbate on immune cells

Immune cells comprise T cells and Macrophages, wherein the T cells are cultured with a 1640 medium, and the macrophages are cultured with a DMEM medium. The particular procedures are follows:
(1) Cells were plated in a 10 cm culture plate to grow a density of about 90%. The medium was removed, and the adherent cells were washed once with a PBS buffer. 1 mL of trypsin was added, and immediately discarded after mixing well. The cells were placed in the incubator and digested for about 5 min, and then about 10 ml of fresh medium was added. 10 µL was taken for counting.
(2) 10 µL of the well-mixed cells were taken and mixed into 10 µL of Trypan Blue solution, and counted.
(3) After counting, the cells were diluted to 1 x 10⁵ cells/ml. The diluted cells were mixed well, and plated into a 24-well plate with 500 µL per well, i.e., about 50,000 cells per well.
(4) The cells plated in the 24-well plate were cultured for about 24h, and then drugs were added. Various test groups comprise 5 groups as follows: the single-drug group of PKF 118-310 (with a concentration of 0.1 µM), the group of ascorbic acid (with a concentration of 0.5 mM), the group of PKF118-310 (with a concentration of 0.1 µM) in combination with ascorbic acid (with a concentration of 0.5 mM); the control group that is a cell group to which only solvent DMSO is added; and the blank group that is a group to which no cell is added and only solvent (DMSO) is added.
(5) After 24h of dosing, the medium was discarded. About 150 µL of trypsin was added and then discarded. The cells were digested for 5 min;
(6) After digestion, equivalent amounts of PBS were added into each well and mixed uniformly. Then, 10 µL /well of Trypan Blue solution was added and mixed uniformly. 10 µL of the well-mixed solution was taken and counted. In accordance with "Cell viability = Cell Count of Test Group / Cell Count of Control Group x 100%", the cell viability of various groups was calculated.

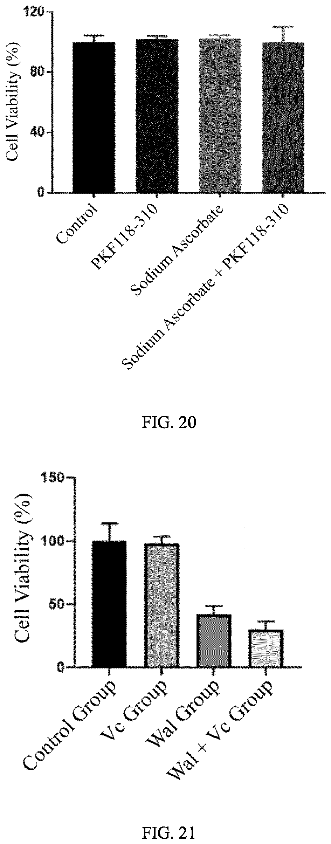

The results are shown in Figs. 19-20. Fig. 19 shows an effect of PKF 118-310 and sodium ascorbate alone or in combination on the T cells, and Fig. 20 shows PKF118-310 and sodium ascorbate alone or in combination on the macrophages, both of which indicate that the drug combination does not have effect on the immune cells, specifically kill tumor cells, and have safety.

### Example 10. Inhibitory effect of PKF118-310 derivatives and sodium ascorbate on osteosarcoma cells

The inhibitory effect of PKF118-310 derivatives, e.g., comprising, but being not limited to, the compound C-1 of the present application, the compound C-2 of the present application, Fervenulin, 3-Methyltoxoflavin, Walrycin B, BI-D1870, and Lumazine on the osteosarcoma cells was detected in accordance with the method of Example 11.

The synthetic route of the compound C-1 of the present application can be as follows: 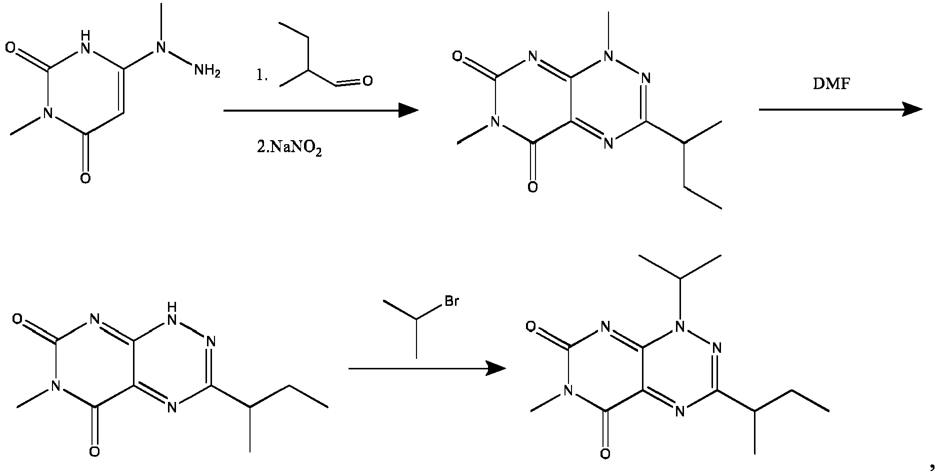 wherein, Step 1: 5 g of reactant, 45 mL of AcOH, and 15 mL of water were mixed, cooled and stirred; 5 g of 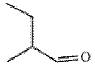 was added; the reaction mixture was stirred for reaction for 1 hr, and 4.05 g of NaNO₂ was added; and the reaction mixture reacted at low temperature for 40 min, and then at room temperature for additional 14 hr; and then EtOH was added for concentration.

Step 2: 24 g of reactant was dissolved in 30 mL of DMF, heated to 140°C, stirred for reaction for 4 hr, concentrated to remove the DMF, and underwent column separation for purification.

Step 3: 0.2 g of reactant, 0.31 g of isopropyl bromide, 0.24 g of potassium carbonate, and 4 mL of ethylene oxide were mixed, heated to 120°C, and reacted for 3.5 hr, and cooled to room temperature to give the compound C-1 of the present application.

The synthetic route of the compound C-2 of the present application can be as follows: 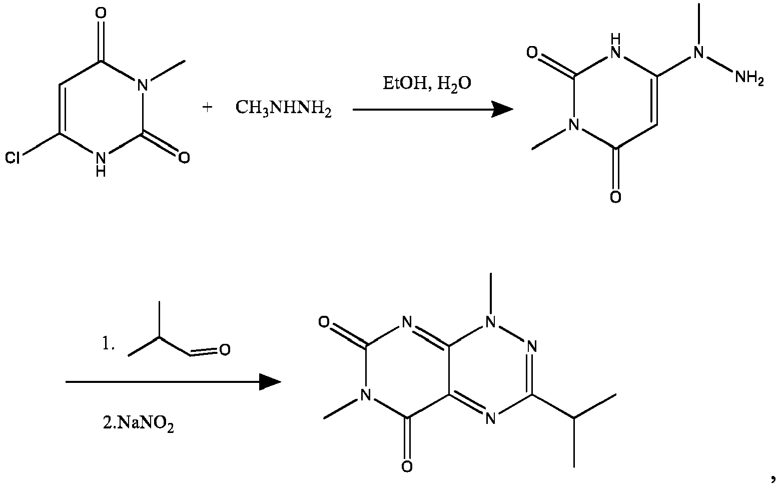 wherein, Step 1: 10 g of reactant was mixed with 3 eq of CH₃NHNH₂, heated at 90°C in an oil bath and stirred for reaction for 1 hr, concentrated to dry, and purified by column separation.

Step 2: 0.5 g of reactant, 4.5 mL of AcOH, and 1.5 mL of water were mixed, cooled and stirred. At 0-5°C, 0.85 g of 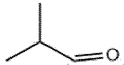 was added dropwise, followed by supplement of 4.5 mL ofAcOH and 15 mL of water. The mixture stood for 1 hr, and then 0.5 mL of NaNOz (5.88 mmol) was added dropwise. After 0.5 hr, the mixture was stirred for reaction at room temperature to give the compound C-2 of the present application.

For example, as for HOS osteosarcoma cells, the control group involves adding the solvent DMSO alone, the Vc group involves adding 0.5 mM of ascorbic acid, the Wal group involves adding 10 µM of Walrycin B, and the Wal + Vc group involves adding 10 µM of Walrycin B and 0.5 mM of ascorbic acid.

Fig. 21 shows that, as compared with the cell viability of the control group, the HOS cells have cell viability of 98% under the condition of treatment with 0.5 mM of ascorbic acid; 42% under the condition of treatment with 10 µM of Walrycin B; and 30% under the condition of treatment with 10 µM of Walrycin B and 0.5 mM of ascorbic acid. The coefficient of drug interaction for HOS cells are obtained in accordance with the CDI calculation equation in Example 1, and the CDI value for the combination of Walrycin B with ascorbic acid is 0.73, indicating that they have synergistic effect therebetween. At the same time, the Walrycin B in combination with ascorbic acid has a better inhibitor effect on the HOS cells than the corresponding single-drug groups.

For example, as for HOS osteosarcoma cells, the control group involves adding the solvent DMSO alone, the Vc group involves adding 0.5 mM of ascorbic acid, the 3-Met group involves adding 3-Methyltoxoflavin with a concentration of 10 µM, and the 3-Met + Vc group involves adding 10 µM of 3-Methyltoxoflavin and 0.5 mM of ascorbic acid.

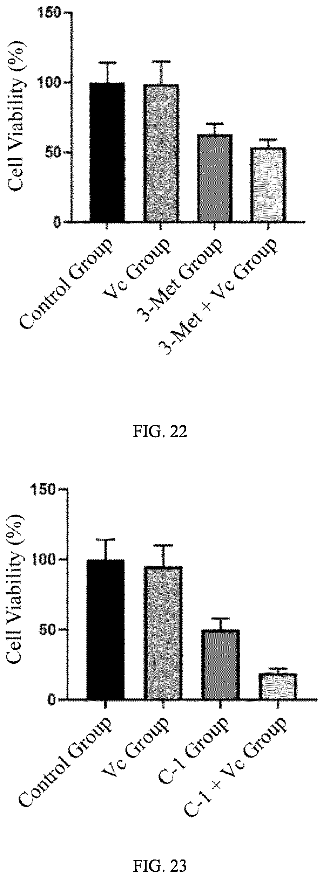

Fig. 22 shows that, as compared with the cell viability of the control group, the HOS cells have cell viability of 99% under the condition of treatment with 0.5 mM of ascorbic acid; 63% under the condition of treatment with 10 µM of 3-Methyltoxoflavin; and 54% under the condition of treatment with 10 µM of 3-Methyltoxoflavin and 0.5 mM of ascorbic acid. The coefficient of drug interaction for HOS cells are obtained in accordance with the CDI calculation equation in Example 1, and the CDI value for the combination of 3-Methyltoxoflavin with ascorbic acid is 0.86, indicating that they have synergistic effect therebetween. At the same time, the 3-Methyltoxoflavin in combination with ascorbic acid has a better inhibitor effect on the HOS cells than the corresponding single-drug groups.

For example, as for 143B osteosarcoma cells, the control group involves adding the solvent DMSO alone, the Vc group involves adding 0.5 mM of ascorbic acid, the C-1 group involves adding 5 µM of the compound C-1 of the present application, and the C-1 + Vc group involves adding 5 µM of the compound C-1 of the present application and 0.5 mM of ascorbic acid. The compound C-1 of the present application has a structure of:

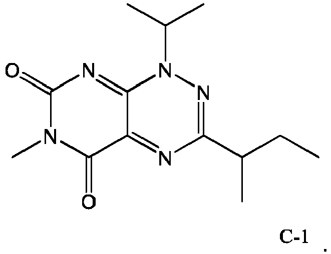

Fig. 23 shows that, as compared with the cell viability of the control group, the 143B osteosarcoma cells have cell viability of 96% under the condition of treatment with 0.5 mM of ascorbic acid; 50% under the condition of treatment with 5 µM of the compound C-1 of the present application; and 19% under the condition of treatment with 5 µM of the compound C-1 of the present application and 0.5 mM of ascorbic acid. The coefficient of drug interaction for 143B osteosarcoma cells obtained in accordance with the CDI calculation equation in Example 1, and the CDI value for the combination of the compound C-1 of the present application with ascorbic acid is 0.40, indicating that they have significant synergistic effect therebetween. At the same time, the compound C-1 of the present application in combination with ascorbic acid has a better inhibitor effect on the 143B osteosarcoma cells than the corresponding single-drug groups.

For example, as for 143B osteosarcoma cells, the control group involves adding the solvent DMSO alone, the Vc group involves adding 0.5 mM of ascorbic acid, the C-2 group involves adding 1 µM of the compound C-2 of the present application, and the C-2 + Vc group involves adding 1 µM of the compound C-2 of the present application and 0.5 mM of ascorbic acid. The compound C-2 of the present application has a structure of:

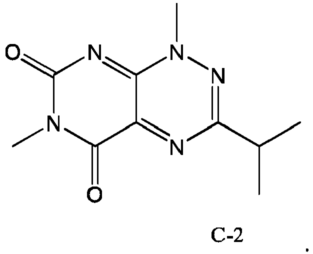

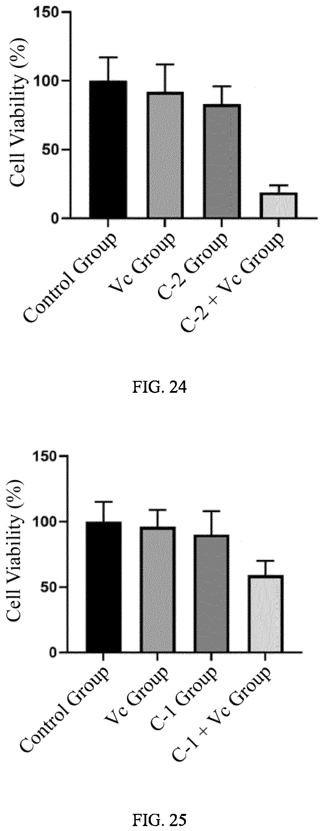

Fig. 24 shows that, as compared with the cell viability of the control group, the 143B osteosarcoma cells have cell viability of 92% under the condition of treatment with 0.5 mM of ascorbic acid; 83% under the condition of treatment with 1 µM of the compound C-2 of the present application; and 28% under the condition of treatment with 1 µM of the compound C-2 of the present application and 0.5 mM of ascorbic acid. The coefficient of drug interaction for 143B osteosarcoma cells obtained in accordance with the CDI calculation equation in Example 1, and the CDI value for the combination of the compound C-2 of the present application with ascorbic acid is 0.37, indicating that they have significant synergistic effect therebetween. At the same time, the compound C-2 of the present application in combination with ascorbic acid has a better inhibitor effect on the 143B osteosarcoma cells than the corresponding single-drug groups.

The results show that the respective combination of the aforesaid PKF118-310 derivatives with sodium ascorbate has significantly better inhibitory effect on osteosarcoma cells than the corresponding single-drug groups, and they have synergistic effect therebetween.

### Example 11. Inhibitory effect of PKF118-310 derivatives and sodium ascorbate on ovarian cancer cells

The inhibitory effect of PKF118-310 derivatives, e.g., comprising, but being not limited to, the compound C-1 of the present application, the compound C-2 of the present application, Fervenulin, 3-Methyltoxoflavin, Walrycin B, BI-D1870 Lumazine on the ovarian cancer cells was detected in accordance with the corresponding method of Example 1. The results show that the respective combination of the aforesaid drugs with sodium ascorbate has significantly better inhibitory effect on ovarian cancer cells than the corresponding single-drug groups, and they have synergistic effect therebetween.

### Example 12. Inhibitory effect of PKF118-310 derivatives and sodium ascorbate on colon cancer cells

The inhibitory effect of PKF118-310 derivatives, e.g., comprising, but being not limited to, the compound C-1 of the present application, the compound C-2 of the present application, Fervenulin, 3-Methyltoxoflavin, Walrycin B, BI-D1870 Lumazine on the colon cancer cells was detected in accordance with the corresponding method of Example 1.

For example, as for MC38 colon cancer cells, the control group involves adding the solvent DMSO alone, the Vc group involves adding 0.5 mM of ascorbic acid, the C-1 group involves adding 5 µM of the compound C-1 of the present application, and the C-1 + Vc group involves adding 5 µM of the compound C-1 of the present application and 0.5 mM of ascorbic acid. The compound C-1 of the present application has a structure of:

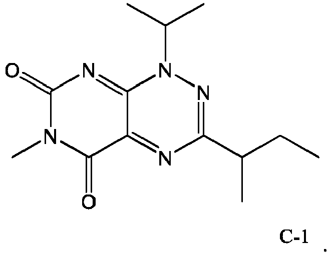

Fig. 25 shows that, as compared with the cell viability of the control group, the MC38 cells have cell viability of 96% under the condition of treatment with 0.5 mM of ascorbic acid; 90% under the condition of treatment with 5 µM of the compound C-1 of the present application; and 59% under the condition of treatment with 5 µM of the compound C-1 of the present application and 0.5 mM of ascorbic acid. The coefficient of drug interaction for MC38 cells obtained in accordance with the CDI calculation equation in Example 1, and the CDI value for the combination of the compound C-1 of the present application with ascorbic acid is 0.68, indicating that they have significant synergistic effect therebetween. At the same time, the compound C-1 of the present application in combination with ascorbic acid has a better inhibitor effect on the MC38 cells than the corresponding single-drug groups.

For example, as for MC38 colon cancer cells, the control group involves adding the solvent DMSO alone, the Vc group involves adding 0.5 mM of ascorbic acid, the C-2 group involves adding 1 µM of the compound C-2 of the present application, and the C-2 + Vc group involves adding 1 µM of the compound C-2 of the present application and 0.5 mM of ascorbic acid. The compound C-2 of the present application has a structure of:

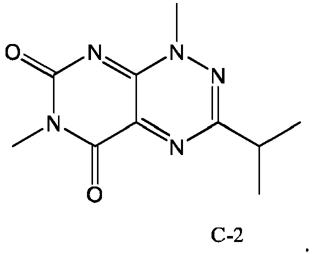

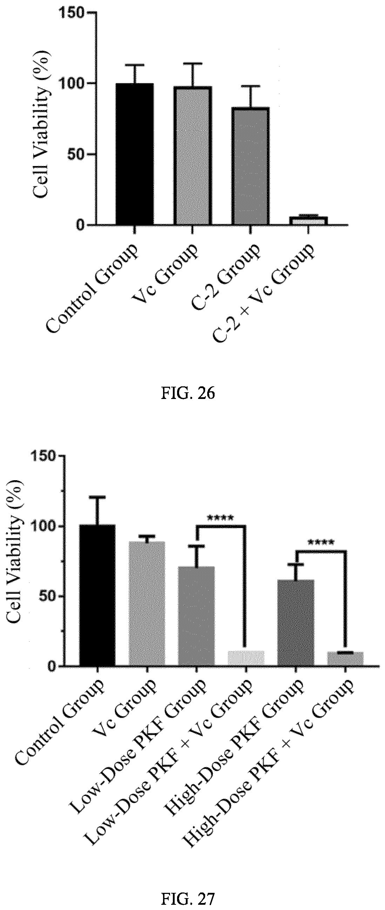

Fig. 26 shows that, as compared with the cell viability of the control group, the MC38 cells have cell viability of 98% under the condition of treatment with 0.5 mM of ascorbic acid; 83% under the condition of treatment with 1 µM of the compound C-2 of the present application; and 6% under the condition of treatment with 1 µM of the compound C-2 of the present application and 0.5 mM of ascorbic acid. The coefficient of drug interaction for MC38 cells obtained in accordance with the CDI calculation equation in Example 1, and the CDI value for the combination of the compound C-2 of the present application with ascorbic acid is 0.07, indicating that they have significant synergistic effect therebetween. At the same time, the compound C-2 of the present application in combination with ascorbic acid has a better inhibitor effect on the MC38 cells than the corresponding single-drug groups.

The results show that the respective combination of the aforesaid PKF118-310 derivatives with sodium ascorbate has significantly better inhibitory effect on colon cancer cells than the corresponding single-drug groups, and they have synergistic effect therebetween.

### Example 13. Inhibitory effect of PKF118-310 and derivatives thereof and sodium ascorbate on lymphoma cells

The inhibitory effect of PKF118-310 and PKF118-310 derivatives, e.g., comprising, but being not limited to, the compound C-1 of the present application, the compound C-2 of the present application, Fervenulin, 3-Methyltoxoflavin, Walrycin B, BI-D1870, and Lumazine on the lymphoma cells was detected in accordance with the method of Example 1.

For example, as for the DB lymphoma cells, the control group involves adding the solvent DMSO alone, the Vc group involves adding 2 mM of ascorbic acid, the low-dose PKF group involves adding 2 µM of PKF118-310, the low-dose PKF + Vc group involves adding 2 µM of PKF118-310 and 2 mM of ascorbic acid, the high-dose PKF group involves adding 5 µM of PKF118-310, and the high-dose PKF + the Vc group involves adding 5 µM of PKF118-310 and 2 mM of ascorbic acid.

Fig. 27 shows that, as compared with the cell viability of the control group, the DB cells have cell viability of 91% under the condition of treatment with 2 mM of ascorbic acid; 72% under the condition of treatment with 2 µM of PKF118-310; 12% under the condition of treatment with 2 µM of PKF118-310 and 2 mM of ascorbic acid; 67% under the condition of treatment with 5 µM of PKF118-310; and 11% under the condition of treatment of 5 µM ofPKF118-310 and 2 mM of ascorbic acid. The coefficient of drug interaction for DB cells obtained in accordance with the CDI calculation equation in Example 1, the CDI value for the combination of the low-dose PKF118-310 with ascorbic acid is 0.17, and the CDI value for the combination of the high-dose PKF118-310 with ascorbic acid is 0.18, indicating that they have significant synergistic effect therebetween. At the same time, the inhibitory effect of the low- and the high-dose PKF118-310s in combination with ascorbic acid on the DB cells is significantly better than that of the corresponding single-drug groups (P<0.0001).

The results show that the respective combination of the aforesaid PKF118-310 and derivatives thereof with sodium ascorbate has significantly better inhibitory effect on lymphoma cells than the corresponding single-drug groups, and they have synergistic effect therebetween.

### Example 14. Inhibitory effect of NCB-0846 or PRI-724 and sodium ascorbate on ovarian cancer cells/colon cancer cells/lymphoma cells

The inhibitory effect of NCB-0846 or PRI-724 on the ovarian cancer cells/colon cancer cells/lymphoma cells was detected in accordance with the corresponding method of Example 1. The results show that the respective combination of the aforesaid drugs with sodium ascorbate has significantly better inhibitory effect on ovarian cancer cells/colon cancer cells/lymphoma cells than the corresponding single-drug groups, and they have synergistic effect therebetween.

The aforesaid detailed description is provided in an illustrative and exemplary manner, and are not intended to limit the scope of the appended claims. Various modifications of embodiments currently listed in the present application are apparent for persons skilled in the art, and encompassed within the scope of the appended claims and their equivalences.

## Claims

1. A pharmaceutical combination, comprising:
a) a prophylactically and/or therapeutically effective amount of ascorbic acid or a derivative thereof; and
b) a prophylactically and/or therapeutically effective amount of a substance affecting the formation of a TCF/β-catenin complex.

2. The pharmaceutical combination according to claim 1, wherein the substance affecting the formation of a TCF/β-catenin complex comprises a TCF/LEF inhibitor.

3. The pharmaceutical combination according to claim 2, wherein the TCF/LEF inhibitor comprises NCB-0846.

4. The pharmaceutical combination according to any one of claims 1-3, wherein the substance affecting the formation of a TCF/β-catenin complex comprises a substance affecting the formation of a β-catenin/CBP complex.

5. The pharmaceutical combination according to claim 4, wherein the substance affecting the formation of a β-catenin/CBP complex comprises PRI-724.

6. The pharmaceutical combination according to any one of claims 1-5, wherein the substance affecting the formation of a TCF/β-catenin complex comprises a β-catenin inhibitor.

7. The pharmaceutical combination according to claim 6, wherein the β-catenin inhibitor comprises a substance down-regulating the protein expression level and/or activity of the β-catenin.

8. The pharmaceutical combination according to claim 7, wherein the substance down-regulating the protein expression level and/or activity of the β-catenin comprises siRNA and/or shRNA.

9. The pharmaceutical combination according to claim 8, wherein the siRNA comprises a nucleotide sequence as shown in any one of SEQ ID NOS. 1-6.

10. The pharmaceutical combination according to any one of claims 1-9, wherein the substance affecting the formation of a TCF/β-catenin complex comprises a compound having a structure of Formula I, II or III or a pharmaceutically acceptable salt or hydrate thereof: 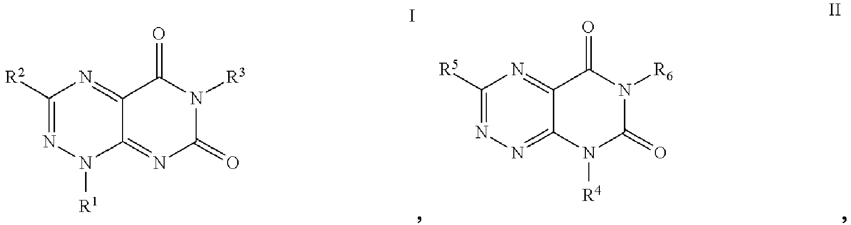 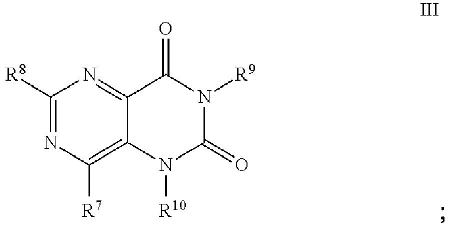
wherein, R¹ and R³ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R² is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl;
R⁴ and R⁶ are each independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R⁵ is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl, optionally substituted heteroaryl, optionally substituted C₁₋₂₀ alkylamino, optionally substituted C₁₋₂₀ alkoxy and optionally substituted C₁₋₂₀ alkylformamido;
R⁷, R⁸, R⁹, and R¹⁰ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl.

11. The pharmaceutical combination according to claim 10, wherein the R² is C₄₋₆ alkyl.

12. The pharmaceutical combination according to any one of claims 10-11, wherein the R² is C₄ alkyl.

13. The pharmaceutical combination according to any one of claims 10-12, wherein the R² is isobutyl.

14. The pharmaceutical combination according to any one of claims 10-13, wherein the R¹ is selected from the group consisting of hydrogen and C₁₋₆ alkyl.

15. The pharmaceutical combination according to any one of claims 10-14, wherein the R¹ is C₃ alkyl.

16. The pharmaceutical combination according to any one of claims 10-15, wherein the R¹ is isopropyl.

17. The pharmaceutical combination according to any one of claims 10-16, wherein the R³ is selected from the group consisting of hydrogen and C₁₋₆ alkyl.

18. The pharmaceutical combination according to any one of claims 10-17, wherein the R³ is methyl.

19. The pharmaceutical combination according to claim 10, wherein the compound having a structure of Formula I comprises PKF118-310 or a derivative thereof.

20. The pharmaceutical combination according to claim 19, wherein the derivative of PKF118-310 comprises 3-Methyltoxoflavin and/or Walrycin B.

21. The pharmaceutical combination according to claim 10, wherein the compound having a structure of Formula II comprises Fervenulin.

22. The pharmaceutical combination according to any one of claims 1-21, wherein the substance affecting the formation of a TCF/β-catenin complex comprises BI-D1870 and/or Lumazine.

23. The pharmaceutical combination according to any one of claims 1-22, wherein the derivative of ascorbic acid comprises a pharmaceutically acceptable ascorbate.

24. The pharmaceutical combination according to claim 23, wherein the pharmaceutically acceptable ascorbate comprises sodium ascorbate.

25. The pharmaceutical combination according to any one of claims 1-24, wherein the ratio of the effective amount of the substance affecting the formation of a TCF/β-catenin complex to the effective amount of the ascorbic acid or a derivative thereof is about 1% to about 10%.

26. The pharmaceutical combination according to any one of claims 1-25, wherein the mass ratio of the substance affecting the formation of a TCF/β-catenin complex to the ascorbic acid or a derivative thereof is about 90% to about 99%.

27. The pharmaceutical combination according to any one of claims 1-26, wherein the substance affecting the formation of a TCF/β-catenin complex and the ascorbic acid or a derivative thereof are each present in different containers.

28. The pharmaceutical combination according to any one of claims 1-27, wherein the pharmaceutical combination comprises a first preparation and a second preparation, wherein the first preparation comprises the ascorbic acid or a derivative thereof and a pharmaceutically acceptable first carrier, and the second preparation comprises the substance affecting the formation of a TCF/β-catenin complex and a pharmaceutically acceptable second carrier.

29. The pharmaceutical combination according to any one of claims 1-28, which comprises a pharmaceutical composition comprising the substance affecting the formation of a TCF/β-catenin complex and the ascorbic acid or a derivative thereof.

30. The pharmaceutical combination according to claim 29, wherein the substance affecting the formation of a TCF/β-catenin complex has a content of about 5% to 20% (w/w) in the pharmaceutical composition.

31. The pharmaceutical combination according to any one of claims 29-30, wherein the ascorbic acid or a derivative thereof has a content of about 80% to 95% (w/w) in the pharmaceutical composition.

32. A kit comprising the pharmaceutical combination according to any one of claims 1-31.

33. Use of a combination of ascorbic acid or a derivative thereof with a substance affecting the formation of a TCF/β-catenin complex in the manufacture of a drug for preventing and/or treating tumors.

34. The use according to claim 33, wherein the substance affecting the formation of a TCF/β-catenin complex comprises TCF/LEF inhibitor.

35. The use according to claim 34, wherein the TCF/LEF inhibitor comprises NCB-0846.

36. The use according to any one of claims 33-35, wherein the substance affecting the formation of a TCF/β-catenin complex comprises a substance affecting the formation of a β-catenin/CBP complex.

37. The use according to claim 36, wherein the substance affecting the formation of a β-catenin/CBP complex comprises PRI-724.

38. The use according to any one of claims 33-37, wherein the substance affecting the formation of a TCF/β-catenin complex comprises a β-catenin inhibitor.

39. The use according to claim 38, wherein the β-catenin inhibitor comprises a substance down-regulating the protein expression level and/or activity of the β-catenin.

40. The use according to claim 39, wherein the substance down-regulating the protein expression level and/or activity of the β-catenin comprises siRNA and/or shRNA.

41. The use according to claim 40, wherein the siRNA comprises a nucleotide sequence as shown in any one of SEQ ID NOS. 1-6.

42. The use according to any one of claims 33-41, wherein the substance affecting the formation of a TCF/β-catenin complex comprises a compound having a structure of Formula I, II or III or a pharmaceutically acceptable salt or hydrate thereof: 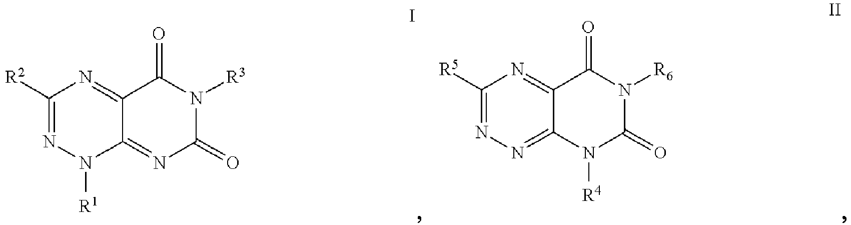 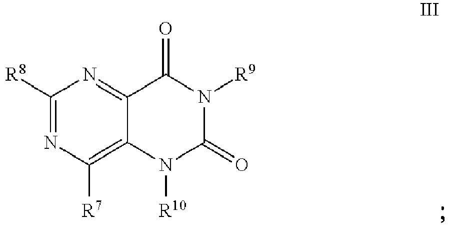
wherein, R¹ and R³ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R² is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl;
R⁴ and R⁶ are each independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R⁵ is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl, optionally substituted heteroaryl, optionally substituted C₁₋₂₀ alkylamino, optionally substituted C₁₋₂₀ alkoxy and optionally substituted C₁₋₂₀ alkylformamido;
R⁷, R⁸, R⁹, and R¹⁰ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl.

43. The use according to claim 42, wherein the R² is C₄₋₆ alkyl.

44. The use according to any one of claims 42-43, wherein the R² is C₄ alkyl.

45. The use according to any one of claims 42-44, wherein the R² is isobutyl.

46. The use according to any one of claims 42-45, wherein the R¹ is selected from the group consisting of hydrogen and C₁₋₆ alkyl.

47. The use according to any one of claims 42-46, wherein the R¹ is C₃ alkyl.

48. The use according to any one of claims 42-47, wherein the R¹ is isopropyl.

49. The use according to any one of claims 42-48, wherein the R³ is selected from the group consisting of hydrogen and C₁₋₆ alkyl.

50. The use according to any one of claims 42-49, wherein the R³ is methyl.

51. The use according to claim 42, wherein the compound having a structure of Formula I comprises PKF118-310 or a derivative thereof.

52. The use according to claim 51, wherein the derivative of PKF118-310 comprises 3-Methyltoxoflavin and/or Walrycin B.

53. The use according to claim 42, wherein the compound having a structure of Formula II comprises Fervenulin.

54. The use according to any one of claims 33-53, wherein the substance affecting the formation of a TCF/β-catenin complex comprises BI-D1870 and/or Lumazine.

55. The use according to claim 33-54, wherein the tumors comprise solid tumors or non-solid tumors.

56. The use according to any one of claims 33-55, wherein the tumors comprise osteosarcoma, colon cancer, ovarian cancer, and lymphoma.

57. The use according to any one of claims 33-56, wherein the derivative of ascorbic acid comprises a pharmaceutically acceptable ascorbate.

58. The use according to claim 57, wherein the pharmaceutically acceptable ascorbate comprises sodium ascorbate.

59. The use according to any one of claims 33-58, wherein the substance affecting the formation of a TCF/β-catenin complex and the ascorbic acid or a derivative thereof are formulated to be simultaneously administered to a subject.

60. The use according to any one of claims 33-58, wherein the substance affecting the formation of a TCF/β-catenin complex and the ascorbic acid or a derivative thereof are formulated to be separated administered to a subject.

61. The use according to any one of claims 33-60, wherein the drug is formulated so that the substance affecting the formation of a TCF/β-catenin complex is administered at a dose of about 0.05 mg/kg to about 40 mg/kg.

62. The use according to any one of claims 33-61, wherein the drug is formulated so that the ascorbic acid or a derivative thereof are administered at a dose of about 0.05 g/kg to about 2.5 g/kg.

63. The use according to any one of claims 33-62, wherein the drug is formulated so that the substance affecting the formation of a TCF/β-catenin complex and the ascorbic acid or a derivative thereof are administered at a mass ratio of about 1:30 to about 1:5000.

64. The use according to any one of claims 33-63, wherein the drug is formulated so that the substance affecting the formation of a TCF/β-catenin complex and the ascorbic acid or a derivative thereof are administered at an effective amount ratio of about 1:30 to about 1:5000.

65. A method for preventing and/or treating tumors, comprising administering to a subject in need thereof:
a) ascorbic acid or a derivative thereof; and
b) a substance affecting the formation of a TCF/β-catenin complex.

66. The method according to claim 65, wherein the substance affecting the formation of a TCF/β-catenin complex comprises a TCF/LEF inhibitor.

67. The method according to claim 66, wherein the TCF/LEF inhibitor comprises NCB-0846.

68. The method according to any one of claims 65-67, wherein the substance affecting the formation of a TCF/β-catenin complex comprises a substance affecting the formation of a β-catenin/CBP complex.

69. The method according to claim 68, wherein the substance affecting the formation of a β-catenin/CBP complex comprises PRI-724.

70. The method according to any one of claims 65-69, wherein the substance affecting the formation of a TCF/β-catenin complex comprises a β-catenin inhibitor.

71. The method according to claim 70, wherein the β-catenin inhibitor comprises a substance down-regulating the protein expression level and/or activity of the β-catenin.

72. The method according to claim 71, wherein the substance down-regulating the protein expression level and/or activity of the β-catenin comprises siRNA and/or shRNA.

73. The method according to claim 72, wherein the siRNA comprises a nucleotide sequence as shown in any one of SEQ ID NOS. 1-6.

74. The method according to any one of claims 65-73, wherein the substance affecting the formation of a TCF/β-catenin complex comprises a compound having a structure of Formula I, II or III or a pharmaceutically acceptable salt or hydrate thereof: 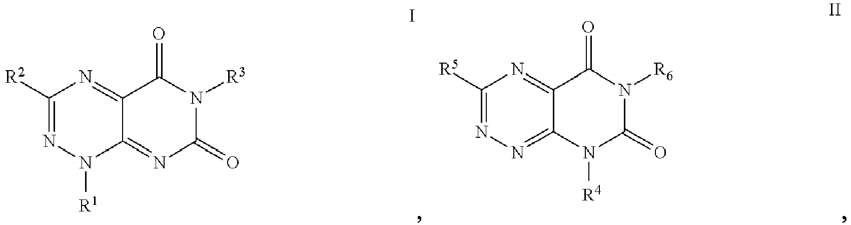 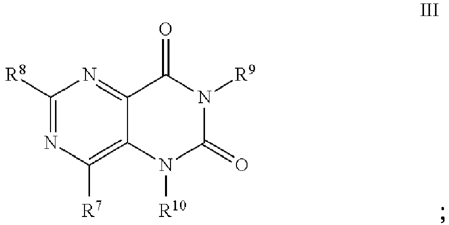
wherein, R¹ and R³ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R² is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl;
R⁴ and R⁶ are each independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R⁵ is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl, optionally substituted heteroaryl, optionally substituted C₁₋₂₀ alkylamino, optionally substituted C₁₋₂₀ alkoxy and optionally substituted C₁₋₂₀ alkylformamido;
R⁷, R⁸, R⁹, and R¹⁰ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl.

75. The method according to claim 74, wherein the R² is C₄₋₆ alkyl.

76. The method according to any one of claims 74-75, wherein the R² is C₄ alkyl.

77. The method according to any one of claims 74-76, wherein the R² is isobutyl.

78. The method according to any one of claims 74-77, wherein the R¹ is selected from the group consisting of hydrogen and C₁₋₆ alkyl.

79. The method according to any one of claims 74-78, wherein the R¹ is C₃ alkyl.

80. The method according to any one of claims 74-79, wherein the R¹ is isobutyl.

81. The method according to any one of claims 74-80, wherein the R³ is selected from the group consisting of hydrogen and C₁₋₆ alkyl.

82. The method according to any one of claims 74-81, wherein the R³ is methyl.

83. The method according to claim 74, wherein the compound having a structure of Formula I comprises PKF118-310 or a derivative thereof.

84. The method according to claim 83, wherein the derivative of PKF118-310 comprises 3-Methyltoxoflavin and/or Walrycin B.

85. The method according to claim 74, wherein the compound having a structure of Formula II comprises Fervenulin.

86. The method according to any one of claims 65-85, wherein the substance affecting the formation of a TCF/β-catenin complex comprises BI-D1870 and/or Lumazine.

87. The method according to claim 65-86, wherein the tumors comprise solid tumors or non-solid tumors.

88. The method according to any one of claims 65-87, wherein the tumors comprise osteosarcoma, colon cancer, ovarian cancer, and lymphoma.

89. The method according to any one of claims 65-88, wherein the derivative of ascorbic acid comprises a pharmaceutically acceptable ascorbate.

90. The method according to claim 89, wherein the pharmaceutically acceptable ascorbate comprises sodium ascorbate.

91. The method according to any one of claims 65-90, wherein the substance affecting the formation of a TCF/β-catenin complex and the ascorbic acid or a derivative thereof are formulated to be simultaneously administered to a subject.

92. The method according to any one of claims 65-90, wherein the substance affecting the formation of a TCF/β-catenin complex and the ascorbic acid or a derivative thereof are formulated to be separated administered to a subject.

93. The method according to any one of claims 65-92, wherein the drug is formulated so that the substance affecting the formation of a TCF/β-catenin complex is administered at a dose of about 0.05 mg/kg to about 40 mg/kg.

94. The method according to any one of claims 65-93, wherein the drug is formulated so that the ascorbic acid or a derivative thereof are administered at a dose of about 0.05 g/kg to about 2.5 g/kg.

95. The method according to any one of claims 65-94, wherein the drug is formulated so that the substance affecting the formation of a TCF/β-catenin complex and the ascorbic acid or a derivative thereof are administered at a mass ratio of about 1:30 to about 1:5000.

96. The method according to any one of claims 65-95, wherein the drug is formulated so that the substance affecting the formation of a TCF/β-catenin complex and the ascorbic acid or a derivative thereof are administered at an effective amount ratio of about 1:30 to about 1:5000.

97. A method for monitoring a response to a drug in a subject, comprising:
a) administering to the subject ascorbic acid or a derivative thereof and a substance affecting the formation of a TCF/β-catenin complex; and
b) detecting a change in one or more of the level and/or activity of blood alkaline phosphatase, the level and/or activity of lactic dehydrogenase, the expression level of CADM1 gene, the expression level of CD44 gene, the expression level of livin gene, the expression level of HIF-1α gene, the expression level of ET-1 gene, the expression level of IGF-1R gene, the expression level of STAT3 gene, the expression level of CEA gene, the expression level of CA199 gene, the expression level of CA125 gene, the expression level of AFP gene, the expression level of CA724 gene, and the expression level of β-HCG gene in the subject after the administration of a).

98. The method according to claim 97, wherein the substance affecting the formation of a TCF/β-catenin complex comprises a TCF/LEF inhibitor.

99. The method according to claim 98, wherein the TCF/LEF inhibitor comprises NCB-0846.

100. The method according to any one of claims 97-99, wherein the substance affecting the formation of a TCF/β-catenin complex comprises a substance affecting the formation of a β-catenin/CBP complex.

101. The method according to claim 100, wherein the substance affecting the formation of a β-catenin/CBP complex comprises PRI-724.

102. The method according to any one of claims 97-101, wherein the substance affecting the formation of a TCF/β-catenin complex comprises a β-catenin inhibitor.

103. The method according to claim 102, wherein the β-catenin inhibitor comprises a substance down-regulating the protein expression level and/or activity of the β-catenin.

104. The method according to claim 103, wherein the substance down-regulating the protein expression level and/or activity of the β-catenin comprises siRNA and/or shRNA.

105. The method according to claim 104, wherein the siRNA comprises a nucleotide sequence as shown in any one of SEQ ID NOS. 1-6.

106. The method according to any one of claims 97-105, wherein the substance affecting the formation of a TCF/β-catenin complex comprises a compound having the structure of Formula (I), (II) or (III) or a pharmaceutically acceptable salt or hydrate thereof: 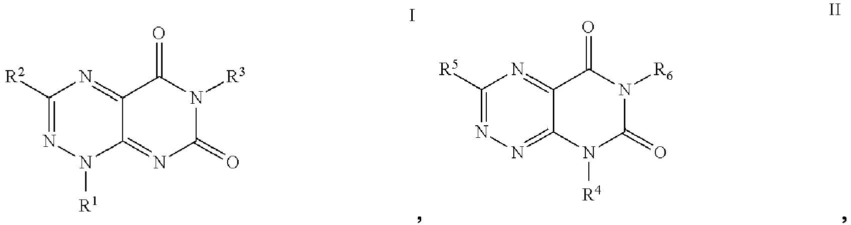 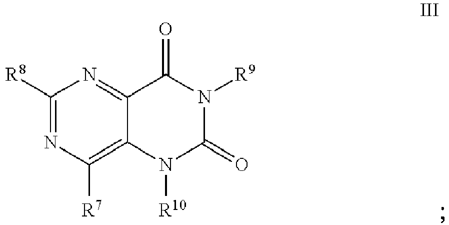
wherein, R¹ and R³ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R² is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl and optionally substituted heteroaryl;
R⁴ and R⁶ are each independently selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl; R⁵ is selected from the group consisting of hydrogen, optionally substituted C₁₋₂₀ alkyl, optionally substituted C₂₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl, optionally substituted heteroaryl, optionally substituted C₁₋₂₀ alkylamino, optionally substituted C₁₋₂₀ alkoxy and optionally substituted C₁₋₂₀ alkylformamido;
R⁷, R⁸, R⁹, and R¹⁰ are each independently selected from the group consisting of hydrogen, C₁₋₂₀ alkyl, optionally substituted C₁₋₂₀ alkenyl, optionally substituted C₂₋₂₀ alkynyl, optionally substituted C₃₋₂₀ cycloalkyl, optionally substituted C₂₋₂₀ heterocycloalkyl, optionally substituted aryl and optionally substituted heteroaryl.

107. The method according to claim 106, wherein the R² is C₄₋₆ alkyl.

108. The method according to any one of claims 106-107, wherein the R² is C₄ alkyl.

109. The method according to any one of claims 106-108, wherein the R² is isobutyl.

110. The method according to any one of claims 106-109, wherein the R¹ is selected from the group consisting of hydrogen and C₁₋₆ alkyl.

111. The method according to any one of claims 106-110, wherein the R¹ is C₃ alkyl.

112. The method according to any one of claims 106-111, wherein the R¹ is isobutyl.

113. The method according to any one of claims 106-112, wherein the R³ is selected from the group consisting of hydrogen and C₁₋₆ alkyl.

114. The method according to any one of claims 106-113, wherein the R³ is methyl.

115. The method according to claim 106, wherein the compound having the structure of Formula (I) comprises PKF 118-310 or a derivative thereof.

116. The method according to claim 115, wherein the derivative of PKF118-310 comprises 3-Methyltoxoflavin and/or Walrycin B.

117. The method according to claim 106, wherein the compound having a structure of Formula II comprises Fervenulin.

118. The method according to any one of claims 97-117, wherein the substance affecting the formation of a TCF/β-catenin complex comprises BI-D1870 and/or Lumazine.

119. The method according to claim 97-118, wherein the tumors comprise solid tumors or non-solid tumors.

120. The method according to any one of claims 97-119, wherein the tumors comprise osteosarcoma, colon cancer, ovarian cancer, and lymphoma.

121. The method according to any one of claims 97-120, wherein the derivative of ascorbic acid comprises a pharmaceutically acceptable ascorbate.

122. The method according to claim 121, wherein the pharmaceutically acceptable ascorbate comprises sodium ascorbate.

123. The method according to any one of claims 97-122, wherein the substance affecting the formation of a TCF/β-catenin complex and the ascorbic acid or a derivative thereof are formulated to be simultaneously administered to a subject.

124. The method according to any one of claims 97-122, wherein the substance affecting the formation of a TCF/β-catenin complex and the ascorbic acid or a derivative thereof are formulated to be separated administered to a subject.

125. The method according to any one of claims 97-124, wherein the drug is formulated so that the substance affecting the formation of a TCF/β-catenin complex is administered at a dose of about 0.05 mg/kg to about 40 mg/kg.

126. The method according to any one of claims 97-125, wherein the drug is formulated so that the ascorbic acid or a derivative thereof is administered at a dose of about 0.05 g/kg to about 2.5 g/kg.

127. The method according to any one of claims 97-126, wherein the drug is formulated so that the substance affecting the formation of a TCF/β-catenin complex and the ascorbic acid or a derivative thereof are administered at a mass ratio of about 1:30 to about 1:5000.

128. The method according to any one of claims 97-127, wherein the drug is formulated so that the substance affecting the formation of a TCF/β-catenin complex and the ascorbic acid or a derivative thereof are administered at an effective amount ratio of about 1:30 to about 1:5000.
